(19) Europäisches Patentamt
European Patent Office
Office européen des brevets

(11) **EP 4 273 169 A1**

(12) **EUROPEAN PATENT APPLICATION**
published in accordance with Art. 153(4) EPC

(43) Date of publication:
**08.11.2023 Bulletin 2023/45**

(51) International Patent Classification (IPC):
*C07K 16/28* (2006.01)   *C07K 16/46* (2006.01)
*C07K 19/00* (2006.01)   *C12N 15/13* (2006.01)
*C12N 15/63* (2006.01)   *A61K 39/395* (2006.01)
*A61P 31/12* (2006.01)   *A61P 35/00* (2006.01)
*A61P 37/06* (2006.01)

(21) Application number: **21914680.0**

(22) Date of filing: **30.12.2021**

(52) Cooperative Patent Classification (CPC):
**A61K 39/395; A61K 45/06; A61P 31/12;
A61P 35/00; A61P 37/02; A61P 37/06;
C07K 16/00; C07K 16/28; C07K 16/46;
C07K 19/00; C12N 15/63**

(86) International application number:
**PCT/CN2021/143371**

(87) International publication number:
**WO 2022/143951 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.12.2020  CN 202011627881**

(71) Applicant: **Keymed Biosciences Co.,Ltd.
Chengdu, Sichuan 610219 (CN)**

(72) Inventors:
• **CHEN, Bo**
**Chengdu, Sichuan 610219 (CN)**
• **XU, Gang**
**Chengdu, Sichuan 610219 (CN)**
• **WANG, Changyu**
**Chengdu, Sichuan 610219 (CN)**
• **YU, Juntao**
**Chengdu, Sichuan 610219 (CN)**

(74) Representative: **Elzaburu S.L.P.
Edificio Torre de Cristal
Paseo de la Castellana 259 C, planta 28
28046 Madrid (ES)**

Remarks:
The complete document including Reference Table(s) and the Sequence Listing(s) can be downloaded from the EPO website

(54) **DEVELOPMENT AND USE OF FUNCTION-ENHANCED ANTIBODY BLOCKING AGENT**

(57) Provided is an antibody or antigen-binding portion thereof that binds to CD70, comprising heavy chain CDRs selected from the group consisting of amino acid sequences SEQ ID NOs: 5, 6, 7, 15, 16, 22, 29, 30, 31, 37, 38, 44, 45, 46, 54, 55, 56, 64, 65, 77, 83, 84, 85, 91, 97, 98, 106, 107, 114, 115, 116, 124, 131, 132, 135, 142, 160 or any variant thereof, and/or light chain CDRs selected from the group consisting of amino acid sequences SEQ ID NOs: 10, 11, 12, 19, 25, 26, 34, 41, 49, 50, 51, 59, 60, 61, 68, 69, 74, 80, 88, 101, 102, 103, 110, 111, 119, 120, 121, 127, 128 or any variant thereof, which can block CD70-CD27 interaction and downstream signal conduction, and inhibit tumors. Also provided is a malignant tumor therapeutic agent containing the CD70 antibody, or antigen-binding portion thereof.

EP 4 273 169 A1

**Description**

**Technical Field**

The present disclosure relates to the development of a function-enhanced antibody blocking agent and use thereof, particularly an antibody binding to CD70 and use thereof.

**Background Art**

CD70 is a type II transmembrane glycoprotein belonging to the tumor necrosis factor superfamily. The interaction of CD70 with CD27 is known as the "CD70-CD27 signaling axis" and plays an important role in cell proliferation and differentiation. Studies have shown that CD70 on the surface of the acute myeloid leukemia cell interacts with CD27 on the surface of the T cell, stimulating $CD4^+$ regulatory $T_{reg}$ cell expansion in the tumor microenvironment, or significantly increasing the proportion of effector $CD8^+$ T cells in the bone marrow that fail, resulting in immune escape from the tumor. Meanwhile, CD27 can also be expressed on the surface of some tumor cells, and the CD70-CD27 activation loop formed between tumor cells can prolong the survival time of tumor cells or lead to proliferation.

Both malignantly proliferating tumor cells and leukemic stem cells ($CD34^+CD38^-$) were detected to extensively express CD70 molecules in leukemia patients, while soluble CD27 was significantly elevated in the patient's serum, which was negatively correlated with prognosis. Blocking CD70-CD27 interaction by antibody can induce leukemia stem cells to produce asymmetric cell division and differentiation, inhibit the formation of tumor colonies, and significantly prolong the survival time of the tumor model mouse.

CD70 is not expressed in normal tissue and hematopoietic progenitor cell but is highly expressed in T-cell lymphoma, B-cell lymphoma, and a substantial fraction of solid tumors. In addition to high expression in tumor cells, other hematological tumor targets such as CD33 or CD123 are also expressed in normal hematopoietic progenitor cells or normal tissues (e.g., lung, prostate, skin, peripheral blood T cells). Thus, the development of CD70 monoclonal antibodies with killing and blocking activity can specifically deplete tumor cells or leukemia stem cells in a patient without affecting the patient's hematopoietic progenitor cells and the restored hematopoietic function.

Acute myeloid leukemia (AML) is a group of highly heterogeneous clonal diseases, which is caused by the over-proliferation or dysdifferentiation of bone marrow hematopoietic stem cells, resulting in the accumulation of a large number of undifferentiated cells in the bone marrow and the rejection of normal blood cells and immune cells, and may also extensively infiltrate the liver, spleen, lymph nodes and other extramedullary organs. The patient eventually died of concurrent infection or other diseases due to anemia and poor immune function. The treatment of acute myeloid leukemia has progressed slowly over the past 40 years and the standard regimen used clinically is still the cytarabine plus daunorubicin therapy introduced in the 1970s.

CN200680021851.8 describes a CD70 antibody with antibody Fc effect that can mediate ADCC, ADCP, and/or CDC effects and can exert cytostatic, cytotoxic, or immunomodulatory effects without or with conjugated to a cytotoxic therapeutic agent. CN200680035376. X describes a CD70 antibody that binds to the human CD70 protein and inhibits the tumor cell growth, which binds to human CD70 with a $K_D$ of $1 \times 10^{-7}$ M or less, and binds to and is internalized by a renal cell carcinoma tumor cell line expressing CD70.

CN201280013552.5 describes CD70 antibodies derived from alpaca that bind with high affinity to human CD70 and exhibit a dissociation rate of less than $7 \times 10^{-4}s^{-1}$ or less. Some preferred embodiments may exhibit strong blocking or inhibition of the interaction of CD70 with its ligand CD27, superior to the CD70 antibodies described in CN200680021851.8 and CN200680035376. X.

**Summary of the Invention**

The present inventors immunized mice with the recombinant CD70 protein to obtain multiple strains of high-affinity antibodies that recognize recombinant human and cynomolgus monkey CD70 proteins. The antibody of the present disclosure has very high ADCC and CDC activities and is significantly superior to the CD70 antibodies of the prior art. At the same time, it can block the CD70-CD27 interaction and downstream signaling with high efficiency, with a significantly stronger inhibitory effect on tumors than existing antibodies.

In one aspect, the present disclosure provides an antibody binding to CD70 or antigen-binding portion thereof.

In one aspect, the present disclosure provides a bispecific antibody or antigen-binding portion thereof.

In one aspect, the present disclosure provides a nucleic acid molecule encoding the aforementioned antibody binding to CD70 or antigen-binding portion thereof or the bispecific antibody or antigen-binding portion thereof.

In one aspect, the present disclosure provides a vector comprising the aforementioned nucleic acid.

In one aspect, the present disclosure provides a cell comprising the aforementioned vector.

In one aspect, the present disclosure provides a pharmaceutical composition or kit comprising the aforementioned antibody or antigen-binding portion thereof, or encoded nucleic acid molecules and pharmaceutically acceptable vectors.

In one aspect, the present disclosure provides an antibody-drug conjugate comprising any of the aforementioned antibody or antigen-binding portion thereof, bispecific or multispecific molecule covalently attached to a therapeutic moiety.

In one aspect, the present disclosure provides a method for treating a CD70-associated condition, which includes the following steps: administering a therapeutically effective amount of antibody or antigen-binding fragment thereof or nucleic acid molecule or vector or cell or pharmaceutical composition of any one of the aforementioned aspects to a subject.

In one aspect, the present disclosure provides use of the antibody or the antigen-binding fragment thereof or the nucleic acid or the vector or the cell or the pharmaceutical composition of any one of the aforementioned aspects in the preparation of a medicament or kit for treating a CD70-associated condition in a subject.

In one aspect, the present disclosure provides use of the antibody or the antigen-binding fragment thereof or the nucleic acid or the vector or the cell or the pharmaceutical composition of any one of the aforementioned aspects in the preparation of a medicament or kit for treating a viral infection in a subject.

**Brief Description of the Drawings**

FIG. 1 shows the result of the SDS-PAGE assay of human CD70 extracellular domain protein recombinantly expressed in HEK293 cells.

FIG. 2-1 shows the results of CD70 expression in human CD70/CHO stably-transfected cell lines using flow cytometry.

FIG. 2-2 shows the results of CD70 expression in monkey CD70/CHO stably-transfected cell lines using flow cytometry.

FIG. 3 shows antibody titers detected by FACS in the serums of immunized mice.

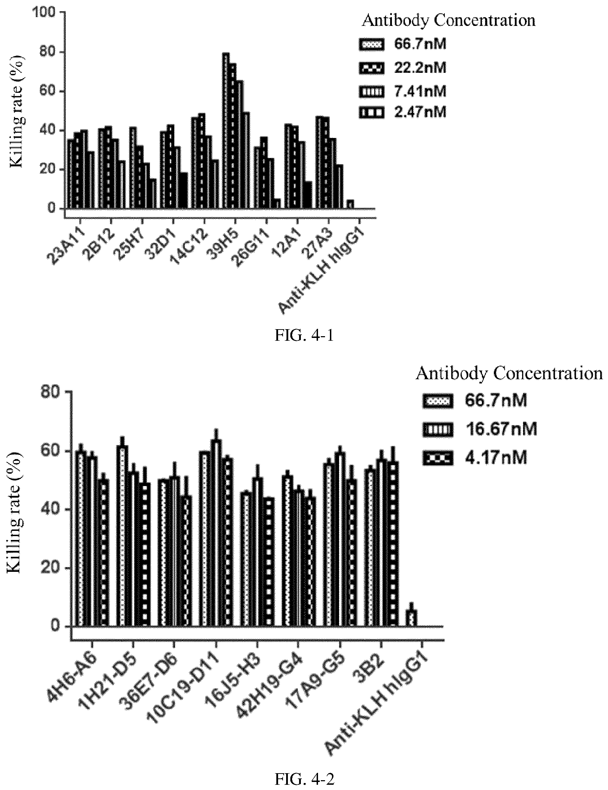

FIGs. 4-1 and 4-2 show CD70 monoclonal antibody CDC activity screening results.

FIG. 5 shows the CD70 monoclonal antibody ADCC activity.

FIG. 6-1 shows the results of the FACS assay of chimeric antibodies binding to CHO-human CD70.

FIG. 6-2 shows the results of the FACS assay of chimeric antibodies binding to CHO-cynomolgus monkey CD70.

FIG. 7-1 shows the results of human CD70/CD27 blocking assays for chimeric antibodies.

FIG. 7-2 shows the results of the cynomolgus monkey CD70/CD27 blocking assay for the chimeric antibodies.

FIG. 8 shows the CDC killing effect of different humanized antibodies on tumor cell Daudi.

FIG. 9-1 shows the results of the FACS assay of humanized antibodies binding to CHO-human CD70.

FIG. 9-2 shows the results of the FACS assay of humanized antibodies binding to CHO-cynomolgus monkey CD70.

FIG. 10-1 shows the results of human CD70/CD27 blocking assays for humanized antibodies.

FIG. 10-2 shows cynomolgus monkey CD70/CD27 blocking assay results for humanized antibodies.

FIG. 11-1 shows the CDC killing effect of humanized antibodies on tumor cell Daudi.

FIG. 11-2 shows the CDC killing effect of humanized antibodies on tumor cell MV-4-11-CD70.

FIG. 11-3 shows the CDC killing effect of humanized antibodies on tumor cell Raji.

FIG. 12-1 shows the ADCC killing effect of humanized antibodies on tumor cell Daudi.

FIG. 12-2 shows the ADCC killing effect of humanized antibodies on tumor cell Raji.

FIG. 13 shows the ADCP killing effect of humanized antibodies on tumor cell Daudi.

FIG. 14 shows the inhibitory effect of humanized antibodies on CD70-mediated stimulation of IL-8 secretion.

FIG. 15-1 shows the effect of humanized CD70 antibodies on the growth of human Burkitt's lymphoma Raji subcutaneous xenograft.

FIG. 15-2 shows the effect of humanized CD70 antibodies on the body weight of a tumor-bearing mouse.

FIG. 16 shows the CDC killing activity of CDC-enhanced CD70 humanized antibodies on tumor cell Daudi.

FIGs. 17-1 and 17-2 show the ADCC killing activity mediated by the CDC-enhanced CD70 humanized antibodies in different Fcγ RIII alleles, PBMC.

FIG. 18 shows the anti-tumor effect of CDC-enhanced CD70 humanized antibodies in a Daudi cell line subcutaneous xenograft NOD/SCID mouse model.

**Detailed Description of the Invention**

**Definitions**

In this disclosure, unless otherwise stated, the scientific and technical terms used herein have the meanings commonly understood by those skilled in the art. Also, as used herein, protein and nucleic acid chemistry, molecular biology, cell and tissue culture, microbiology, immunology, and laboratory procedures used herein are terms and routine procedures widely used in the corresponding fields. Meanwhile, in order to better understand the present disclosure, definitions and explanations of related terms are provided below.

Provided herein is an antibody (e.g. monoclonal antibody) specifically binding to CD70 and antigen-binding fragment thereof. In particular aspects, provided herein is a monoclonal anti-CD70 antibody specifically binding to CD70, wherein the anti-CD70 antibody includes variants of the parent antibody. In a particular aspect, provided herein is an antibody specifically binding to CD70 (e.g. human CD70). In a particular aspect, provided herein is an anti-CD70 antibody comprising a modification in one or more amino acid residues (e.g. 5-13 amino acid substitutions in the framework region of the heavy chain variable region) that maintain an affinity for an antigen as compared to the parent antibody without the modification.

The term "CD70" includes variants, isoforms, homologs, orthologs, and intraspecies homologs. For example, an antibody specific to a human CD70 protein may in some cases cross-react with a CD70 protein from a species other than human. In another embodiment, an antibody specific for a human CD70 protein may be completely specific for a human CD70 protein and may not exhibit species or other types of cross-reactivity, or may cross-react with CD70 from some but not all other species (e.g. cross-react with primate CD70 but not with mouse CD70). The term "human CD70" refers to the human sequence CD70, such as the complete amino acid sequence of human CD70 having Genbank Accession No. P32970. The term "mouse CD70" refers to the mouse sequence CD70, such as the complete amino acid sequence of mouse CD70 having Genbank Accession No. NP_035747. By having, for example, a mutation in a conservative or non-conservative region, the human CD70 sequence may differ from the human CD70 of Genbank Accession No. P32970 and the CD70 have substantially the same biological function as the human CD70 of Genbank Accession No. P32970. For example, one biological function of human CD70 is to bind to the cytokine receptor CD27.

The use of the term "cell surface" is in accordance with its normal meaning in the art and thus includes the exterior of a cell accessible by binding to proteins and other molecules.

The term "signal transduction pathway" refers to the biochemical relationship between multiple signal transduction molecules that play a role in the transmission of signals from one part of the cell to another. The phrase "cell surface receptor" as used herein includes, for example, molecules and complexes of molecules that are capable of receiving a signal and transmitting such a signal across the cell membrane of a cell. One example of a "cell surface receptor" of the present disclosure is the CD70 receptor.

As used herein and unless otherwise specified, the term "about" or "approximately" means within plus or minus 10% of a given value or range. Where an integer is required, the term refers to rounding up or down to the nearest integer within plus or minus 10% of the given value or range.

The phrase "substantially identical" with respect to an antibody chain polypeptide sequence is understood to mean an antibody chain that exhibits at least 60%, 65%, 70%, 75%, 80%, 85%, 90%, 95%, 96%, 97%, 98%, 99% or more sequence identity to a reference polypeptide sequence. With respect to a nucleic acid sequence, the term is understood to mean a nucleotide sequence that exhibits at least greater than 60%, 65%, 70%, 75%, 80%, 85%, 90%, 95%, 96%, 97%, 98%, 99% or more sequence identity to a reference nucleic acid sequence.

Sequence "identity" or "homoousia" has its art-recognized meaning and the percentage of sequence identity between two nucleic acid or polypeptide molecules or regions can be calculated using published techniques. Sequence identity can be measured along the entire length of a polynucleotide or polypeptide or along a region of the molecule. While there are many methods of measuring identity between two polynucleotides or polypeptides, the term "identity" is well known to the skilled artisan (Carrillo, H. & Lipman, D., SIAM J Applied Math 48:1073 (1988)).

A "substitutional" variant is a variant in which at least one amino acid residue in the native sequence has been removed and inserted at its same position by a different amino acid. The substitution may be single, wherein only one amino acid in the molecule is substituted; or the substitution may be multiple, wherein the same molecule has two or more amino acids substituted. Multiple substitutions may be at consecutive positions. Likewise, an amino acid may be substituted with multiple residues, wherein such variants include both substitutions and insertions. An "insertional" variant is a variant in which one or more amino acids are inserted immediately adjacent to an amino acid at a particular position in a native sequence. An immediately adjacent amino acid means an amino acid attached to the $\alpha$-carboxy or $\alpha$-amino functional group of the amino acid. A "deletional" variant is a variant in which one or more amino acids in the native amino acid sequence have been removed. Typically, deletional variants have one or two amino acids deleted in a particular region of the molecule.

With respect to the variable domains of antibodies, the term "variable" refers to certain portions of related molecules that differ widely in sequence between antibodies and are used for specific recognition and binding of a particular antibody to its specific target. However, the variability is not uniformly distributed throughout the variable domain of the antibody. Variability is concentrated in what is known as the complementary determining region (CDRs, i.e. CDR1, CDR2, CDR3) or three segments of the hypervariable region, all of which are located within the variable domains of the light and heavy chains. The more conserved portions within the variable domains are referred to as framework (FR) regions or framework sequences. Each variable domain of a native heavy and light chain comprises four FR regions, predominantly in a β-folded configuration, joined by three CDRs, the CDRs forming loops that join and in some cases form part of the β-folded structure. The CDRs of each chain are typically joined together in close proximity by FR regions and aid in the formation of antibody target binding sites (epitopes or determinants) by means of CDR from other chains. As used herein, immunoglobulin amino acid residue numbering is in accordance with the immunoglobulin amino acid residue numbering system of Kabat et al. unless otherwise indicated. One CDR may have the ability to specifically bind to an associated epitope.

As used herein, an "antibody fragment" or "antigen-binding fragment" of an antibody refers to any portion of a full-length antibody that is less than full-length but that comprises at least a portion of the variable region (e.g. one or more CDRs and/or one or more antibody binding sites) of the antibody that binds to an antigen and thus retains binding specificity as well as at least a portion of the specific binding capacity of the full-length antibody. Thus, an antigen-binding fragment refers to an antibody fragment that comprises an antigen-binding portion that binds the same antigen as the antibody from which the antibody fragment is derived. Antibody fragments include antibody derivatives produced by enzymatic treatment of full-length antibodies, as well as synthetically produced derivatives, e.g. recombinantly produced derivatives. Antibodies include antibody fragments. Examples of antibody fragments include but are not limited to, Fab, Fab', F(ab')$_2$, single chain Fv(scFv), Fv, dsFv, diabodies, Fd and Fd' fragments, and other fragments, including modified fragments. The fragments may comprise multiple chains linked together, for example by disulfide bonds and/or by peptide linkers. Antibody fragments generally comprise at least or about 50 amino acids, and typically at least or about 200 amino acids. Antigen-binding fragments include any antibody fragment that, when inserted into an antibody framework (e.g. by displacement of the corresponding region), results in an antibody that immunospecifically binds to (i.e. exhibits a Ka of at least or at least about $10^7$-$10^8$ M$^{-1}$) an antigen. A "functional fragment" or "analog of an anti-CD70 antibody" is a fragment or analog that prevents or substantially reduces the ability of the receptor to bind a ligand or initiate signal transduction. As used herein, the functional fragment generally has the same meaning as an antibody fragment, and with respect to an antibody, it may refer to a fragment that can prevent or substantially reduce the ability of the receptor to bind to a ligand or initiate signal signaling, such as Fv, Fab, F (ab') $_2$, etc. An "Fv" fragment consists of a dimer formed by a variable domain of a heavy chain and a variable domain of a light chain in the manner of a non-covalent association (V$_H$-V$_L$ dimer). In this configuration, the three CDRs of each variable domain interact to define a target binding site on the surface of V$_H$-V$_L$ dimer, as is the case with intact antibodies. The six CDRs together confer target binding specificity to the intact antibody. However, even a single variable domain (or half of an Fv comprising only three target-specific CDRs) may still have the ability to recognize and bind targets.

As used herein, the term "bispecific antibody" (BsAb) refers to antibodies and/or antigen-binding molecules capable of specifically binding to two different antigenic determinants. Generally, a bispecific antibody and/or antigen-binding molecule comprises two antigen-binding sites, each of which is specific for a different antigenic determinant. In certain embodiments, the bispecific antibody and/or antigen-binding molecule is capable of binding two antigenic determinants simultaneously, particularly two antigenic determinants expressed on two different cells.

As used herein, "monoclonal antibody" refers to a population of identical antibodies, meaning that each individual antibody molecule in the population of monoclonal antibodies is identical to another antibody molecule. This property is in contrast to the property of a polyclonal population of antibodies comprising antibodies having a plurality of different sequences. Monoclonal antibodies can be prepared by a number of well-known methods (Smith et al. (2004) J.Clin.Pathol.57, 912-917; and Nelson et al., J Clin Pathol( 2000), 53, 111-117). For example, monoclonal antibodies can be made by immortalizing B cells, for example by fusion with myeloma cells to produce hybridoma cell lines or by infecting B cells with a virus, such as EBV Recombinant techniques can also be used to prepare antibodies from clonal populations of host cells in vitro by transforming host cells with plasmids carrying artificial sequences encoding the nucleotides of the antibodies.

As used herein, the term "hybridoma" or "hybridoma cell" refers to a cell or cell line (typically a myeloma or lymphoma cell) resulting from the fusion of antibody-producing lymphocytes and non-antibody-producing cancer cells. As is known to those of ordinary skill in the art, hybridomas can be propagated and continuously supplied to produce a particular monoclonal antibody. Methods for producing hybridomas are known in the art. When referring to the term "hybridoma" or "hybridoma cell", they also include subclones and progeny cells of hybridomas.

As used herein, a full-length antibody is an antibody having two full-length heavy chains (e.g. VH-CH1-CH2-CH3 or VH-CH1-CH2-CH3-CH4) and two full-length light chains (VL-CL) and a hinge region, e.g. an antibody naturally produced by an antibody-secreting B cell as well as a synthetically produced antibody having the same domain.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to a form of non-human (e.g. mouse) antibody that is a chimeric immunoglobulin, immunoglobulin chain or fragment thereof (e.g., Fv, Fab, Fab', F(ab')2 or other antigen-binding subsequences of an antibody) that contains minimal sequence derived from non-human immunoglobulin. Preferably, the humanized antibody is a human immunoglobulin (recipient antibody) in which residues of the complementarity-determining region (CDR) of the recipient antibody are replaced by CDR residues from a non-human species (donor antibody) such as mouse, rat, or rabbit having the desired specificity, affinity, and capacity.

Furthermore, in humanization it is also possible to mutate amino acid residues within the CDR1, CDR2, and/or CDR3 regions of VH and/or VL, thereby improving one or more binding properties (e.g. affinity) of the antibody. Mutations can be introduced, e.g. by PCR-mediated mutagenesis, and their effect on antibody binding or other functional properties can be assessed using the in vitro or in vivo assays described herein. Typically, conservative mutations are introduced. Such mutations may be amino acid substitutions, additions, or deletions. In addition, mutations within CDR typically do not exceed one or two. Thus, the humanized antibodies of the present disclosure also encompass antibodies comprising one or two amino acid mutations within CDR.

As used herein, the term "CDR" refers to a complementarity-determining region known to have three CDR per heavy and light chain of an antibody molecule. CDR is also known as a hypervariable region and is present in the variable region of each of the heavy and light chains of an antibody with a very high site of variability in the primary structure of CDR. In the present specification, the CDR of the heavy chain is represented by CDR1, CDR2, CDR3 from the amino terminus of the amino-terminal sequence of the heavy chain, and the CDR of the light chain is represented by CDR1, CDR2, CDR3 from the amino terminus of the amino-terminal sequence of the light chain. These sites are adjacent to each other in the tertiary structure and determine the specificity of the antigen to which the antibody binds.

As used herein, the term "epitope" refers to any antigenic determinant on an antigen to which the paratope of an antibody binds. Epitopic determinants usually comprise chemically active surface types of molecules, such as amino acids or sugar side chains, and usually have specific three-dimensional structural characteristics as well as specific charge characteristics.

As used herein, "specific binding" or "immunospecifically binding" with respect to an antibody or antigen-binding fragment thereof is used interchangeably herein and refers to the ability of an antibody or antigen-binding fragment to form one or more non-covalent bonds with the same antigen through non-covalent interactions between the antibody and the antibody binding site of the antigen. The antigen may be an isolated antigen or present in a tumor cell. Generally, an antibody that immunospecifically binds (or specifically binds) to an antigen is one that binds to the antigen with an affinity constant (Ka) of about or of $1 \times 10^7$ M$^{-1}$ or $1 \times 10^8$ M$^{-1}$ or more (or a dissociation constant (Kd) of $1 \times 10^{-7}$ M or $1 \times 10^{-8}$ M or less). Affinity constants can be determined by standard kinetic methods for antibody reactions, e.g. immunoassays, surface plasmon resonance (SPR) (Rich and Myszka (2000) Curr. Opin.Biotechnol 11:54; Englebienne (1998) Analyst. 123:1599), isothermal titration calorimetry (ITC), or other kinetic interaction assays known in the art. Instruments and methods for detecting and monitoring the rate of binding in real time are known and commercially available (with reference to BiaCore 2000, Biacore AB, Upsala, Sweden and GE Healthcare Life Sciences; Malmqvist (2000) Biochem.Soc.Trans. 27:335).

As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA), typically linked together by phosphodiester bonds. As used herein, the term "nucleic acid molecule" is intended to include DNA molecules and RNA molecules. The nucleic acid molecule may be single-stranded or double-stranded and may be cDNA.

As used herein, an isolated nucleic acid molecule is a nucleic acid molecule isolated from other nucleic acid molecules present in the natural source of the nucleic acid molecule. An "isolated" nucleic acid molecule, such as a cDNA molecule, can be substantially free of other cellular material or culture medium when prepared by recombinant techniques, or substantially free of chemical precursors or other chemical components when chemically synthesized. Exemplary isolated nucleic acid molecules provided herein include isolated nucleic acid molecules encoding a provided antibody or antigen-binding fragment.

As used herein, "operably linked" with respect to a nucleic acid sequence, region, element or domain means that the nucleic acid regions are functionally related to one another. For example, a promoter can be operably linked to a nucleic acid encoding a polypeptide such that the promoter regulates or mediates transcription of the nucleic acid.

Also provided are "conservative sequence modifications" of the sequences set forth in the sequence listings described herein, i.e. nucleotide and amino acid sequence modifications that do not eliminate binding of an antibody encoded by or containing an amino acid sequence to an antigen. These conservative sequence modifications include conservative nucleotide and amino acid substitutions and nucleotide and amino acid additions and deletions. For example, modifications can be introduced into the sequence listings described herein by standard techniques known in the art, such as site-directed mutagenesis and PCR-mediated mutagenesis. Conservative sequence modifications include conservative amino acid substitutions in which an amino acid residue is replaced with an amino acid residue having a similar side chain. Families of amino acid residues having similar side chains have been defined in the art. These families include amino acids with basic side chains (e.g. lysine, arginine, histidine), amino acids with acidic side chains (e.g. aspartic acid, glutamic acid), amino acids with uncharged polar side chains (e.g. glycine, asparagine, glutamine, serine, threonine, tyrosine, cysteine, tryptophan), amino acids with nonpolar side chains (e.g. alanine, valine, leucine, isoleucine, proline, phenylalanine, methionine), amino acids with β-branched side chains (e.g. threonine, valine, isoleucine) and amino acids with aromatic side chains (e.g. tyrosine, phenylalanine, tryptophan, histidine). Thus, a predicted nonessential amino acid residue in an anti-CD70 antibody is preferably replaced with another amino acid residue from the same side chain family. Methods for identifying conservative substitutions of nucleotides and amino acids that do not eliminate antigen-binding are well known in the art (see, e.g. Brummell et al., Biochem. 32: 1180-1187 (1993); Kobayashi et al., Protein Eng. 12(10): 879-884 (1999); Burks et al., Proc.Natl.Acad.Sci.USA 94: 412-417 (1997)).

Alternatively, in another embodiment, mutations can be randomly introduced along all or a portion of the anti-CD70 antibody coding sequence by, for example, saturation mutagenesis, and the resulting modified anti-CD70 antibody can be screened for improved binding activity.

As used herein, "expression" refers to the process of producing a polypeptide by transcription and translation of a polynucleotide. The level of expression of a polypeptide can be assessed using any method known in the art, including, for example, methods of determining the amount of polypeptide produced from a host cell. Such methods may include, but are not limited to, quantitation of polypeptides in cell lysates by ELISA, gel electrophoresis followed by Coomassie blue staining, Lowry protein assay, and Bradford protein assay.

As used herein, a "host cell" is a cell for receiving, maintaining, replicating, and expanding a vector. Host cells may also be used to express the polypeptide encoded by the vector. As the host cell divides, the nucleic acid contained in the vector replicates, thereby amplifying the nucleic acid. The host cell may be a eukaryotic cell or a prokaryotic cell. Suitable host cells include but are not limited to, CHO cells, various COS cells, HeLa cells, and HEK cells such as HEK 293 cells.

As used herein, a "vector" is a replicable nucleic acid from which one or more heterologous proteins can be expressed when the vector is transformed into an appropriate host cell. References to vectors include those into which a nucleic acid encoding a polypeptide or fragment thereof may be introduced, typically by restriction digestion and ligation. References to vectors also include those comprising a nucleic acid encoding a polypeptide. vectors are used to introduce a nucleic acid encoding a polypeptide into a host cell, to amplify the nucleic acid, or to express/display the polypeptide encoded by the nucleic acid. The vector is usually kept episomal but can be designed such that the gene or part thereof is integrated into the chromosome of the genome. Also contemplated are vectors for artificial chromosomes, such as yeast artificial vectors and mammalian artificial chromosomes. The selection and use of such vehicles are well-known to those skilled in the art.

As used herein, a vector also includes a "viral vector" or a "viral vector". A viral vector is an engineered virus operably linked to a foreign gene to transfer (as a vehicle or shuttle) the foreign gene into a cell.

As used herein, an "expression vector" includes a vector capable of expressing a DNA operably linked to regulatory sequences, such as a promoter region, capable of affecting the expression of such a DNA fragment. Such additional segments may include promoter and terminator sequences and optionally may include one or more origins of replication, one or more selectable markers, enhancers, polyadenylation signals, and the like. Expression vectors are typically derived from plasmid or viral DNA, or may contain elements of both. Thus, an expression vector refers to a recombinant DNA or RNA construct, such as a plasmid, phage, recombinant virus or other vector, which when introduced into an appropriate host cell, results in the expression of a cloned DNA. Suitable expression vectors are well known to those of skill in the art and include expression vectors that are replicable in eukaryotic and/or prokaryotic cells as well as expression vectors that remain episomal or that are integrated into the host cell genome. As used herein, "treating" a subject with a disease or condition means that the subject's symptoms are partially or completely alleviated or remain unchanged after treatment. Thus, treatment includes prophylaxis, treatment, and/or cure. Prevention refers to preventing the underlying disease and/or preventing the worsening of symptoms or the progression of the disease. Treatment also includes any antibody or antigen-binding fragment thereof provided and any pharmaceutical use of the compositions provided herein.

As used herein, "therapeutic effect" refers to an effect resulting from the treatment of a subject that alters, typically ameliorates, or ameliorates a symptom of a disease or condition, or cures a disease or condition.

As used herein, a "therapeutically effective amount" or "therapeutically effective dose" refers to an amount of a substance, compound, material, or composition comprising a compound that is at least sufficient to produce a therapeutic effect after administration to a subject. Thus, it is an amount necessary to prevent, cure, ameliorate, block, or partially block the symptoms of a disease or condition.

As used herein, a "prophylactically effective amount" or "prophylactically effective dose" refers to an amount of a substance, compound, material, or composition comprising a compound that, when administered to a subject, has the desired prophylactic effect, e.g. prevents or delays the occurrence or recurrence of a disease or condition, reduces the likelihood of the occurrence or recurrence of a disease or condition. A fully prophylactically effective dose does not necessarily occur by administration of one dose, and may occur only after administration of a series of doses. Thus, a prophylactically effective amount can be administered in one or more administrations.

As used herein, the term "patient" refers to a mammal, such as a human.

**Detailed description.**

In one aspect, this disclosure provides an antibody or antigen-binding portion thereof that binds to CD70, comprising heavy chain CDRs selected from the group consisting of amino acid sequences SEQ ID NOs: 5, 6, 7, 15, 16, 22, 29, 30, 31, 37, 38, 44, 45, 46, 54, 55, 56, 64, 65, 77, 83, 84, 85, 91, 97, 98, 106, 107, 114, 115, 116, 124, 131, 132, 135, 142, 160 or any variant thereof, and/or light chain CDRs selected from the group consisting of amino acid sequences SEQ ID NOs: 10, 11, 12, 19, 25, 26, 34, 41, 49, 50, 51, 59, 60, 61, 68, 69, 74, 80, 88, 101, 102, 103, 110, 111, 119, 120, 121, 127, 128 or any variant thereof. The antibody or antigen-binding portion thereof according to the previous aspect, comprising a heavy chain CDR1 selected from the group consisting of amino acid sequences SEQ ID NOs: 5, 15, 29, 44, 54, 83, 96, 114, 131 or any variant thereof, a heavy chain CDR2 selected from the group consisting of amino acid sequences SEQ ID NOs: 6, 30, 37, 45, 55, 64, 77, 84, 91, 97, 106, 115, 124, 132, 135, 142 or any variant thereof, a heavy chain CDR3 selected from the group consisting of amino acid sequences SEQ ID NOs: 7, 16, 22, 31, 38, 46, 56, 65, 85, 98, 107, 116 or any variant thereof; and/or a light chain CDR1 selected from the group consisting of amino acid sequences SEQ ID NOs: 10, 16, 25, 34, 51, 59, 59, 38, 74, 88, 101, 110, 119, 127, 149 or any variant thereof, a light chain CDR2 selected from the group consisting of amino acid sequences SEQ ID NOs: 11, 50, 60, 80, 102, 111, 120, 128, 160 or any variant thereof, a light chain CDR3 selected from the group consisting of amino acid sequences SEQ ID NOs: 12, 26, 51, 61, 69, 103, 121 or any variant thereof.

The antibody or antigen-binding portion thereof according to the previous aspect, comprising a CDR combination of the heavy chain and light chain selected from the group consisting of:

(1) heavy chain CDR1, CDR2, CDR3 sequences comprising SEQ ID NOs: 5, 6, 7, and/or light chain CDR1, CDR2, CDR3 sequences comprising SEQ ID NOs: 10, 11, 12, respectively;
(2) heavy chain CDR1, CDR2, CDR3 sequences comprising SEQ ID NOs: 15, 6, 16, and/or light chain CDR1, CDR2, CDR3 sequences comprising SEQ ID NOs: 19, 11, 12, respectively;
(3) heavy chain CDR1, CDR2, CDR3 sequences comprising SEQ ID NOs: 5, 6, 22, and/or light chain CDR1, CDR2, CDR3 sequences comprising SEQ ID NOs: 25, 11, 26, respectively;
(4) heavy chain CDR1, CDR2, CDR3 sequences comprising SEQ ID NOs: 29, 30, 31, and/or light chain CDR1, CDR2, CDR3 sequences comprising SEQ ID NOs: 34, 11, 26, respectively;
(5) heavy chain CDR1, CDR2, CDR3 sequences comprising SEQ ID NOs: 5, 37, 38, and/or light chain CDR1, CDR2, CDR3 sequences comprising SEQ ID NOs: 41, 11, 12, respectively;
(6) heavy chain CDR1, CDR2, CDR3 sequences comprising SEQ ID NOs: 44, 45, 46, and/or light chain CDR1, CDR2, CDR3 sequences comprising SEQ ID NOs: 49, 50, 51, respectively;
(7) heavy chain CDR1, CDR2, CDR3 sequences comprising SEQ ID NOs: 54, 55, 56, and/or light chain CDR1, CDR2, CDR3 sequences comprising SEQ ID NOs: 59, 60, 61, respectively;
(8) heavy chain CDR1, CDR2, CDR3 sequences comprising SEQ ID NOs: 5, 64, 65, and/or light chain CDR1, CDR2, CDR3 sequences comprising SEQ ID NOs: 68, 11, 69, respectively;
(9) heavy chain CDR1, CDR2, CDR3 sequences comprising SEQ ID NOs: 29, 30, 31, and/or light chain CDR1, CDR2, CDR3 sequences comprising SEQ ID NOs: 74, 11, 26, respectively;
(10) heavy chain CDR1, CDR2, CDR3 sequences comprising SEQ ID NOs: 44, 77, 46, and/or light chain CDR1, CDR2, CDR3 sequences comprising SEQ ID NOs: 49, 80, 51, respectively;
(11) heavy chain CDR1, CDR2, CDR3 sequences comprising SEQ ID NOs: 83, 84, 85, and/or light chain CDR1, CDR2, CDR3 sequences comprising SEQ ID NOs: 88, 11, 12, respectively;
(12) heavy chain CDR1, CDR2, CDR3 sequences comprising SEQ ID NOs: 5, 91, 85, and/or light chain CDR1, CDR2, CDR3 sequences comprising SEQ ID NOs: 19, 11, 12, respectively;
(13) heavy chain CDR1, CDR2, CDR3 sequences comprising SEQ ID NOs: 96, 97, 98, and/or light chain CDR1, CDR2, CDR3 sequences comprising SEQ ID NOs: 101, 102, 103, respectively;
(14) heavy chain CDR1, CDR2, CDR3 sequences comprising SEQ ID NOs: 15, 106, 107, and/or light chain CDR1, CDR2, CDR3 sequences comprising SEQ ID NOs: 110, 111, 103, respectively;
(15) heavy chain CDR1, CDR2, CDR3 sequences comprising SEQ ID NOs: 114, 115, 116, and/or light chain CDR1, CDR2, CDR3 sequences comprising SEQ ID NOs: 119, 120, 121, respectively;
(16) heavy chain CDR1, CDR2, CDR3 sequences comprising SEQ ID NOs: 15, 124, 16, and/or light chain CDR1, CDR2, CDR3 sequences comprising SEQ ID NOs: 127, 128, 103, respectively;
(17) heavy chain CDR1, CDR2, CDR3 sequences comprising SEQ ID NOs: 131, 132, 116, and/or light chain CDR1, CDR2, CDR3 sequences comprising SEQ ID NOs: 119, 120, 121, respectively;
(18) heavy chain CDR1, CDR2, CDR3 sequences comprising SEQ ID NOs: 44, 135, 46, and/or light chain CDR1, CDR2, CDR3 sequences comprising SEQ ID NOs: 149, 80, 51, respectively;
(19) heavy chain CDR1, CDR2, CDR3 sequences comprising SEQ ID NOs: 29, 142, 31, and/or light chain CDR1, CDR2, CDR3 sequences comprising SEQ ID NOs: 25, 11, 26, respectively; and

(20) heavy chain CDR1, CDR2, CDR3 sequences comprising SEQ ID NOs: 29, 142, 31, and/or light chain CDR1, CDR2, CDR3 sequences comprising SEQ ID NOs: 25, 160, 26, respectively.

The antibody or antigen-binding portion thereof according to the previous aspect, comprising a heavy chain variable region selected from the group consisting of amino acid sequences SEQ ID NOs: 3, 13, 20, 27, 35, 42, 52, 62, 70, 75, 81, 89, 94, 104, 112, 122, 129, 133, 136, 138, 140, 143, 145 or any variant thereof, and/or a light chain variable region selected from the group consisting of amino acid sequences SEQ ID NOs: 8, 17, 23, 32, 39, 47, 57, 66, 72, 78, 86, 92, 99, 108, 117, 125, 147, 150, 152, 154, 156, 158 or any variant thereof.

In a preferred embodiment of the present disclosure, the antibody or antigen-binding portion thereof comprises the heavy chain variable region of the amino acid sequence SEQ ID NO: 3 or any variant thereof, and the light chain variable region of the amino acid sequence SEQ ID NO: 8 or any variant thereof.

In a preferred embodiment of the present disclosure, the antibody or antigen-binding portion thereof comprises the heavy chain variable region of the amino acid sequence SEQ ID NO: 13 or any variant thereof, and the light chain variable region of the amino acid sequence SEQ ID NO: 17 or any variant thereof.

In a preferred embodiment of the present disclosure, the antibody or antigen-binding portion thereof comprises the heavy chain variable region of the amino acid sequence SEQ ID NO: 20 or any variant thereof, and the light chain variable region of the amino acid sequence SEQ ID NO: 23 or any variant thereof.

In a preferred embodiment of the present disclosure, the antibody or antigen-binding portion thereof comprises the heavy chain variable region of the amino acid sequence SEQ ID NO: 27 or any variant thereof, and the light chain variable region of the amino acid sequence SEQ ID NO: 32 or any variant thereof.

In a preferred embodiment of the present disclosure, the antibody or antigen-binding portion thereof comprises the heavy chain variable region of the amino acid sequence SEQ ID NO: 35 or any variant thereof, and the light chain variable region of the amino acid sequence SEQ ID NO: 39 or any variant thereof.

In a preferred embodiment of the present disclosure, the antibody or antigen-binding portion thereof comprises the heavy chain variable region of the amino acid sequence SEQ ID NO: 42 or any variant thereof, and the light chain variable region of the amino acid sequence SEQ ID NO: 47 or any variant thereof.

In a preferred embodiment of the present disclosure, the antibody or antigen-binding portion thereof comprises the heavy chain variable region of the amino acid sequence SEQ ID NO: 52 or any variant thereof, and the light chain variable region of the amino acid sequence SEQ ID NO: 57 or any variant thereof.

In a preferred embodiment of the present disclosure, the antibody or antigen-binding portion thereof comprises the heavy chain variable region of the amino acid sequence SEQ ID NO: 62 or any variant thereof, and the light chain variable region of the amino acid sequence SEQ ID NO: 66 or any variant thereof.

In a preferred embodiment of the present disclosure, the antibody or antigen-binding portion thereof comprises the heavy chain variable region of the amino acid sequence SEQ ID NO: 70 or any variant thereof, and the light chain variable region of the amino acid sequence SEQ ID NO: 72 or any variant thereof.

In a preferred embodiment of the present disclosure, the antibody or antigen-binding portion thereof comprises the heavy chain variable region of the amino acid sequence SEQ ID NO: 75 or any variant thereof, and the light chain variable region of the amino acid sequence SEQ ID NO: 78 or any variant thereof.

In a preferred embodiment of the present disclosure, the antibody or antigen-binding portion thereof comprises the heavy chain variable region of the amino acid sequence SEQ ID NO: 81 or any variant thereof, and the light chain variable region of the amino acid sequence SEQ ID NO: 86 or any variant thereof.

In a preferred embodiment of the present disclosure, the antibody or antigen-binding portion thereof comprises the heavy chain variable region of the amino acid sequence SEQ ID NO: 89 or any variant thereof, and the light chain variable region of the amino acid sequence SEQ ID NO: 92 or any variant thereof.

In a preferred embodiment of the present disclosure, the antibody or antigen-binding portion thereof comprises the heavy chain variable region of the amino acid sequence SEQ ID NO: 94 or any variant thereof, and the light chain variable region of the amino acid sequence SEQ ID NO: 99 or any variant thereof.

In a preferred embodiment of the present disclosure, the antibody or antigen-binding portion thereof comprises the heavy chain variable region of the amino acid sequence SEQ ID NO: 104 or any variant thereof, and the light chain variable region of the amino acid sequence SEQ ID NO: 108 or any variant thereof.

In a preferred embodiment of the present disclosure, the antibody or antigen-binding portion thereof comprises the heavy chain variable region of the amino acid sequence SEQ ID NO: 112 or any variant thereof, and the light chain variable region of the amino acid sequence SEQ ID NO: 117 or any variant thereof.

In a preferred embodiment of the present disclosure, the antibody or antigen-binding portion thereof comprises the heavy chain variable region of the amino acid sequence SEQ ID NO: 122 or any variant thereof, and the light chain variable region of the amino acid sequence SEQ ID NO: 125 or any variant thereof.

In a preferred embodiment of the present disclosure, the antibody or antigen-binding portion thereof comprises the heavy chain variable region of the amino acid sequence SEQ ID NO: 129 or any variant thereof, and the light chain variable region of the amino acid sequence SEQ ID NO: 117 or any variant thereof.

In a preferred embodiment of the present disclosure, the antibody or antigen-binding portion thereof comprises the heavy chain variable region of the amino acid sequence SEQ ID NO: 133 or any variant thereof, and the light chain variable region of the amino acid sequence SEQ ID NO: 147 or any variant thereof.

In a preferred embodiment of the present disclosure, the antibody or antigen-binding portion thereof comprises the heavy chain variable region of the amino acid sequence SEQ ID NO: 133 or any variant thereof, and the light chain variable region of the amino acid sequence SEQ ID NO: 150 or any variant thereof.

In a preferred embodiment of the present disclosure, the antibody or antigen-binding portion thereof comprises the heavy chain variable region of the amino acid sequence SEQ ID NO: 133 or any variant thereof, and the light chain variable region of the amino acid sequence SEQ ID NO: 152 or any variant thereof.

In a preferred embodiment of the present disclosure, the antibody or antigen-binding portion thereof comprises the heavy chain variable region of the amino acid sequence SEQ ID NO: 136 or any variant thereof, and the light chain variable region of the amino acid sequence SEQ ID NO: 147 or any variant thereof.

In a preferred embodiment of the present disclosure, the antibody or antigen-binding portion thereof comprises the heavy chain variable region of the amino acid sequence SEQ ID NO: 136 or any variant thereof, and the light chain variable region of the amino acid sequence SEQ ID NO: 150 or any variant thereof.

In a preferred embodiment of the present disclosure, the antibody or antigen-binding portion thereof comprises the heavy chain variable region of the amino acid sequence SEQ ID NO: 136 or any variant thereof, and the light chain variable region of the amino acid sequence SEQ ID NO: 152 or any variant thereof.

In a preferred embodiment of the present disclosure, the antibody or antigen-binding portion thereof comprises the heavy chain variable region of the amino acid sequence SEQ ID NO: 138 or any variant thereof, and the light chain variable region of the amino acid sequence SEQ ID NO: 147 or any variant thereof.

In a preferred embodiment of the present disclosure, the antibody or antigen-binding portion thereof comprises the heavy chain variable region of the amino acid sequence SEQ ID NO: 138 or any variant thereof, and the light chain variable region of the amino acid sequence SEQ ID NO: 150 or any variant thereof.

In a preferred embodiment of the present disclosure, the antibody or antigen-binding portion thereof comprises the heavy chain variable region of the amino acid sequence SEQ ID NO: 138 or any variant thereof, and the light chain variable region of the amino acid sequence SEQ ID NO: 152 or any variant thereof.

In a preferred embodiment of the present disclosure, the antibody or antigen-binding portion thereof comprises the heavy chain variable region of the amino acid sequence SEQ ID NO: 140 or any variant thereof, and the light chain variable region of the amino acid sequence SEQ ID NO: 154 or any variant thereof.

In a preferred embodiment of the present disclosure, the antibody or antigen-binding portion thereof comprises the heavy chain variable region of the amino acid sequence SEQ ID NO: 140 or any variant thereof, and the light chain variable region of the amino acid sequence SEQ ID NO: 156 or any variant thereof.

In a preferred embodiment of the present disclosure, the antibody or antigen-binding portion thereof comprises the heavy chain variable region of the amino acid sequence SEQ ID NO: 140 or any variant thereof, and the light chain variable region of the amino acid sequence SEQ ID NO: 158 or any variant thereof.

In a preferred embodiment of the present disclosure, the antibody or antigen-binding portion thereof comprises the heavy chain variable region of the amino acid sequence SEQ ID NO: 143 or any variant thereof, and the light chain variable region of the amino acid sequence SEQ ID NO: 154 or any variant thereof.

In a preferred embodiment of the present disclosure, the antibody or antigen-binding portion thereof comprises the heavy chain variable region of the amino acid sequence SEQ ID NO: 143 or any variant thereof, and the light chain variable region of the amino acid sequence SEQ ID NO: 156 or any variant thereof.

In a preferred embodiment of the present disclosure, the antibody or antigen-binding portion thereof comprises the heavy chain variable region of the amino acid sequence SEQ ID NO: 143 or any variant thereof, and the light chain variable region of the amino acid sequence SEQ ID NO: 158 or any variant thereof.

In a preferred embodiment of the present disclosure, the antibody or antigen-binding portion thereof comprises the heavy chain variable region of the amino acid sequence SEQ ID NO: 145 or any variant thereof, and the light chain variable region of the amino acid sequence SEQ ID NO: 154 or any variant thereof.

In a preferred embodiment of the present disclosure, the antibody or antigen-binding portion thereof comprises the heavy chain variable region of the amino acid sequence SEQ ID NO: 145 or any variant thereof, and the light chain variable region of the amino acid sequence SEQ ID NO: 156 or any variant thereof.

In a preferred embodiment of the present disclosure, the antibody or antigen-binding portion thereof comprises the heavy chain variable region of the amino acid sequence SEQ ID NO: 145 or any variant thereof, and the light chain variable region of the amino acid sequence SEQ ID NO: 158 or any variant thereof.

In one aspect, the present disclosure provides a bispecific or multispecific molecule that includes the antibody or antigen-binding portion thereof according to any one of the aforementioned aspects.

A nucleic acid molecule encoding antibody or antigen-binding portion thereof or the bispecific or multispecific molecule according to any one of the aforementioned aspects.

In a preferred embodiment of the present disclosure, provided is a nucleic acid molecule encoding the antibody or antigen-binding portion thereof, the bispecific or multispecific molecule comprising an antibody heavy chain nucleic acid sequence selected from the group consisting of SEQ ID NOs: 4, 14, 21, 28, 36, 43, 53, 63, 71, 76, 82, 90, 95, 105, 113, 123, 130, 134, 137, 139, 141, 144, 146 or any variant thereof, and/or an antibody light chain nucleic acid sequence selected from the group consisting of SEQ ID NOs: 9, 18, 24, 33, 40, 48, 58, 67, 73, 79, 87, 93, 100, 109, 118, 126, 148, 151, 153, 155, 157, 159 or any variant thereof.

In a preferred embodiment disclosed in this disclosure, the nucleic acid molecule comprises the antibody heavy chain variable region nucleotide sequence of the nucleotide sequence SEQ ID NO: 4 or any variant thereof, and/or the light chain variable region nucleotide sequence of the nucleotide sequence SEQ ID NO: 9 or any variant thereof.

In a preferred embodiment disclosed in this disclosure, the nucleic acid molecule comprises the antibody heavy chain variable region nucleotide sequence of the nucleotide sequence SEQ ID NO: 14 or any variant thereof, and/or the light chain variable region nucleotide sequence of the nucleotide sequence SEQ ID NO: 18 or any variant thereof.

In a preferred embodiment disclosed in this disclosure, the nucleic acid molecule comprises the antibody heavy chain variable region nucleotide sequence of the nucleotide sequence SEQ ID NO: 21 or any variant thereof, and/or the light chain variable region nucleotide sequence of the nucleotide sequence SEQ ID NO: 24 or any variant thereof.

In a preferred embodiment disclosed in this disclosure, the nucleic acid molecule comprises the antibody heavy chain variable region nucleotide sequence of the nucleotide sequence SEQ ID NO: 28 or any variant thereof, and/or the light chain variable region nucleotide sequence of the nucleotide sequence SEQ ID NO: 33 or any variant thereof.

In a preferred embodiment disclosed in this disclosure, the nucleic acid molecule comprises the antibody heavy chain variable region nucleotide sequence of the nucleotide sequence SEQ ID NO: 36 or any variant thereof, and/or the light chain variable region nucleotide sequence of the nucleotide sequence SEQ ID NO: 40 or any variant thereof.

In a preferred embodiment disclosed in this disclosure, the nucleic acid molecule comprises the antibody heavy chain variable region nucleotide sequence of the nucleotide sequence SEQ ID NO: 43 or any variant thereof, and/or the light chain variable region nucleotide sequence of the nucleotide sequence SEQ ID NO: 48 or any variant thereof.

In a preferred embodiment disclosed in this disclosure, the nucleic acid molecule comprises the antibody heavy chain variable region nucleotide sequence of the nucleotide sequence SEQ ID NO: 53 or any variant thereof, and/or the light chain variable region nucleotide sequence of the nucleotide sequence SEQ ID NO: 58 or any variant thereof.

In a preferred embodiment disclosed in this disclosure, the nucleic acid molecule comprises the antibody heavy chain variable region nucleotide sequence of the nucleotide sequence SEQ ID NO: 63 or any variant thereof, and/or the light chain variable region nucleotide sequence of the nucleotide sequence SEQ ID NO: 67 or any variant thereof.

In a preferred embodiment disclosed in this disclosure, the nucleic acid molecule comprises the antibody heavy chain variable region nucleotide sequence of the nucleotide sequence SEQ ID NO: 71 or any variant thereof, and/or the light chain variable region nucleotide sequence of the nucleotide sequence SEQ ID NO: 73 or any variant thereof.

In a preferred embodiment disclosed in this disclosure, the nucleic acid molecule comprises the antibody heavy chain variable region nucleotide sequence of the nucleotide sequence SEQ ID NO: 76 or any variant thereof, and/or the light chain variable region nucleotide sequence of the nucleotide sequence SEQ ID NO: 79 or any variant thereof.

In a preferred embodiment disclosed in this disclosure, the nucleic acid molecule comprises the antibody heavy chain variable region nucleotide sequence of the nucleotide sequence SEQ ID NO: 82 or any variant thereof, and/or the light chain variable region nucleotide sequence of the nucleotide sequence SEQ ID NO: 87 or any variant thereof.

In a preferred embodiment disclosed in this disclosure, the nucleic acid molecule comprises the antibody heavy chain variable region nucleotide sequence of the nucleotide sequence SEQ ID NO: 90 or any variant thereof, and/or the light chain variable region nucleotide sequence of the nucleotide sequence SEQ ID NO: 93 or any variant thereof.

In a preferred embodiment disclosed in this disclosure, the nucleic acid molecule comprises the antibody heavy chain variable region nucleotide sequence of the nucleotide sequence SEQ ID NO: 95 or any variant thereof, and/or the light chain variable region nucleotide sequence of the nucleotide sequence SEQ ID NO: 100 or any variant thereof.

In a preferred embodiment disclosed in this disclosure, the nucleic acid molecule comprises the antibody heavy chain variable region nucleotide sequence of the nucleotide sequence SEQ ID NO: 105 or any variant thereof, and/or the light chain variable region nucleotide sequence of the nucleotide sequence SEQ ID NO: 109 or any variant thereof.

In a preferred embodiment disclosed in this disclosure, the nucleic acid molecule comprises the antibody heavy chain variable region nucleotide sequence of the nucleotide sequence SEQ ID NO: 113 or any variant thereof, and/or the light chain variable region nucleotide sequence of the nucleotide sequence SEQ ID NO: 118 or any variant thereof.

In a preferred embodiment disclosed in this disclosure, the nucleic acid molecule comprises the antibody heavy chain variable region nucleotide sequence of the nucleotide sequence SEQ ID NO: 123 or any variant thereof, and/or the light chain variable region nucleotide sequence of the nucleotide sequence SEQ ID NO: 126 or any variant thereof.

In a preferred embodiment disclosed in this disclosure, the nucleic acid molecule comprises the antibody heavy chain variable region nucleotide sequence of the nucleotide sequence SEQ ID NO: 130 or any variant thereof, and/or the light chain variable region nucleotide sequence of the nucleotide sequence SEQ ID NO: 118 or any variant thereof.

In a preferred embodiment disclosed in this disclosure, the nucleic acid molecule comprises the antibody heavy chain variable region nucleotide sequence of the nucleotide sequence SEQ ID NO: 134 or any variant thereof, and/or the light chain variable region nucleotide sequence of the nucleotide sequence SEQ ID NO: 148 or any variant thereof.

In a preferred embodiment disclosed in this disclosure, the nucleic acid molecule comprises the antibody heavy chain variable region nucleotide sequence of the nucleotide sequence SEQ ID NO: 134 or any variant thereof, and/or the light chain variable region nucleotide sequence of the nucleotide sequence SEQ ID NO: 151 or any variant thereof.

In a preferred embodiment disclosed in this disclosure, the nucleic acid molecule comprises the antibody heavy chain variable region nucleotide sequence of the nucleotide sequence SEQ ID NO: 134 or any variant thereof, and/or the light chain variable region nucleotide sequence of the nucleotide sequence SEQ ID NO: 153 or any variant thereof.

In a preferred embodiment disclosed in this disclosure, the nucleic acid molecule comprises the antibody heavy chain variable region nucleotide sequence of the nucleotide sequence SEQ ID NO: 137 or any variant thereof, and/or the light chain variable region nucleotide sequence of the nucleotide sequence SEQ ID NO: 148 or any variant thereof.

In a preferred embodiment disclosed in this disclosure, the nucleic acid molecule comprises the antibody heavy chain variable region nucleotide sequence of the nucleotide sequence SEQ ID NO: 137 or any variant thereof, and/or the light chain variable region nucleotide sequence of the nucleotide sequence SEQ ID NO: 151 or any variant thereof.

In a preferred embodiment disclosed in this disclosure, the nucleic acid molecule comprises the antibody heavy chain variable region nucleotide sequence of the nucleotide sequence SEQ ID NO: 137 or any variant thereof, and/or the light chain variable region nucleotide sequence of the nucleotide sequence SEQ ID NO: 153 or any variant thereof.

In a preferred embodiment disclosed in this disclosure, the nucleic acid molecule comprises the antibody heavy chain variable region nucleotide sequence of the nucleotide sequence SEQ ID NO: 139 or any variant thereof, and/or the light chain variable region nucleotide sequence of the nucleotide sequence SEQ ID NO: 148 or any variant thereof.

In a preferred embodiment disclosed in this disclosure, the nucleic acid molecule comprises the antibody heavy chain variable region nucleotide sequence of the nucleotide sequence SEQ ID NO: 139 or any variant thereof, and/or the light chain variable region nucleotide sequence of the nucleotide sequence SEQ ID NO: 151 or any variant thereof.

In a preferred embodiment disclosed in this disclosure, the nucleic acid molecule comprises the antibody heavy chain variable region nucleotide sequence of the nucleotide sequence SEQ ID NO: 139 or any variant thereof, and/or the light chain variable region nucleotide sequence of the nucleotide sequence SEQ ID NO: 153 or any variant thereof.

In a preferred embodiment disclosed in this disclosure, the nucleic acid molecule comprises the antibody heavy chain variable region nucleotide sequence of the nucleotide sequence SEQ ID NO: 141 or any variant thereof, and/or the light chain variable region nucleotide sequence of the nucleotide sequence SEQ ID NO: 155 or any variant thereof.

In a preferred embodiment disclosed in this disclosure, the nucleic acid molecule comprises the antibody heavy chain variable region nucleotide sequence of the nucleotide sequence SEQ ID NO: 141 or any variant thereof, and/or the light chain variable region nucleotide sequence of the nucleotide sequence SEQ ID NO: 157 or any variant thereof.

In a preferred embodiment disclosed in this disclosure, the nucleic acid molecule comprises the antibody heavy chain variable region nucleotide sequence of the nucleotide sequence SEQ ID NO: 141 or any variant thereof, and/or the light chain variable region nucleotide sequence of the nucleotide sequence SEQ ID NO: 159 or any variant thereof.

In a preferred embodiment disclosed in this disclosure, the nucleic acid molecule comprises the antibody heavy chain variable region nucleotide sequence of the nucleotide sequence SEQ ID NO: 144 or any variant thereof, and/or the light chain variable region nucleotide sequence of the nucleotide sequence SEQ ID NO: 155 or any variant thereof.

In a preferred embodiment disclosed in this disclosure, the nucleic acid molecule comprises the antibody heavy chain variable region nucleotide sequence of the nucleotide sequence SEQ ID NO: 144 or any variant thereof, and/or the light chain variable region nucleotide sequence of the nucleotide sequence SEQ ID NO: 157 or any variant thereof.

In a preferred embodiment disclosed in this disclosure, the nucleic acid molecule comprises the antibody heavy chain variable region nucleotide sequence of the nucleotide sequence SEQ ID NO: 144 or any variant thereof, and/or the light chain variable region nucleotide sequence of the nucleotide sequence SEQ ID NO: 159 or any variant thereof.

In a preferred embodiment disclosed in this disclosure, the nucleic acid molecule comprises the antibody heavy chain variable region nucleotide sequence of the nucleotide sequence SEQ ID NO: 146 or any variant thereof, and/or the light chain variable region nucleotide sequence of the nucleotide sequence SEQ ID NO: 155 or any variant thereof.

In a preferred embodiment disclosed in this disclosure, the nucleic acid molecule comprises the antibody heavy chain variable region nucleotide sequence of the nucleotide sequence SEQ ID NO: 146 or any variant thereof, and/or the light chain variable region nucleotide sequence of the nucleotide sequence SEQ ID NO: 157 or any variant thereof.

In a preferred embodiment disclosed in this disclosure, the nucleic acid molecule comprises the antibody heavy chain variable region nucleotide sequence of the nucleotide sequence SEQ ID NO: 146 or any variant thereof, and/or the light chain variable region nucleotide sequence of the nucleotide sequence SEQ ID NO: 159 or any variant thereof.

In one aspect, this disclosure provides an antibody or antigen-binding portion thereof that binds to CD70 that has at least greater than 60%, 65%, 70%, 75%, 80%, 85%, 90%, 95%, 96%, 97%, 98%, 99% or more sequence identity to the antibody or antigen-binding portion thereof of any of the previous aspects.

In one aspect, the present disclosure provides a nucleic acid molecule encoding the antibody or antigen-binding portion thereof according to any one of the previous aspects, or a nucleic acid molecule having at least greater than 60%, 65%, 70%, 75%, 80%, 85%, 90%, 95%, 96%, 97%, 98%, 99% or more sequence identity thereto.

In one aspect, the present disclosure provides a vector comprising the nucleic acid molecule of any one of the previous aspects.

In one aspect, the present disclosure provides a cell comprising the vector of any one of the previous aspects.

In one aspect, the present disclosure provides a composition comprising the aforementioned antibody or antigen-binding portion thereof, bispecific or multispecific molecule, nucleic acid molecule, claimed vector, and/or cell.

The antibody of the present disclosure is used as a therapeutic or diagnostic tool for various diseases in which CD70 is unfavorably expressed or found.

In an embodiment of a CD70-associated disease, the expression of CD70 is increased in cells of a diseased tissue or organ compared to the state in a healthy tissue or organ. Increase refers to an increase of at least 10%, especially at least 20%, at least 50%, at least 100%, at least 200%, at least 500%, at least 1000%, at least 10000%, or even more. In an embodiment, expression is found in a diseased tissue only, and the expression in a corresponding healthy tissue is depressed.

CD70 is expressed in a variety of human cancers, including renal cell carcinoma, metastatic breast cancer, brain tumors, leukemias, lymphomas, and nasopharyngeal carcinoma. The anti-CD70 antibody can be used alone to inhibit the growth of cancerous tumors. Alternatively, as described below, the anti-CD70 can be used with other immunogenic agents, standard cancer therapies, or other antibodies.

Preferred cancers whose growth can be inhibited by the antibody of the present disclosure include cancers that generally respond to immunotherapy. Non-limiting examples of preferred cancers that can be treated include renal cancer (e.g. renal cell carcinoma), breast cancer, brain tumors, chronic or acute leukemias including acute myelogenous leukemia, chronic myelogenous leukemia, acute lymphoblastic leukemia, chronic lymphocytic leukemia, lymphomas (e.g. Hodgkin's and non-Hodgkin's lymphoma, lymphocytic lymphoma, primary CNS lymphoma, T-cell lymphoma), and nasopharyngeal carcinoma. Examples of other cancers that can be treated by the method of the present disclosure include melanoma (e.g. metastatic malignant melanoma), prostate cancer, colon cancer, lung cancer, bone cancer, pancreatic cancer, skin cancer, head and neck cancer, skin or intraocular malignant melanoma, uterine cancer, ovarian cancer, rectal cancer, anal region cancer, gastric Cancer, testicular cancer, uterine cancer, fallopian tube cancer, endometrial cancer, cervical cancer, vaginal cancer, vulval cancer, esophageal cancer, small intestine cancer, endocrine system cancer, thyroid cancer, parathyroid cancer, adrenal cancer, soft tissue sarcoma, urethral cancer, penile cancer, solid tumors in children, bladder cancer, renal or ureteral cancer, renal pelvis cancer, central nervous system (CNS) tumors, tumor angiogenesis, spinal tumors, brain stem gliomas, pituitary adenomas, Kaposi's sarcoma, epidermoid cancer, squamous cell cancer, environmentally induced cancers including asbestos induced cancers such as mesothelioma, and combinations of cancers.

Furthermore, in view of the expression of CD70 on a variety of tumor cells, the human antibody, antibody composition and methods of the present disclosure can be used to treat a subject suffering from a tumorigenic disease, e.g. a disease characterized by the presence of CD70-expressing tumor cells, including, for example, renal cell carcinoma (RCC), such as clear cell RCC, glioblastoma, breast cancer, brain tumors, nasopharyngeal carcinoma, non-Hodgkin's lymphoma (NHL), acute lymphocytic leukemia (ALL), chronic lymphocytic leukemia (CLL), Burkitt's lymphoma, anaplastic large cell lymphoma (ALCL), multiple myeloma, cutaneous T-cell lymphoma, nodular small cleaved cell lymphoma, lymphocytic lymphoma, peripheral T-cell lymphoma, Lennert's lymphoma, immunoblastic lymphoma, T-cell leukemia/lymphoma (ATLL), adult T-ALL, centroblastic/centrocytic (cb/cc) follicular lymphoma, B-lineage diffuse large cell lymphoma, angioimmunoblastic lymphadenopathy (AILD)-like T-cell lymphoma, HIV-associated body cavity lymphoma, embryonal carcinoma, undifferentiated nasopharyngeal carcinoma (e.g. Schmincke's tumor), Castleman's disease, Kaposi's sarcoma, multiple myeloma, Waldenstrom's macroglobulinemia, and B-cell lymphoma.

In addition, the interaction of CD70 with CD27 has also been suggested to play a role in cell-mediated autoimmune diseases such as experimental autoimmune encephalomyelitis (EAE). This effect is believed to be mediated, in part, by inhibition of TNF-a production. Moreover, blockade of the CD70 signal inhibits CD40-mediated clonal expansion of CD8[+] T-cells and reduces the production of CD8[+] memory T-cells (Taraban et al., (2004)J.Immunol.173: 6542-6). As such, the human antibody, antibody composition, and method of the present disclosure can be used to treat patients suffering from autoimmune diseases, such as diseases characterized by the presence of CD70-expressing B cells, including, for example, experimental autoimmune encephalomyelitis. Other autoimmune diseases in which the antibodies of the disclosure can be used include, but are not limited to, systemic lupus erythematosus (SLE), insulin-dependent diabetes mellitus (IDDM), inflammatory bowel disease (IBD) including Crohn's disease, ulcerative colitis, and celiac disease, multiple sclerosis (MS), psoriasis, autoimmune thyroiditis, rheumatoid arthritis (RA), and glomerulonephritis. In addition, the antibody compositions of the present disclosure can be used to inhibit or prevent transplant rejection or to treat graft-versus-host (GVHD).

In addition, the interaction of CD70 with CD27 has also been suggested to play a role in CD4+ T cell signaling. Certain viruses have been shown to signal the CD27 pathway, resulting in disruption of the neutralizing antibody response (Matter et al. (2006)J Exp Med 203: 2145-55). As such, the human antibody, antibody composition, and method of the present disclosure can be used to treat patients with viral infections, including, for example, human immunodeficiency virus (HIV), hepatitis (A, B, C), herpes viruses (e.g. VZV, HSV-1, HAV-6, HSV-II, and CMV, Epstein-Barr virus), adenovirus, influenza virus, arbovirus, echovirus, rhinovirus, coxsackie virus, coronavirus, respiratory syncytial virus, mumps virus, rotavirus, measles virus, rubella virus, parvovirus, vaccinia virus, HTLV virus, dengue virus, papilloma virus, molluscum virus, polio virus, rabies virus, JC virus and arbovirus encephalitis virus and lymphocytic choriomeningitis virus (LCMV), or for the treatment of HIV infections/AIDS. In addition, the human antibody, antibody composition, and method of the present disclosure can be used to inhibit TNF-alpha production.

A method for treating a disease or disorder with the CD70 antibody of the present disclosure includes the following steps: administering a therapeutically effective amount of antibody or antigen-binding fragment thereof or nucleic acid molecule or vector or cell or pharmaceutical composition of any one of the aforementioned aspects to a subject.

In some embodiments, the present disclosure provides a method for treating or preventing cancer, which includes the following steps: an antibody that can bind to CD70 is administered to a patient, so as to provide a serum level of at least 40 $\mu$g/mL. In different embodiments, the antibody is administered to provide at a serum level of at least 50 $\mu$g/mL, at least 150 $\mu$g/mL, at least 300 $\mu$g/mL, at least 400 $\mu$g/mL, or at least 500 $\mu$g/mL. In different embodiments, the antibody is administered to provide a serum level of no more than 800 $\mu$g/mL, 700 $\mu$g/mL, 600 $\mu$g/mL, 550 $\mu$g/mL, or 500 $\mu$g/mL. In an embodiment, the provided serum level is 40 $\mu$g/mL to 700 $\mu$g/mL, preferably 40 $\mu$g/mL to 600 $\mu$g/mL, preferably 50 $\mu$g/mL to 500 $\mu$g/mL, such as 150 $\mu$g/mL to 500 $\mu$g/mL and 300 $\mu$g/mL to 500 $\mu$g/mL. The term "serum level" as used in this description refers to the concentration of the substance in question in the serum. In an embodiment, the serum level is provided for at least 7 days or at least 14 days. In an embodiment, the method includes administering the antibody in a dose of at least 300 mg/m$^2$, such as at least 600 mg/m$^2$, preferably up to 1500 mg/m$^2$, up to 1200 mg/m$^2$, or up to 1000 mg/m$^2$.

In some embodiments, the present disclosure provides a method for treating or preventing cancer, which includes administering to a patient an antibody that can bind to CD70 in a dose of at least 300 mg/m$^2$, such as at least 600 mg/m$^2$, and preferably up to 1500 mg/m$^2$, up to 1200 mg/m$^2$ or up to 1000 mg/m$^2$.

In some embodiments, the disclosure provides a method of treating or preventing a cancer disease comprising administering an antibody that can bind to CD70 to a patient, wherein at least 50%, preferably 60%, 70%, 80%, or 90% of the cancer cells of the patient are positive for CD70 and/or at least 40%, preferably 50% or 60% of the cancer cells of the patient are positive for surface expression of CD70. In this aspect, the present disclosure also provides a method for treating or preventing cancer, which includes the following steps: a. identifying a patient showing at least 50%, preferably 60%, 70%, 80%, or 90%, of CD70-positive cancer cells and/or at least 40%, preferably 50% or 60%, of cancer cells, the cancer cells are positive for the surface expression of CD70; and b. administering an antibody binding to CD70 to the patient. In an embodiment, at least 95% or at least 98% of cancer cells in the patient are positive for CD70. In an embodiment, at least 70%, at least 80%, or at least 90% of cancer cells in the patient are positive for the surface expression of CD70.

In an embodiment of the method of any aspect described herein, the outcome of cancer treatment is to achieve disease stabilization. In an embodiment, the disease stabilization is achieved for at least 2 months, at least 3 months, or at least 6 months.

In some embodiments, the present disclosure provides a method for achieving the disease stabilization of a cancer patient, which includes administering an antibody that can bind to CD70 to a patient. In an embodiment, the disease stabilization is achieved for at least 2 months, at least 3 months, or at least 6 months.

In an embodiment of the method of any aspect described herein, the antibody is administered in a single dose or multiple doses.

In some embodiments, the present disclosure provides a method for treating or preventing cancer, which includes administering an antibody that can bind to CD70 to a patient in multiple doses.

In a case that the antibody is administered in multiple doses according to the present disclosure, the antibody is administered in preferably at least 3 doses, at least 4 doses, at least 5 doses, at least 6 doses, at least 7 doses, at least 8 doses, at least 9 doses or at least 10 doses, and preferably up to 30 doses, 25 doses, 20 doses, 15 doses or 10 doses. Doses of the antibody are preferably administered at intervals of at least 7 days, at least 10 days, at least 14 days, or at least 20 days. Doses of the antibody are preferably administered at intervals of 7 to 30 days or 10 to 20 days, preferably about 14 days.

In an embodiment, the antibody is administered to provide a serum level of at least 40 $\mu$g/mL. In different embodiments, the antibody is administered to provide at a serum level of at least 50 $\mu$g/mL, at least 150 $\mu$g/mL, at least 300 $\mu$g/mL, at least 400 $\mu$g/mL, or at least 500 $\mu$g/mL. In different embodiments, the antibody is administered to provide a serum level of no more than 800 $\mu$g/mL, 700 $\mu$g/mL, 600 $\mu$g/mL, 550 $\mu$g/mL, or 500 $\mu$g/mL. In one embodiment, the serum level provided is from 40 $\mu$g/ml to 700 $\mu$g/ml, preferably from 40 $\mu$g/ml to 600 $\mu$g/ml, preferably from 50 $\mu$g/ml to 500 $\mu$g/ml, such as from 150 $\mu$g/ml to 500 $\mu$g/ml or from 300 $\mu$g/ml to 500 $\mu$g/ml. In an embodiment, the serum level is provided for at least 7 days or at least 14 days. In one embodiment, the method comprises administering the antibody in a dose of at least 300 mg/m$^2$, such as at least 600 mg/m$^2$ and preferably at most 1500 mg/m$^2$, at most 1200 mg/m$^2$, or at most 1000 mg/m$^2$.

Use of the antibody or the antigen-binding fragment thereof or the nucleic acid or the vector or the cell or the pharmaceutical composition of any one of the aforementioned aspects in the preparation of a medicament for treating a CD70-associated condition in a subject is provided.

According to any one of the aforementioned aspects, optionally, the antibody is conjugated to other medicaments such as a labeled or cytotoxic conjugate.

In one aspect, the present disclosure provides a kit comprising the aforementioned antibody or antigen-binding portion thereof, bispecific or multispecific molecule, nucleic acid molecule, vector, cell, composition, and/or antibody-drug conjugate.

The kit disclosed in this disclosure includes the antibody or a fragment or homolog or derivative thereof of the present disclosure, such as a labeled or cytotoxic conjugate, antibody instructions, a conjugate that kills specific types of cells, etc. The instructions may include directions for using the antibody, conjugate, etc. in vitro, in vivo, or ex vivo. The antibody may be in the form of a liquid or solid and is usually lyophilized. This kit may contain other appropriate reagents, such as a buffer, a reconstitution solution, and other components necessary for the intended use. A reagent combination packaged in predetermined doses and instructions for its use are contemplated, and the use is, for example, therapeutic use or diagnostic assay. In the case that the antibody is labeled with, for example, an enzyme, the kit may include a substrate and a cofactor required for the enzyme (e.g., a substrate precursor that provides a detectable chromophore or fluorophore). In addition, other additives, such as a stabilizer and a buffer (e.g., a blocking buffer and a lysis buffer), may be included. The relative amounts of various reagents can be varied such that a concentrate of a reagent solution is provided, which achieves user flexibility, space savings, reagent savings, etc. These reagents may also be provided in the form of dry powder, usually in a lyophilized form, including an excipient, which can provide a reagent solution at an appropriate concentration when dissolved.

In one aspect, the present disclosure provides the use of the aforementioned antibody or antigen-binding portion, bispecific or multispecific molecule, nucleic acid molecule, vector, cell, composition, and/or antibody-drug conjugate in the preparation of a medicament or kit for diagnosis, treatment or prevention of a CD70-associated condition. In some embodiments, the CD70-associated condition includes the tumor and the autoimmune disease.

In some preferred embodiments, the tumor is a cancer disease; preferably, the cancer disease is selected from the group consisting of renal cell carcinoma (RCC), clear cell RCC, glioblastoma, non-Hodgkin's lymphoma (NHL), acute lymphocytic leukemia (ALL), chronic lymphocytic leukemia (CLL), Burkitt's lymphoma, anaplastic large cell lymphoma (ALCL), multiple myeloma, cutaneous T-cell lymphoma, nodular small cleaved cell lymphoma, lymphocytic lymphoma, peripheral T-cell lymphoma, Lennert's lymphoma, immunoblastic lymphoma, T-cell leukemia/lymphoma (ATLL), adult T-ALL, centroblastic/centrocytic (cb/cc) follicular lymphoma, B-lineage diffuse large cell lymphoma, angioimmunoblastic lymphadenopathy (AILD)-like T-cell lymphoma, HIV-associated body cavity lymphoma, embryonal carcinoma, undifferentiated nasopharyngeal carcinoma, Schmincke's tumor, Castleman's disease, Kaposi's sarcoma, multiple myeloma, Waldenstrom's macroglobulinemia, and B-cell lymphoma.

In some preferred embodiments, the autoimmune disease is lupus.

In one aspect, the present disclosure provides the use of the aforementioned antibody or antigen-binding portion thereof, bispecific or multispecific molecule, nucleic acid molecule, vector, cell, composition, and/or antibody-drug conjugate in the manufacture of a medicament or kit for treating a viral infection in a subject.

In some preferred embodiments, the viral infection is an infection selected from the group consisting of human immunodeficiency virus (HIV), hepatitis (A, B, C), herpes viruses (e.g. VZV, HSV-1, HAV-6, HSV-II and CMV, E-B virus), adenovirus, influenza virus, arbovirus, echovirus, rhinovirus, coxsackie virus, coronavirus, respiratory syncytial virus, mumps virus, rotavirus, measles virus, rubella virus, parvovirus, vaccinia virus, HTLV virus, dengue virus, papilloma virus, molluscum virus, polio virus, rabies virus, JC virus and arbovirus encephalitis virus, and lymphocytic choriomeningitis virus (LCMV).

In addition, the antibody of the present disclosure can also be used for immunoassay, a purification method, and other methods using immunoglobulins or fragments thereof. Such use is well-known to those skilled in the art.

Correspondingly, the present disclosure also provides a composition containing the anti-CD70 antibody or the fragment thereof of the present disclosure, and the antibody is conveniently combined with a pharmaceutically acceptable carrier, a diluent or an excipient, as is common practice in the art.

The term "pharmaceutical composition" refers to the preparation of various preparations. A preparation containing a therapeutically effective amount of polyvalent antibody is a sterile liquid solution, liquid suspension, or lyophilized form, optionally containing a stabilizer or excipient.

The antibody of the present disclosure may be used as a composition that is administered alone, or used in combination with other active agents.

In some embodiments, the humanized antibody of the present disclosure is conjugated to a therapeutic portion (i.e., a drug). The therapeutic portion may be, for example, a cytotoxin, a chemotherapeutic agent, a cytokine, an immunosuppressive drug, an immunostimulant, a lytic peptide, or a radioisotope. Such a conjugate is referred to as an "antibody-drug conjugate" or "ADC" herein.

In some embodiments, the antibody is conjugated to a cytotoxic portion. The cytotoxic portion can be selected from the group consisting of, for example, paclitaxel; cytochalasin B; gramicidin D; ethidium bromide; emetine; mitomycin; etoposide; teniposide; vincristine; vinblastine; colchicine; doxorubicin; daunorubicin; dihydroxy anthracenedione; a tubulin inhibitor such as maytansine and an analog or derivative thereof; an antimitotic agent such as monomethyl auristatin E and F and analogs or derivatives thereof; hareotoxin 10 and 15 and analogs thereof; irinotecan and an analog thereof; mitoxantrone; mithramycin; actinomycin D; 1-dehydrotestosterone; a glucocorticoid; procaine; tetracaine; lidocaine; propranolol; puromycin; calicheamicin and an analog or derivative thereof; an anti-metabolite such as methotrexate, 6-mercaptopurine, 6-thioguanine, cytarabine, fludarabine, 5-fluorouracil, decadiazine, hydroxyurea, asparaginase, gemcitabine, and cladribine; an alkylating agent such as dichloromethyldiethylamine, thiopurine, chlorambucil, melphalan, carmustine (BSNU), lomustine (CCNU), cyclophosphamide, busulfan, mitobronitol, streptozotocin, dacarbazine (DTIC), procarbazine, and mitomycin C; a platinum derivative such as cisplatin or carboplatin; duocarmycin A, duocarmycin SA, rapamycin (CC-1065), and analogs or derivatives thereof; an antibiotic such as actinomycin, bleomycin, daunorubicin, doxorubicin, idarubicin, mithramycin, mitomycin, mitoxantrone, perimycin, and anthramycin (AMC); pyrrolo[2,1-c][1,4]-benzodiazepine (PDB); diphtheria toxin and related molecules such as diphtheria A chain and an active fragment and a hybrid molecule thereof, ricin such as ricin A and deglycosylated ricin A chain toxin, cholera toxin, a Shiga-like toxin such as SLT I, SLT II and SLT IIV, LT toxin, C3 toxin, Shiga toxin, pertussis toxin, tetanus toxin, soybean Bowman-Birk protease inhibitor, Pseudomonas exotoxin, alorin, saporin, modeccin, gelsolin, abrin A chain, modeccin A chain, α-sarcin, Aleurites fordii protein, caryophyllin protein, Phytolacca americana protein such as PAPI, PAPII and PAP-S, Momordica charantia inhibitor, curcin, crotin, Sapaonaria officinalis inhibitor, gelonin, mitomycin, restrictocin, phenomycin, and enomycin toxin; ribonuclease (RNase); DNase I and Staphylococcus endotoxin A; pokeweed antiviral protein; and diphtheria toxin and Pseudomonas endotoxin.

In some embodiments, the antibody is conjugated to an auristatin or a peptide analog, derivative or prodrug thereof. It has been shown that auristatins interfere with microtubule dynamics, GTP hydrolysis, and nuclear and cell division, and have anticancer and antifungal activity. For example, auristatin E can react with p-acetylbenzoic acid or benzoylvaleric acid to respectively produce AEB and AEVB. Other typical auristatin derivatives include AFP, monomethyl auristatin F (MMAF), and monomethyl auristatin E (MMAE). Appropriate auristatins and analogs, derivatives, and prodrugs thereof, as well as an appropriate adaptor for conjugating auristatins to Ab are described in, for example, U.S. patent NO. 5,635,483, 5,780,588 and 6,214,345, as well as international patent application publications WO02088172, WO2004010957, WO2005081711, WO2005084390, WO2006132670, WO03026577, WO200700860, WO207011968 and WO205082023.

In some embodiments, the antibody is conjugated to pyrrolo[2,1-c][1,4]-benzodiazepine (PDB) or a peptide analog, derivative or prodrug thereof. Suitable PDB and PDB derivatives and related art are described, for example, in Hartley J.A.et al., Cancer Res 2010; 70(17):6849-6858; Antonow D. et al., Cancer J 2008; 14(3):154-169; Howard P.W. et al., Bioorg Med Chem Lett 2009; 19:6463-6466 and Sagnou et al, Bioorg MedChem Lett 2000; 10(18):2083-2086.

In some embodiments, the antibody is conjugated to a cytotoxic portion of an anthracycline, maytansine, a calicheamicin, duocarmycin, rapamycin (CC-1065), aplysiatoxin 10, aplysiatoxin 15, irinotecan, monomethyl auristatin E, monomethyl auristatin F, PDB, or any analog, derivative or prodrug thereof.

In some embodiments, the antibody is conjugated to an anthracycline or an analog, derivative or prodrug thereof. In some embodiments, the antibody is conjugated to maytansine or an analog, derivative or prodrug thereof. In some embodiments, the antibody is conjugated to a calicheamicin or an analog, derivative or prodrug thereof. In some embodiments, the antibody is conjugated to duocarmycin or an analog, derivative or prodrug thereof. In some embodiments, the antibody is conjugated to rapamycin (CC-1065) or an analog, derivative or prodrug thereof. In some embodiments, the antibody is conjugated to aplysiatoxin 10 or an analog, derivative or prodrug thereof. In some embodiments, the antibody is conjugated to aplysiatoxin 15 or an analog, derivative or prodrug thereof. In some embodiments, the antibody is conjugated to monomethyl auristatin E or an analog, derivative or prodrug thereof. In some embodiments, the antibody is conjugated to monomethyl auristatin F or an analog, derivative or prodrug thereof. In some embodiments, the antibody is conjugated to pyrrolo[2,1-c][1,4]-benzodiazepine or an analog, derivative or prodrug thereof. In some embodiments, the antibody is conjugated to irinotecan or an analog, derivative or prodrug thereof.

In some embodiments, the antibody is conjugated to a cytokine (e.g. IL-2, IL-4, IL-6, IL-7, IL-10, IL-12, IL-13, IL-15, IL-18, IL-23, IL-24, IL-27, IL-28a, IL-28b, IL-29, KGF, IFN α, IFN β, IFN γ, GM-CSF, CD40L, Flt3 ligand, stem cell factor, ancestine, and TNF alpha). In some embodiments, the antibody is conjugated to a radioisotope or a chelate containing a radioisotope. For example, the antibody may be conjugated to a chelate adaptor (e.g., DOTA, DTPA, and thiacetam) that allows the antibody to be complexed with the radioisotope. The antibody may also or optionally contain one or more radiolabeled amino acids or other radiolabeled molecules or is conjugated to the radiolabeled amino acids or radiolabeled molecules. Non-limiting examples of radioisotopes include $^{3}$H, $^{14}$C, $^{15}$N, $^{35}$S, $^{90}$Y, $^{99}$Tc, $^{125}$I, $^{131}$I, $^{186}$Re,

$^{213}$Bi, $^{225}$Ac and $^{227}$Th. For therapeutic purposes, radioisotopes emitting beta or alpha particle radiation may be used, such as $^{131}$I, $^{90}$Y, $^{211}$At, $^{212}$Bi, $^{67}$Cu, $^{186}$Re, $^{188}$Re and $^{212}$Pb.

The technologies for conjugating molecules to antibodies are well-known in the art. Usually, a nucleic acid molecule is covalently linked to lysine or cysteine on an antibody through an N-hydroxysuccinimide eater or maleimide functional group. It is reported that the homogeneity of a conjugate can be improved by conjugation methods using engineered cysteine or integrating unnatural amino acids. Particularly, those skilled in the art can also expect to use a tag (e.g., a tag containing Gln peptide and a Q-tag) containing glutamine serving as an acyl donor or polypeptide engineering (e.g., amino acid deletions, insertions, substitutions and mutations on a polypeptide) to generate reactive endogenous glutamine engineered Fc-containing polypeptide. Then, a transglutaminase can be covalently cross-linked with an amine donor agent (e.g., a small molecule containing or linked to a reactive amine) to form a population of stable and homogeneous engineered Fc-containing polypeptide conjugates in which the amine donor agent is specifically conjugated to the Fc-containing polypeptide through the tag containing glutamine serving as an acyl donor tag or accessible/exposed/reactive endogenous glutamine sites (WO2012059882).

It should be understood that therapeutic agents according to the described embodiments will be administered with suitable pharmaceutically acceptable carriers, excipients, and other reagents incorporated into the preparation to provide improved transfer, delivery, tolerability, etc. A large number of appropriate preparations can be found in the pharmacopeia known to all medicinal chemists: Remington's Pharmaceutical Sciences (the 15th edition, Mack Publishing Company, Easton, Pa. (1975)), especially Chapter 87 of Blaug and Seymour therein. These preparations include, for example, powders, pastes, ointments, gels, waxes, oils, lipids, lipid-containing (cation or anion) carriers (such as Lipofectin™), DNA conjugates, anhydrous slurries, oil-in-water and water-in-oil emulsions, emulsion polyethylene glycols (polyethylene glycols of various molecular weights), semisolid gels, and semisolid mixtures containing polyethylene glycol. Any of the above mixtures is applicable to the treatment or therapy according to the present disclosure under the conditions that an active ingredient of the preparation is not inactivated by the preparation, and the preparation is physiologically compatible and tolerates the route of administration.

In an embodiment, the antibody can be used as a therapeutic agent. Such a therapeutic agent is usually used for treating, alleviating and/or preventing a disease or pathology associated with aberrant CD70 expression, activity and/or signaling in a subject. A treatment regimen can be implemented using standard methods by identifying a subject, such as a human patient suffering from (or at risk or developing) CD70-associated conditions such as a disease or disorder associated with aberrant CD70 expression, activity and/or signaling. An antibody preparation, preferably an antibody preparation with high specificity and high affinity for its target antigen, is administered to a subject and will generally produce an effect due to its binding to the target. The administered antibody can eliminate or inhibit or hinder the expression, activity and/or signal transduction function of the target (e.g., CD70). The administered antibody can eliminate or inhibit or hinder the binding of the target (e.g., CD70) to an endogenous ligand to which it naturally binds. For example, the antibody binds to the target and regulates, blocks, inhibits, reduces, antagonizes, neutralizes and/or hinders the expression, activity and/or signal transduction of CD70 in other ways. In some embodiments, in order to treat an abnormal CD70 expression-associated disease or disorder, an antibody having a heavy chain and light chain CDRs can be administered to a subject.

In another embodiment, antibodies against CD70 can be used in methods known in the art related to CD70 localization and/or quantification (e.g., measurement of CD70 and/or a CD70 level in an appropriate physiological sample, diagnostic methods, and protein imaging). In a given embodiment, an antibody having the specificity for CD70 or a derivative, fragment, analog or homolog thereof and containing an antigen-binding domain derived from the antibody is used as a pharmaceutically active compound (referred to as a "therapeutic agent" hereinafter).

In another embodiment, an antibody having the specificity for CD70 is used for separating CD70 polypeptide through a standard technology such as immunoaffinity, chromatography and immunoprecipitation. An antibody (or a fragment thereof) against CD70 protein can be used for detecting proteins in a biological sample. In some embodiments, the detection of CD70 in a biological sample can be used as part of a clinical testing procedure, for example, used for determining the efficacy of a given treatment regimen. Detection can be facilitated by conjugating (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, and acetylcholinesterase; examples of suitable prosthetic groups include streptavidin/biotin and avidin/biotin; examples of suitable fluorescent materials include umbelliferone, fluorescein, fluorescein isothiocyanate, rhodamine, dichlorotriazine fluorescein, dansyl chloride, and phycoerythrin; an example of the luminescent material includes luminol; examples of the bioluminescent materials include luciferase, fluorescein and aequorin, and examples of suitable radioactive materials include $^{125}$I, $^{131}$I, $^{35}$S or $^{3}$H.

In another embodiment, the antibody of the present disclosure can be used as a reagent for detecting the presence of CD70 or a protein fragment thereof in a sample. In some embodiments, the antibody contains a detectable label. The antibody is a polyclonal antibody, or more preferably a monoclonal antibody. The whole antibody or a fragment (e.g., Fab, scFv, and F(ab')$_2$) is used. The term "label" with respect to an antibody is intended to include direct labeling of the antibody by conjugating (i.e., physically linking) a detectable substance to the antibody, and indirect labeling of the antibody by reaction with another directly labeled reagent. Examples of indirect labeling include detection of a primary antibody with a fluorescently-labeled secondary antibody, and end-labeling of the antibody with biotin to enable detection with fluorescently-labeled streptavidin. The term "biological sample" is intended to include tissues, cells, and biological fluids separated from a subject, as well as tissues, cells, and fluids present in the subject. Therefore, the term "biological sample" used includes blood and fractions or components of blood, including serum, plasma, and lymph. In other words, the detection method of the embodiment can be used to detect an analyte mRNA, protein or genomic DNA in a biological sample both in vitro and in vivo. For example, the analyte mRNA in-vitro detection technologies include Northern hybridization and in situ hybridization. The analyte protein in-vitro detection technologies include enzyme-linked immunosorbent assay (ELISA), Western blotting, immunoprecipitation, and immunofluorescence. The analyte genomic DNA in-vitro detection technologies include Southern hybridization. In addition, the analyte protein in-vivo detection technologies include the introduction of a labeled anti-analyte protein antibody into a subject. For example, the antibody may be labeled with a radioactive label, and the presence and position of the radioactive label in the subject are detected by standard imaging technology.

The antibody or the derivative, fragment, analog and homolog thereof described herein can be incorporated into a pharmaceutical composition suitable for administration. The principles and considerations involved in the preparation of such a composition and guidance for selecting components are well-known in the art. See, e.g. Remington's Pharmaceutical Sciences: The Science And Practice Of Pharmacy 19th edition (edited by Alfonso R. Gennaro et al.) Mack Pub. Co., Easton, Pa.: 1995.

Such a composition usually contains an antibody and a pharmaceutically acceptable carrier. In the case that an antibody fragment is used, the minimum inhibitory fragment that specifically binds to a binding domain of a target protein may be preferred. For example, based on the variable region of the antibody, a peptide molecule that retains the ability to bind to the sequence of the target protein can be designed. Such a peptide can be chemically synthesized and/or by DNA recombination (referring to, for example, Marasco, et al., Proc. Natl. Acad. Sci. USA, 90:7889-7893(1993)).

As used herein, the term "pharmaceutically acceptable carrier" is intended to include any and all solvents, dispersion media, coatings, antibacterial agents and antifungal agents, isotonic agents and absorption delaying agents, etc., compatible with pharmaceutical administration. Suitable pharmaceutically acceptable carriers are described in the latest edition of Remington's Pharmaceutical Sciences, which is a standard reference work in the art and incorporated herein by reference. Preferred examples of such carriers or diluents include but are not limited to, water, saline, Ringer's solution, dextrose solution, and 5% human serum albumin. Liposomes and non-aqueous carriers, such as immobilized oils, can also be used. The use of such media and reagents for pharmaceutically active substances is well-known in the art. Except for their incompatibility with the antibody, use of any conventional medium or reagent in the composition is envisioned.

The pharmaceutical composition of the aforementioned embodiment is prepared to be compatible with its intended route of administration. Examples of routes of administration include parenteral administration, such as intravenous, intradermal, subcutaneous, oral (e.g., inhalation), transdermal (i.e., topical), transmucosal and rectal administration. A solution or suspension for parenteral, intradermal, or subcutaneous administration may include the following components: a sterile diluent for injection such as water, a saline solution, a fixed oil, polyethylene glycol, glycerin, propylene glycol, and other synthesized solvent; an antibacterial agent such as benzyl alcohol and methylparaben; an antioxidant such as ascorbic acid and sodium bisulfite; a chelating agent such as ethylenediaminetetraacetic acid (EDTA); a buffer such as acetate, citrate, and phosphate; and a reagent that regulates osmotic pressure such as sodium chloride and dextrose. The pH can be regulated with an acid or base, such as hydrochloric acid and sodium hydroxide. A parenteral administration may be packaged into an ampoule, a disposable syringe, or a glass or plastic multi-dose vial.

A pharmaceutical composition suitable for injection includes sterile aqueous solutions (water-soluble herein) or dispersions and sterile powders for the extemporaneous preparation of sterile injectable solutions or dispersions. For intravenous administration, suitable pharmaceutically acceptable carriers include normal saline, bacteriostatic water, Cremophor EL™ (BASF, Parsippany, N.J.) or phosphate buffered saline (PBS). In all cases, the composition is required to be sterile and should be fluid to the extent of ease of injection. The composition is required to be stable under the preparation and storage conditions and is required to prevent the contamination of microorganisms such as bacteria and fungi. The carrier may be a solvent or dispersion medium containing, for example, water, ethanol, and polyol (e.g., glycerin, propylene glycol, and liquid polyethylene glycol), or an appropriate mixture thereof. For example, for dispersion, the required particle size is maintained through a coating such as lecithin, and appropriate liquidity can be kept through a surfactant. The action of microorganisms can be prevented through various antibacterial and antifungal agents, such as paraben, chlorobutanol, phenol, ascorbic acid, and thimerosal. In many cases, the composition preferably contains an isotonic agent, such as a saccharide, a polyol (such as mannitol and sorbitol), and sodium chloride. Absorption of the composition for injection can be prolonged by adding a reagent for delaying absorption, such as aluminum monostearate and gelatin, in the composition.

As required, a sterile injectable solution can be prepared by incorporating a required amount of antibody into a suitable solvent with one or a combination (as required) of the ingredients enumerated above, filtering, and sterilizing. In general, a dispersion is prepared by incorporating an antibody into a sterile carrier containing an alkaline dispersion medium and required other ingredients among those enumerated above. For sterile powder for preparation of a sterile injectable solution, the preparation methods are vacuum-drying and freeze-drying to obtain a powder that contains an active ingredient and any additional desired ingredient from a sterile-filtered solution of these ingredients described above.

For inhalation administration, a compound is delivered as an aerosol spray from a pressurized vessel or dispenser or nebulizer containing a suitable propellant, such as a gas such as carbon dioxide.

Systemic administration can also be performed via transmucosal or transdermal routes. For transmucosal or transdermal administration, a penetrant suitable for permeating the barrier is used in preparation. Such a penetrant is generally known in the art and includes, for example, a detergent, a bile salt, and a fusidic acid derivative for transmucosal administration. Transmucosal administration can be performed by using a nasal spray or suppository. For transdermal administration, one or more antibodies can be prepared into a paste, ointment, gel or cream as generally known in the art.

The compound can also be prepared in the form of a suppository (e.g., with a conventional suppository base such as cocoa butter and other glycerides) or a retention enema for delivery via the rectum.

In an embodiment, the antibody can be prepared with a carrier that will protect the antibody from rapid elimination from the body, such as a sustained release/controlled release preparation, including an implant and a microencapsulated delivery system. Biodegradable or biocompatible polymers such as ethylene vinyl acetate, a polyanhydride, polyglycolic acid, collagen, a polyorthoester, and polylactic acid, can be used. Preparation methods of such preparations are apparent to those skilled in the art.

It is especially advantageous to prepare parenteral compositions in the dosage unit form for ease of administration and uniformity of dosage. As used herein, the dose unit form refers to a physically separable unit suitable as a unit dose for a subject to be treated; and each unit contains predetermined amounts of one or more antibodies that are calculated to achieve a desired therapeutic effect when used in combination with a required drug carrier. The specification for the dose unit form of the embodiment is dictated by and directly dependent on the unique characteristics of the antibody and a particular therapeutic effect to be achieved, and the limitations inherent in the art of formulation of such antibodies for the treatment of individuals.

The pharmaceutical composition may be placed in a vessel, pack or dispenser together with instructions for administration.

The preparation described herein may also contain more than one antibody, preferably those with complementary activities that do not adversely affect each other, depending on the particular condition to be treated. Alternatively, or in addition, a composition may contain, for example, an agent that enhances its function, such as a cytotoxic agent, a cytokine, a chemotherapeutic agent, and a growth inhibitor. Such molecules are appropriately present together in amounts effective for the intended purpose. For example, the molecules may be present together in a kit or use together.

In an embodiment, one or more antibodies can be administered in combination treatment, that is, administered in combination with other agents such as a therapeutic agent (which can be used for treating a pathological condition or disorder, such as various forms of cancer, autoimmune disorders and inflammatory diseases). The term "combination" herein refers to substantially synchronous, simultaneous or sequential administration of reagents. In the case of sequential administration, when a second compound is started to be administered, a first compound of the two compounds is still preferably detected at a treatment site at an effective concentration. In one case, "combination" may also be that the kit contains both the antibody of the present disclosure and other therapeutic agents. For example, combination treatment may include co-preparation and/or co-administration of one or more antibodies described herein and one or more additional therapeutic agents (e.g., one or more cytokine and growth factor inhibitors, immunosuppressants, anti-inflammatory agents, metabolic inhibitors, enzyme inhibitors, and/or cytotoxins or cytostatic agents, as described in more detail below). Such combination treatment can advantageously utilize lower doses of therapeutic agents administered, thus avoiding possible toxicity or complications associated with various monotherapies.

For purposes of clarity and conciseness of description, features are described herein as part of the same or separate embodiments. However, it should be understood that the scope of the present disclosure may include some embodiments with combinations of all or some of the described features.

## Example

### Example 1: Preparation of recombinant CD70 protein

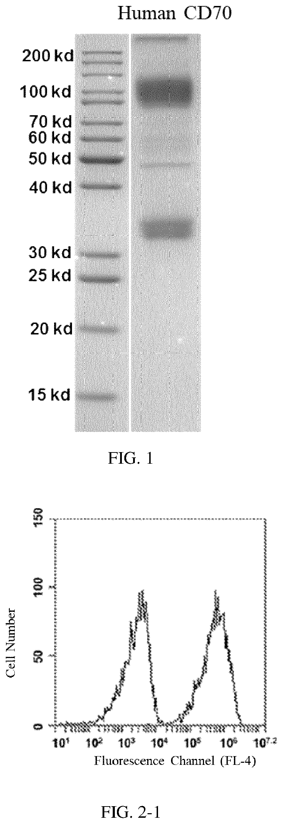

The cDNA plasmid for human CD70 (Sino Biological, Cat. No: HG10780-M) was taken as a template, and the human CD70 extracellular domain fragment (Glu13-Pro155) was amplified by PCR. The CD70 extracellular domain trimeric structure was constructed by linking peptide GSGSGNGS (SEQ ID NO: 161). The C-terminal was fused with mouse IgG Fc (knob) and Avi tag GSGLNDIFEAQKIEWHE (SEQ ID NO: 162) to obtain purified human CD70 mIgG Fc (knob) (the amino acid sequence is shown in SEQ ID NO: 1). The plasmid containing the gene encoding the human CD70 mIgG Fc (knob) was co-transfected into a HEK293E cell with the plasmid mouse IgG Fc (hole) (the amino acid sequence is shown in SEQ ID NO: 2). After 7 days of culture, the culture supernatant was collected and purified by Protein G to obtain the recombinant trimeric protein of human CD70 extracellular region. See FIG. 1 for SDS-PAGE.

Example 2. Construction of CD70-CHO stably-transfected cell line

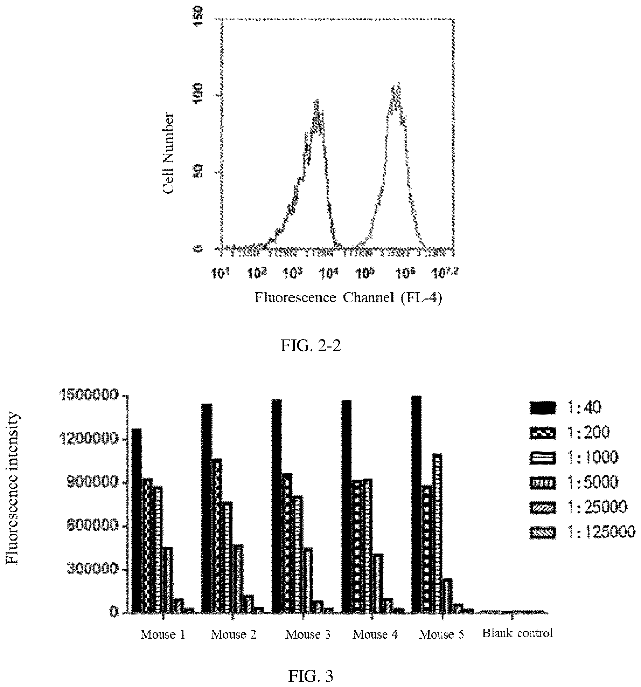

Human CD70 cDNA and monkey CD70 cDNA (Sino Biological, Cat. No: CG90089-G) were used to construct lentivirus vector plasmids comprising the full-length sequence of human and monkey CD70, respectively. According to the instructions for the lentiviral packaging kit (Lenti-Pac HIV Expression Packaging Kit, Gene Copoeia, Cat. No: HPK-LvTR-20), the constructed lentiviral plasmid and the packaging plasmid were co-transfected into HEK293T cells for lentiviral packaging. After 48 hours of transfection, the culture medium was collected, and centrifuged at 500 * g for 10 minutes to remove cell debris, and the culture supernatant containing lentiviral particles was obtained. After filtration through a 0.45 $\mu$m PES membrane, the supernatant was dispensed into 1.5 mL EP tubes, 10 $\mu$L of each was taken to infect $1 \times 10^6$ CHO cells, and 10 $\mu$g/mL puromycin was added to the culture medium for selection. After positive clones were obtained, stably-transfected cells CHO-human CD70-B07 and CHO-monkey CD70-D01 expressing human and monkey full-length CD70 were finally obtained by limiting dilution. See FIG. 2-1 and FIG. 2-2 for the results.

**Example 3: Preparation of anti-human CD70 monoclonal antibody**

The recombinant human CD70 protein as antigen was mixed with an equal amount of immunoadjuvant (Freund's adjuvant) and six 6-week female Balb/c mice per group were immunized subcutaneously. Boosting was performed two weeks after the primary immunization. After three immunizations, orbital blood was collected and serum titer was determined by flow cytometry. See FIG. 3 for the results. As can be seen in FIG. 3, high-titer CD70 antibodies were detected in all mouse serums.

Mice immunized with recombinant human CD70 protein were subjected to rush immunization by tail vein injection. Three days later, the mice were killed by cervical dislocation. The spleen and some peripheral lymph nodes were collected, ground in a DMEM medium, and centrifuged. The supernatant was decanted. Then, the cell mass was gently broken. 5 mL of red blood cell lysis solution was added for performing 50 seconds of lysis. 40 mL of DMEM medium was added for centrifugation to obtain a red blood cell-free B cell suspension. After mixing an appropriate amount of B cell suspension with SP2/0, cell fusion was performed by using an electric fusion instrument. The fused hybridoma cells were cultured in a DMEM complete medium containing HAT under 5% $CO_2$ at 37°C. Total RNA was extracted from the remaining B cells by the Trizol lysis method. Using a reverse transcription kit (SuperScript First-Strand Synthesis System, Cat. No: 18080051), reverse transcription was performed by using the heavy chain specific primers to obtain an antibody heavy chain cDNA library. Using the cDNA as a template, the heavy chain variable region fragment of the antibody was amplified by the heavy chain variable region primer PCR and then cloned into the phage plasmid after double digestion of NcoI and NheI. Similarly, reverse transcription was performed by using the light-chain specific primers to obtain a light chain cDNA library. Using the cDNA as a template, the light chain variable region fragment of the antibody was amplified by the light chain variable region primer PCR and then cloned into the phage plasmid after double digestion with NcoI and BsiwI. A mouse Fab phage display library based on filamentous phage M13 was then constructed with a capacity of $1 \times 10^{10}$.

**Example 4: Preparation of anti-human CD70 monoclonal antibody**

1. Screening of antibody phage display library

The phage display library was screened for positive clones by using classical panning. Recombinantly expressed human CD70 protein was coated on an Immunotube tube, and added with $1 \times 10^{12}$ phage for incubation and panning. The phage particles with high binding affinity were eluted with 100 mM triethylamine, neutralized with 1 M Tris-HCl (pH 7.4) and infected into E. coli TG1 bacteria. The phage enriched after repackaging was used for the next round of panning. The clones were obtained after 2 rounds of panning and enrichment, a monoclone was picked and inoculated into a 96-well U-plate for culture and IPTG induction of expression. The supernatant was taken for ELISA detection.

1) Screening for CD70-binding Fab antibodies by enzyme-linked immunosorbent assay (ELISA)

The recombinant CD70 protein was coated at 1 $\mu$g/mL in a 96-well microtiter plate and incubated overnight at 4°C. The next day, the plate was washed 3 times with PBS, blocked with 200 $\mu$L of 2% defatted dry milk/PBS for 2 hours at room temperature, and washed once with PBS, 30 $\mu$L of induced supernatant was added, and incubation was performed for 1 hour at room temperature. The plate was washed 3 times repeatedly with PBST (PBS + 0.05% Tween-20) and PBS, added with 100 μL/well HRP labeled Anti-Flag secondary antibody solution, and incubated for 60 minutes at room temperature. After washing three times each with PBST and PBS, 100 μL of the developing solution (TMB solution, Sigma, Cat. No. T2885) was added. After standing at 37°C for 5 minutes, the reaction was terminated by adding 50 μL of 2M concentrated sulfuric acid solution. The OD450nm was immediately measured with an enzyme-linked detector (BioTek, Synergy HT).

2) Elisa screening for Fab antibodies that block the interaction between CD70 and CD27

Positive monoclone binding to CD70 antigen was re-induced by IPTG, and purified to obtain Fab antibody protein, which was screened by ELISA assay. Recombinant human or cynomolgus monkey CD27 protein was coated at 1 μg/mL onto a 96-well microtiter plate, respectively, and incubated overnight at 4°C. The next day, the plate was washed 3 times with PBS, added with 200 μL of 2% defatted dry milk/PBS, blocked at room temperature for 2 hours, and washed once with PBS, 50 μL of different dilution gradients of Fab antibodies were added and incubated at room temperature for 30 minutes. 50 μL of Biotin-labeled recombinant CD70 protein was then added to each well and incubated at room temperature for 60 minutes. The plate was washed 3 times with PBST and PBS and dried by spinning. A secondary antibody solution Streptavidin-coupled horseradish peroxidase SA-HRP (BD bioscience, Cat. No: 6222697) was diluted with 0.5% BSA in a ratio of 1:8000. 100 μl of the diluent was added into each well and incubate at room temperature for 30 minutes. The plate was washed 3 times with PBST and PBS and dried by spinning. 100 μl of the color development solution (TMB solution, Sigma, Cat. No: T2885) was added into each well and protected from light at 37°C for reaction. After the substrate developed moderately, 50 μl of 2M concentrated sulfuric acid solution was added into each well to terminate the reaction. The OD450 nm was determined by using an enzyme-linked detector within 30 minutes, and the data was collected. See Table 1 for the results.

3) Screening of Fab antibodies specifically binding to CD70 on the cell surface by Flow cytometry

The positive clones binding to CD70 antibodies detected by ELISA were further verified for binding to CD70-positive cells. The blank control cell CHO and stably-transfected cell line CHO-CD70 were prepared and added at $5 \times 10^4$ cells/well into a 96-well plate respectively. 50 μL of Fab antibodies with different dilution gradients were added into each well and incubated at 4°C for 60 minutes. The centrifuge was performed to discard the supernatant. The plate was washed with 0.5% BSA/PBS and added with 50 μL of a secondary antibody solution (Anti-human IgG, F (ab') 2-AF647, Jackson Immuno Research) and incubated at 4°C for 45 minutes. The excess secondary antibody was washed off with 0.5% BSA. The PBS solution was added to resuspend cells for flow cytometry. The data was collected. See Table 1 for the results.

4) Octet screening for Fab antibodies specifically binding to CD70

10 μg/mL biotin-labeled CM352 antigen was immobilized on a SA probe by biomembrane interference technique (BLI), with the time set to 300s, and bound different concentrations of CM352 Fab antibodies, with the binding time set to 200s and dissociation time to 300s, to obtain the dissociation rate kdis (1/s). See Table 1 for the results.

Table 1. Screening of anti-human CD70 antibodies phage display library

| Clone | Elisa blocking IC50(nM) | | FACS binding EC50(nM) | | Octet k-off (1/s) |
|---|---|---|---|---|---|
| | Human | Cynomolgus monkey | Human | Cynomolgus monkey | Human |
| 2B12 | 0.29 | 0.65 | 8.6 | 25.3 | $1.25 \times 10^{-4}$ |
| 3B2 | 0.32 | 0.48 | 14.15 | 15.69 | $3.25 \times 10^{-4}$ |
| 12A1 | 1.88 | 15.92 | 17.67 | 34.64 | $3.17 \times 10^{-4}$ |
| 14C12 | 0.79 | 5.15 | 28.01 | 27.74 | $3.21 \times 10^{-4}$ |
| 23A11 | 0.5 | 0.95 | 15.63 | 18.02 | $4.03 \times 10^{-4}$ |
| 27A3 | 0.51 | 1.45 | 12.38 | 18.62 | $1.72 \times 10^{-4}$ |
| 25H7 | 0.76 | 14.44 | 15.48 | 23.39 | $3.26 \times 10^{-4}$ |
| 26G11 | 4.82 | 4.36 | 40.66 | 49.68 | $2.87 \times 10^{-4}$ |
| 32D1 | 2.26 | 3.37 | 8.10 | 9.65 | $18.3 \times 10^{-4}$ |

(continued)

| Clone | Elisa blocking IC50(nM) | | FACS binding EC50(nM) | | Octet k-off (1/s) |
|---|---|---|---|---|---|
| | Human | Cynomolgus monkey | Human | Cynomolgus monkey | Human |
| 39H5 | 0.38 | 0.37 | 8.7 | 10.25 | $0.9 \times 10^{-4}$ |

2. Selection of Hybridomas

After 7 days of culture, the hybridoma cell culture supernatant was taken for ELISA to obtain multiple positive clones that can specifically recognize CD70 and efficiently block the CD70-CD27 binding. Clones that cannot bind to cell surface CD70 were further excluded by the flow cytometry. See Table 2 for the results.

Table 2. Screening of anti-human CD70 antibody hybridomas

| Clone | ELISA binding | | Elisa blocking | | FACS binding | |
|---|---|---|---|---|---|---|
| | Human | Monkey | Human | Monkey | Human | Monkey |
| 1H21-D5 | + | + | + | + | + | + |
| 10C19-D11 | + | + | + | + | + | + |
| 16J15-H3 | + | + | + | + | + | + |
| 17A9-G5 | + | + | + | + | + | + |
| 36E7-D6 | + | + | + | + | + | + |
| 4H6-A6 | + | + | + | + | + | + |
| 42H19-G4 | + | + | + | + | + | + |

3. Gene cloning and expression of chimeric anti-human CD70 antibodies

According to the results of the hybridoma screening, the RNA of the hybridoma cell candidate was extracted by the TRNzol lysis method. Then, a single-chain cDNA was synthesized by reverse transcription, which was used as the template to amplify the variable region sequence of the antibody in the hybridoma cell candidate. According to the results of phage screening, positive monoclonal bacterial plasmids were extracted. After sequencing, the candidate positive heavy and light chain variable region sequences of the clone were obtained as follows:

Table 3. BCMA murine antibody variable region sequences

| BCMA murine antibodi es | HCVR | | HCD R1 | HCD R2 | HCD R3 | LCVR | | LCD R1 | LCD R2 | LCD R3 |
|---|---|---|---|---|---|---|---|---|---|---|
| | Ami no Acid Seq. | Nucleo tide Seq. | Amin o Acid Seq. | Amin o Acid Seq. | Amin o Acid Seq. | Ami no Acid Seq. | Nucleo tide Seq. | Ami no Acid Seq. | Ami no Acid Seq. | Ami no Acid Seq. |
| 2B12 | 3 | 4 | 5 | 6 | 7 | 8 | 9 | 10 | 11 | 12 |
| 3B2 | 13 | 14 | 15 | 6 | 16 | 17 | 18 | 19 | 11 | 12 |
| 12A1 | 20 | 21 | 5 | 6 | 22 | 23 | 24 | 25 | 11 | 26 |
| 14C12 | 27 | 28 | 29 | 30 | 31 | 32 | 33 | 34 | 11 | 26 |
| 23A11 | 35 | 36 | 5 | 37 | 38 | 39 | 40 | 41 | 11 | 12 |
| 25H7 | 42 | 43 | 44 | 45 | 46 | 47 | 48 | 49 | 50 | 51 |
| 26G11 | 52 | 53 | 54 | 55 | 56 | 57 | 58 | 59 | 60 | 61 |
| 27A3 | 62 | 63 | 5 | 64 | 65 | 66 | 67 | 68 | 11 | 69 |
| 32D1 | 70 | 71 | 29 | 30 | 31 | 72 | 73 | 74 | 11 | 26 |
| 39H5 | 75 | 76 | 44 | 77 | 46 | 78 | 79 | 49 | 80 | 51 |
| 1H21-D 5 | 81 | 82 | 83 | 84 | 85 | 86 | 87 | 88 | 11 | 12 |
| 10C19-D11 | 89 | 90 | 5 | 91 | 85 | 92 | 93 | 19 | 11 | 12 |

(continued)

| BCMA murine antibodi es | HCVR | | HCD R1 | HCD R2 | HCD R3 | LCVR | | LCD R1 | LCD R2 | LCD R3 |
|---|---|---|---|---|---|---|---|---|---|---|
| | Ami no Acid Seq. | Nucleo tide Seq. | Amin oAcid Seq. | Amin oAcid Seq. | Amin oAcid Seq. | Ami no Acid Seq. | Nucleo tide Seq. | Ami no Acid Seq. | Ami no Acid Seq. | Ami no Acid Seq. |
| 16J15-H3 | 94 | 95 | 96 | 97 | 98 | 99 | 100 | 101 | 102 | 103 |
| 17A9-G 5 | 104 | 105 | 15 | 106 | 107 | 108 | 109 | 110 | 111 | 103 |
| 36E7-D 6 | 112 | 113 | 114 | 115 | 116 | 117 | 118 | 119 | 120 | 121 |
| 4H6-A6 | 122 | 123 | 15 | 124 | 16 | 125 | 126 | 127 | 128 | 103 |
| 42H 19-G4 | 129 | 130 | 131 | 132 | 116 | 117 | 118 | 119 | 120 | 121 |
| Clone: 2B12 | | | | | | | | | | |

The amino acid sequence of 2B12 heavy chain VH is shown in SEQ ID NO: 3; its encoding nucleic acid is shown in SEQ ID NO: 4; and its CDR1, CDR2, CDR3 are shown in SEQ ID NO: 5, 6, 7, respectively.

```
<-----------FR1----------->    CDR1    <----FR2----->        CDR2
<---
QVQLQQSGPGLVQPSQSLSITCTVSGFSLTTYAVHWVRQSPGKGLE
WLGVIWSTGITDYNAAFISRLSI
------------FR3------------>    CDR3       <---FR4--->
SKDNSKSQVFFKMNSLQSDDIAIYYCARNPYYGNLNAMDYWGQGT
SVTVSS
```

Nucleic acid sequence

CAGGTGCAGCTGCAGCAGTCAGGACCTGGCCTAGTGCAGCCCTCACAGAGCCT
GTCCATCACCTGCACAGTCTCTGGTTTCTCATTAACTACCTATGCTGTGCACTGG
GTTCGCCAGTCTCCAGGAAAGGGTCTGGAGTGGCTGGGAGTGATATGGAGTAC
TGGAATCACAGACTATAATGCAGCTTTCATATCCAGACTGAGCATCAGCAAGGA
CAACTCCAAGAGCCAAGTTTTCTTTAAAATGAACAGTCTGCAATCTGATGACAT
AGCCATATACTACTGTGCCAGAAATCCCTACTATGGTAACCTCAATGCTATGGAC
TACTGGGGTCAAGGAACCTCAGTCACCGTCTCCTCA

The amino acid sequence of 2B12 light chain VK is shown in SEQ ID NO: 8; its encoding nucleic acid is shown in SEQ ID NO: 9; and its CDR1, CDR2, CDR3 are shown in SEQ ID NO: 10, 11, 12, respectively.

```
<----------FR1-------->        CDR1        <-----FR2----->   CDR2
<--------
DIVLTQSPASLAVSLGQRATISCRASKSVDTSGYSFMHWYQQKPGQ
PPKLLIYLASNLESGVPARFSGS
-----------FR3-------->    CDR3    <---FR4--->
GSGTDFTLNIHPVEEEDAATYYCQHSRELPLTFGAGTKLELK
```

Nucleic acid sequence

GACATTGTGCTGACACAGTCTCCTGCTTCCTTAGCTGTATCTCTGGGGCAGAGG
GCCACCATCTCATGCAGGGCCAGCAAAAGTGTCGATACATCTGGCTATAGTTTT
ATGCACTGGTACCAACAGAAACCAGGACAGCCACCCAAACTCCTCATCTATCTT
GCATCCAACCTAGAATCTGGGGTCCCTGCCAGGTTCAGTGGCAGTGGGTCTGG
GACAGACTTCACCCTCAACATCCATCCTGTGGAGGAGGAGGATGCTGCAACCT
ATTACTGTCAGCACAGTAGGGAGCTTCCTCTCACGTTCGGTGCTGGGACCAAG
CTGGAGCTGAAA

Clone: 3B2

The amino acid sequence of 3B2 heavy chain VH is shown in SEQ ID NO: 13; its encoding nucleic acid is shown in SEQ ID NO: 14; and its CDR1, CDR2, CDR3 are shown in SEQ ID NO: 15, 6, 16, respectively.

```
        <-----------FR1----------->    CDR1     <----FR2----->          CDR2
        <---
        EVQLKQSGPGLVQPSQSLSITCTVSGFSLTTYGVHWVRQSPGKGLE
        WLGVIWSTGITDYNAAFISRLSI
        ------------FR3------------>     CDR3        <---FR4--->
        SKDNSKSQVFFKMNSLQADDTAIYYCARNSYYGNLYVMDYWGQG
        TSVTVSS
```

Nucleic acid sequence

GAGGTGCAGCTGAAGCAGTCAGGACCTGGCCTAGTGCAGCCCTCACAGAGCC
TGTCCATCACCTGCACAGTCTCTGGTTTTTCATTAACTACCTATGGTGTACACTG

GGTTCGCCAGTCTCCAGGAAAGGGTCTGGAGTGGCTGGGAGTGATATGGAGTA
CTGGAATCACAGACTATAATGCAGCTTTCATATCCAGACTGAGCATCAGTAAGG
ACAATTCCAAGAGCCAAGTCTTCTTTAAAATGAACAGTCTGCAAGCTgATGACA
CAGCCATATATTACTGTGCCAGAAATTCCTACTATGGTAACCTTTATGTTATGGAC
TACTGGGGTCAAGGAACCTCAGTCACCGTCTCCTCA

The amino acid sequence of 3B2 light chain VK is shown in SEQ ID NO: 17; its encoding nucleic acid is shown in SEQ ID NO: 18; and its CDR1, CDR2, CDR3 are shown in SEQ ID NO: 19, 11, 12, respectively.

```
<----------FR1-------->        CDR1        <-----FR2----->  CDR2
<--------
DIVLTQSPASLAVSLGQRATISCRASKSVSASGYNFMHWYQQKPGQ
PPKLLIYLASNLESGVPARFSGS
-----------FR3-------->    CDR3    <---FR4--->
GSGTDFTLNIHPVEEEDAATYYCQHSRELPLTFGAGTKLELK
```

Nucleic acid sequence

GACATTGTGCTAACACAGTCTCCTGCTTCCTTAGCTGTGTCTCTGGGGCAGAGG

GCCACCATCTCATGCAGGGCCAGCAAAAGTGTCAGTGCATCTGGCTATAATTTT

ATGCACTGGTATCAACAGAAACCAGGACAGCCACCCAAACTCCTCATCTATCTT

GCATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGAGCTTCCGCTCACGTTCGGTGCTGGGACCAAG

CTGGAGCTGAAA

Clone: 12A1

The amino acid sequence of 12A1 heavy chain VH is shown in SEQ ID NO: 20; its encoding nucleic acid is shown in SEQ ID NO: 21; and its CDR1, CDR2, CDR3 are shown in SEQ ID NO: 5, 6, 22, respectively.

```
<-----------FR1---------->    CDR1    <----FR2----->          CDR2
<---
QVQLQQSGPGLVQPSQSLSITCTVSGFSLTTYAVHWVRQSPGKGLE
WLGVIWSTGITDYNAAFISRLSI
------------FR3------------>     CDR3       <---FR4--->
SKDNSKSQVFFKMDSLQSDDTAIYFCARNPHYGNLNAMDYWGQG
TSVTVSS
```

Nucleic acid sequence

CAGGTGCAGCTGCAGCAGTCAGGACCTGGCCTAGTGCAGCCCTCACAGAGCCT

GTCCATCACCTGCACAGTCTCTGGTTTCTCATTAACTACCTATGCTGTGCACTGG

GTTCGCCAGTCTCCAGGAAAGGGTCTGGAGTGGCTGGGAGTAATATGGAGTAC

TGGAATCACAGACTATAATGCAGCTTTCATATCCAGACTGAGCATCAGCAAGGA

CAACTCCAAGAGCCAAGTTTTCTTTAAAATGGACAGTCTGCAATCTGATGACAC
AGCCATATACTTCTGTGCCAGAAATCCCCACTATGGTAATCTCAATGCTATGGAC
TACTGGGGTCAAGGAACCTCAGTCACCGTCTCCTCA

The amino acid sequence of 12A1 light chain VK is shown in SEQ ID NO: 23; its encoding nucleic acid is shown in SEQ ID NO: 24; and its CDR1, CDR2, CDR3 are shown in SEQ ID NO: 25, 11, 26, respectively.

```
        <----------FR1-------->        CDR1         <-----FR2-----> CDR2
        <--------
        DIVLTQSPASLAVSLGQRATISCRASKSVSASGYTWMHWYQQKPG
        QAPKLLIYLASNLESGVPARFSGS
        -----------FR3-------->     CDR3     <---FR4--->
        GSGTDFTLNIHPVEEEDAATYYCQHSRELPPTFGGGTKLEIK
```

Nucleic acid sequence

GACATTGTGCTGACACAGTCTCCTGCTTCCTTAGCTGTATCTCTGGGGCAGAGG
GCCACCATCTCATGCAGGGCCAGCAAAAGCGTCAGTGCATCTGGCTATACTTGG
ATGCACTGGTACCAACAGAAACCAGGACAGGCACCCAAACTCCTCATCTATCT
TGCATCCAACCTAGAATCTGGGGTCCCTGCCAGGTTCAGTGGCAGTGGGTCTG
GGACAGACTTCACCCTCAACATCCATCCTGTGGAGGAGGAGGATGCTGCAACC
TATTACTGTCAGCACAGTAGGGAGCTTCCTCCGACGTTCGGTGGAGGCACCAA
GCTGGAAATCAAA

Clone: 14C12

The amino acid sequence of 14C12 heavy chain VH is shown in SEQ ID NO: 27; its encoding nucleic acid is shown in SEQ ID NO: 28; and its CDR1, CDR2, CDR3 are shown in SEQ ID NO: 29, 30, 31, respectively.

```
        <-----------FR1----------> CDR1     <----FR2-----> CDR2
        <--
        EVQLQQSGPELVKPGASVKMSCKASGYTFTDYVIHWVKQKPGQGL
        EWIGYLHPYNDDTKYNEKFKGKAT
        ------------FR3------------->    CDR3     <---FR4--->
        LTSDKSSSTAYMELSSLTSEDSAVYYCARRGYYDYAWFADWGQGT
        LVTVSA
```

Nucleic acid sequence

GAGGTCCAGCTGCAGCAGTCTGGACCTGAGCTGGTAAAGCCTGGGGCTTCAGT

GAAGATGTCCTGCAAGGCTTCTGGATACACATTCACTGACTATGTTATACACTG

GGTGAAGCAGAAGCCTGGGCAGGGCCTTGAGTGGATTGGATATCTTCATCCTTA

CAATGATGATACTAAGTACAATGAGAAGTTCAAAGGCAAGGCCACACTGACTT

CAGACAAATCCTCCAGCACAGCCTACATGGAGCTCAGCAGCCTGACCTCTGAG

GACTCTGCGGTCTATTACTGTGCAAGACGGGGGTACTATGATTACGCCTGGTTT

GCTGACTGGGGCCAAGGGACTCTGGTCACTGTCTCTGCA

The amino acid sequence of 14C12 light chain VK is shown in SEQ ID NO: 32; its encoding nucleic acid is shown in SEQ ID NO: 33; and its CDR1, CDR2, CDR3 are shown in SEQ ID NO: 34, 11, 26, respectively.

```
       <----------FR1-------->        CDR1        <-----FR2----->  CDR2
       <--------
       DIVLTQSPASLAVSLGQRATISCRASKSVSASGYTWIHWYQQKPRQ
       APKLLIYLASNLESGVPARFSGS
       -----------FR3-------->     CDR3   <---FR4--->
       GSGTDFTLNIHPVEEEDAATYYCQHSRELPPTFGGGTKLEIK
```

Nucleic acid sequence

GACATTGTGCTAACACAGTCTCCTGCTTCCTTAGCTGTATCTCTGGGGCAGAGG

GCCACCATCTCATGCAGGGCCAGCAAAAGTGTCAGTGCATCTGGCTATACTTGG

ATACACTGGTACCAACAGAAACCAAGACAGGCACCCAAACTTCTCATCTATCTT

GCATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGAACTTCCTCCGACGTTCGGTGGAGGCACCAAG

CTGGAAATCAAA

Clone: 23A11

The amino acid sequence of 23A11 heavy chain VH is shown in SEQ ID NO: 35; its encoding nucleic acid is shown in SEQ ID NO: 36; and its CDR1, CDR2, CDR3 are shown in SEQ ID NO: 5, 37, 38, respectively.

```
       <-----------FR1---------->  CDR1    <----FR2----->        CDR2
       <---
       QVQLQQSGPGLVQPSQSLSITCTVSGFSLTTYAVHWVRQSPGKGLE
       WLGVIWSVGSTDYNAAFISRLSI
       ------------FR3------------>     CDR3        <---FR4--->
       SKDNSKSQVFFKMNSLQADDTAIYYCARNIYYGNVYAMDYWGQG
       TSVTVSS
```

Nucleic acid sequence

CAGGTGCAGCTGCAGCAGTCAGGACCTGGCCTAGTGCAGCCCTCACAGAGCCT
GTCCATCACCTGCACAGTCTCTGGTTTCTCATTAACTACCTATGCTGTACACTGG
GTTCGCCAGTCTCCAGGAAAGGGTCTGGAGTGGCTGGGAGTTATATGGAGTGT
TGGAAGCACAGACTATAATGCAGCTTTCATATCCAGACTGAGCATCAGCAAGGA
CAATTCCAAGAGCCAAGTTTTCTTTAAAATGAACAGTCTGCAAGCTgATGACAC
AGCCATATATTACTGTGCCAGAAATATCTACTATGGTAATGTTTATGCTATGGACT
ACTGGGGTCAAGGAACCTCAGTCACCGTCTCCTCA

The amino acid sequence of 23A11 light chain VK is shown in SEQ ID NO: 39; its encoding nucleic acid is shown in SEQ ID NO: 40; and its CDR1, CDR2, CDR3 are shown in SEQ ID NO: 41, 11, 12, respectively.

```
<----------FR1--------->        CDR1        <-----FR2----->  CDR2
<--------
DIVLTQSPASLAVSLGQRATISC**RASKSVDASGYSFMH**WYQQKAGQ
PPKLLIY<u>LASNLES</u>GVPARFSGR
-----------FR3-------->   CDR3    <---FR4--->
GSGTDFTLNIHPVEEGDAATYYC**QHSRELPLT**FGAGTKLEIK
```

Nucleic acid sequence

GACATTGTGCTAACACAGTCTCCTGCTTCCTTAGCTGTATCTCTGGGGCAGAGG
GCCACCATCTCATGCAGGGCCAGCAAAAGTGTCGATGCATCTGGCTATAGTTTT
ATGCACTGGTACCAACAGAAAGCAGGACAGCCACCCAAACTCCTCATCTATCT
TGCATCCAACCTAGAATCTGGGGTCCCTGCCAGGTTCAGTGGCAGGGGGTCTG
GGACAGACTTCACCCTCAACATCCATCCTGTGGAGGAGGGGGATGCTGCAACC
TATTACTGTCAGCACAGTAGGGAGCTTCCTCTCACGTTCGGGGCTGGGACCAA
GCTGGAAATCAAA

Clone: 25H7

The amino acid sequence of 25H7 heavy chain VH is shown in SEQ ID NO: 42; its encoding nucleic acid is shown in SEQ ID NO: 43; and its CDR1, CDR2, CDR3 are shown in SEQ ID NO: 44, 45, 46, respectively.

```
<-----------FR1----------->    CDR1      <----FR2----->         CDR2
<--
EIQLVQSGPELKKPGETVKISCKASG**YTFTNYGMN**WVKQTPGKGLK
WMG**WINTDTGEPTYADDFKG**RFA
------------FR3------------->      CDR3      <---FR4--->
FSLETSASTAYLQINNLKNEDMATYFCAR**EEDYRYVFNY**WGQGTT
LTVSS
```

Nucleic acid sequence

GAGATCCAGCTGGTGCAGTCTGGACCTGAGCTGAAGAAGCCTGGAGAGACTG
TCAAGATCTCCTGCAAGGCTTCTGGATATACCTTCACAAACTATGGAATGAACT
GGGTGAAGCAGACTCCAGGAAAGGGTTTAAAGTGGATGGGCTGGATAAACAC
CGACACTGGAGAGCCAACATATGCTGATGACTTCAAGGGACGGTTTGCCTTCTC
TTTGGAAACCTCTGCCAGCACTGCCTATTTGCAGATCAACAACCTCAAAAATGA
GGACATGGCTACATATTTCTGTGCAAGAGAGGAGGACTATAGGTACGTTTTTAA
TTACTGGGGCCAAGGCACCACTCTCACAGTCTCCTCA

The amino acid sequence of 25H7 light chain VK is shown in SEQ ID NO: 47; its encoding nucleic acid is shown in SEQ ID NO: 48; and its CDR1, CDR2, CDR3 are shown in SEQ ID NO: 49, 50, 51, respectively.

```
<----------FR1-------->      CDR1        <-----FR2----->  CDR2
<-----------
DIQMTQTTSSLSASLGDRVTISC**RASQDISNYLN**WYQQKPDGTVKLL
IY**YTSRLHS**GVPSRFSGSGSGT
-----FR3---------->      CDR3      <---FR4--->
 DYSLTISNLEQEDIATYFC**QQGVTLPLT**FGAGTKLELK
```

Nucleic acid sequence

GATATCCAGATGACACAGACTACATCCTCCCTGTCTGCCTCTCTGGGAGACAGA
GTCACCATCAGTTGCAGGGCAAGTCAGGACATTAGCAATTATTTAAACTGGTAT
CAGCAGAAACCAGATGGAACTGTTAAACTCCTGATCTACTACACATCAAGATTA
CACTCAGGAGTCCCATCAAGGTTCAGTGGCAGTGGGTCTGGAACAGATTATTCT
CTCACCATTAGCAACCTGGAGCAAGAAGATATTGCCACTTACTTTTGCCAACAG
GGTGTTACGCTTCCTCTCACGTTCGGTGCTGGGACCAAGCTGGAGCTGAAA

Clone: 26G11

The amino acid sequence of 26G11 heavy chain VH is shown in SEQ ID NO: 52; its encoding nucleic acid is shown in SEQ ID NO: 53; and its CDR1, CDR2, CDR3 are shown in SEQ ID NO: 54, 55, 56, respectively.

```
<-----------FR1----------->    CDR1    <----FR2----->          CDR2
<--
QVQLQQSGPELVKPGASVKISCKTSGYTFADYTMHWVRQSQGKSL
EWIGDIYPNDGGPNNNQKFKDKAT
------------FR3------------->      CDR3      <---FR4--->
LTVDKSSSTAYMELRSLTSEDSAVYYCARPFYYGNYAGFAFWGQG
TLVTVSA
```

Nucleic acid sequence

CAGGTTCAGCTGCAACAGTCTGGACCTGAGCTGGTGAAGCCTGGGGCTTCAGT

GAAGATATCCTGCAAGACTTCTGGATACACATTCGCTGATTACACCATGCACTG

GGTGAGGCAGAGCCAAGGAAAGAGCCTTGAGTGGATTGGAGATATTTATCCTA

ACGATGGTGGTCCTAACAACAACCAGAAGTTCAAGGACAAGGCCACATTGACT

GTTGACAAGTCCTCCAGCACAGCCTACATGGAGCTCCGCAGCCTGACATCTGA

GGATTCTGCAGTCTATTACTGTGCAAGACCGTTCTATTATGGTAACTACGCGGGA

TTTGCTTTCTGGGGCCAAGGGACTCTGGTCACTGTCTCTGCA

The amino acid sequence of 26G11 light chain VK is shown in SEQ ID NO: 57; its encoding nucleic acid is shown in SEQ ID NO: 58; and its CDR1, CDR2, CDR3 are shown in SEQ ID NO: 59, 60, 61, respectively.

```
<----------FR1-------->     CDR1     <-----FR2----->   CDR2    <------------
EMVLTQSPAIMSASPGEKVTMTCSASSSVSYMYWYQQKPGSSPRLLI
YDTSNLASGVPVRFSGSGSGTS
-----FR3---------> CDR3         <---FR4--->
```

YSLTISRMEAEDAATYYCQQWSSYPLTFGAGTRLEIK

Nucleic acid sequence

GAAATGGTTCTCACCCAGTCTCCAGCAATCATGTCTGCATCTCCAGGGGAGAA
GGTCACCATGACCTGCAGTGCCAGCTCAAGTGTAAGTTACATGTACTGGTACCA
GCAGAAGCCAGGATCCTCCCCCAGACTCCTGATTTATGACACATCCAACCTGGC
TTCTGGAGTCCCTGTTCGCTTCAGTGGCAGTGGGTCTGGGACCTCTTACTCTCT
CACAATCAGCCGAATGGAGGCTGAAGATGCTGCCACTTATTACTGCCAGCAGT
GGAGTAGTTACCCGCTCACGTTCGGTGCTGGGACCAGACTGGAAATAAAA

Clone: 27A3

The amino acid sequence of 27A3 heavy chain VH is shown in SEQ ID NO: 62; its encoding nucleic acid is shown in SEQ ID NO: 63; and its CDR1, CDR2, CDR3 are shown in SEQ ID NO: 5, 64, 65, respectively.

```
        <-----------FR1----------->    CDR1    <----FR2----->        CDR2
        <---
        EVQLQESGPGLVQPSQSLSITCTVSGFSLTTYAVHWVRQSPGKGLE
        WLGVIWSTGITDYNADFISRLSI
        ------------FR3------------>       CDR3       <---FR4--->
        SKDNSKSQVFFKMDSLQSDDTAIYFCARNHYYGNLNAMDYWGQG
        TSVTVSS
```

Nucleic acid sequence

GAGGTCCAGCTGCAGGAGTCAGGACCTGGCCTAGTGCAGCCCTCACAGAGCCT
ATCCATCACCTGCACAGTCTCTGGTTTCTCATTAACTACCTATGCTGTACACTGG
GTTCGCCAGTCTCCAGGAAAGGGTCTGGAGTGGCTGGGAGTAATATGGAGTAC
TGGAATCACAGACTATAATGCAGATTTCATATCCAGACTGAGCATCAGCAAGGA
CAACTCCAAGAGCCAAGTTTTCTTTAAAATGGACAGTCTGCAATCTGATGACAC
AGCCATATACTTCTGTGCCAGAAATCACTACTATGGCAATCTCAATGCTATGGAC
TACTGGGGTCAAGGAACCTCAGTCACCGTCTCCTCA

The amino acid sequence of 27A3 light chain VK is shown in SEQ ID NO: 66; its encoding nucleic acid is shown in SEQ ID NO: 67; and its CDR1, CDR2, CDR3 are shown in SEQ ID NO: 68, 11, 69, respectively.

```
        <----------FR1-------->        CDR1        <-----FR2----->  CDR2
        <--------
        DIVLTQSPASLAVSLGQRATISCRASKSVSTSGYSFMHWYQQKPGQ
        PPKLLIYLASNLESGVPARFSGS
        -----------FR3-------->    CDR3    <---FR4--->
        GSGTDFTLNIHPVEEEDAATYYCQHSRELPYTFGGGTKLEIK
```

Nucleic acid sequence

GACATTGTGCTAACACAGTCTCCTGCTTCCTTAGCTGTATCTCTGGGGCAGAGG

GCCACCATCTCATGCAGGGCCAGCAAAAGTGTCAGTACATCTGGCTATAGTTTT
ATGCACTGGTACCAACAGAAACCAGGACAGCCACCCAAACTCCTCATCTATCTT
GCATCCAACCTAGAATCTGGGGTCCCTGCCAGGTTCAGTGGCAGTGGGTCTGG
GACAGACTTCACCCTCAACATCCATCCTGTGGAGGAGGAGGATGCTGCAACCT
ATTACTGTCAGCACAGTAGGGAGCTTCCGTACACGTTCGGAGGGGGGACCAAG
CTGGAAATCAAA

Clone: 32D1

The amino acid sequence of 32D1 heavy chain VH is shown in SEQ ID NO: 70; its encoding nucleic acid is shown in SEQ ID NO: 71; and its CDR1, CDR2, CDR3 are shown in SEQ ID NO: 29, 30, 31, respectively.

```
        <-----------FR1----------->    CDR1    <----FR2----->         CDR2
        <--
        EVQLQQSGPELVKPGASLKMSCKASGYTFTDYVIHWVKQKPGQGL
        EWIGYLHPYNDDTKYNEKFKGKAT
        ------------FR3------------->    CDR3    <---FR4--->
        LTSDKSSSTAYMELNSLTSEDSAVYYCARRGYYDYAWFADWGQG
        TLVTVSA
```

Nucleic acid sequence

GAGGTCCAGTTGCAGCAGTCTGGACCTGAGCTGGTAAAGCCTGGGGCTTCACT
GAAGATGTCCTGCAAGGCTTCTGGATACACATTCACTGACTATGTTATACACTG
GGTGAAGCAGAAGCCTGGGCAGGGCCTTGAGTGGATTGGATATCTTCATCCTTA
CAATGATGATACTAAGTACAATGAGAAGTTCAAAGGCAAGGCCACACTGACTT
CAGACAAATCCTCCAGCACAGCCTACATGGAACTCAACAGCCTGACCTCTGAG
GACTCTGCGGTCTATTACTGTGCAAGGCGGGGGTACTATGATTACGCCTGGTTT
GCTGACTGGGGCCAAGGGACTCTGGTCACTGTCTCTGCA

The amino acid sequence of 32D1 light chain VK is shown in SEQ ID NO: 72; its encoding nucleic acid is shown in SEQ ID NO: 73; and its CDR1, CDR2, CDR3 are shown in SEQ ID NO: 74, 11, 26, respectively.

```
<----------FR1-------->        CDR1         <-----FR2----->  CDR2
<--------
DIVLTQSPASLAVSLGQRATISCKASKSVSASGYTWMHWYQQKPG
QAPKLLIYLASNLESGVPARFSGS
-----------FR3-------->    CDR3     <---FR4--->
GSGTDFTLNIHPVEEEDAATYYCQHSRELPPTFGGGTKLEIK
```

Nucleic acid sequence

GACATTGTGCTAACACAGTCTCCTGCTTCCTTAGCTGTATCTCTGGGGCAGAGG

GCCACCATCTCATGCAAGGCCAGCAAAAGTGTCAGTGCATCTGGCTATACTTGG

ATGCACTGGTACCAACAGAAACCAGGACAGGCACCCAAACTCCTCATCTATCT

TGCATCCAACCTAGAATCTGGGGTCCCTGCCAGGTTCAGTGGCAGTGGGTCTG

GGACAGACTTCACCCTCAACATCCATCCTGTGGAGGAGGAGGATGCTGCAACC

TATTACTGTCAGCACAGTAGGGAGCTTCCTCCGACGTTCGGTGGAGGCACCAA

GCTGGAAATCAAA

Clone: 39H5

The amino acid sequence of 39H5 heavy chain VH is shown in SEQ ID NO: 75; its encoding nucleic acid is shown in SEQ ID NO: 76; and its CDR1, CDR2, CDR3 are shown in SEQ ID NO: 44, 77, 46, respectively.

```
<-----------FR1----------->    CDR1     <----FR2----->         CDR2
<--
EIQLVQSGPELKKPGETVKISCKASGYTFTNYGMNWVKQAPGQGL
KWMGWINTYTGEPTYADDFTGRFA
------------FR3------------->    CDR3     <---FR4--->
FSLETSASTAYLQINNLKNEDMAAYFCAREEDYRYVFNYWGQGTT
LTVSS
```

Nucleic acid sequence

GAGATCCAGTTGGTGCAGTCTGGACCTGAGCTGAAGAAGCCTGGAGAGACAG

TCAAGATCTCCTGCAAGGCTTCTGGGTATACCTTCACAAACTATGGAATGAACT

GGGTGAAGCAGGCTCCAGGACAGGGTTTAAAGTGGATGGGCTGGATAAACAC

CTATACTGGAGAGCCAACATATGCTGATGACTTCACGGGACGGTTTGCCTTCTC

CTTGGAAACCTCTGCCAGCACTGCCTATTTGCAGATCAACAACCTCAAAAATGA

GGACATGGCTGCATATTTCTGTGCAAGAGAGGAGGACTATAGGTACGTTTTTAA

CTACTGGGGCCAAGGCACCACTCTCACAGTCTCCTCA

The amino acid sequence of 39H5 light chain VK is shown in SEQ ID NO: 78; its encoding nucleic acid is shown in SEQ ID NO: 79; and its CDR1, CDR2, CDR3 are shown in SEQ ID NO: 49, 80, 51, respectively.

```
<----------FR1-------->    CDR1      <-----FR2----->  CDR2  <-----------
DIQMIQSTSSLSASLGNRVTISCRASQDISNYLNWYQQKPDGTVKLLI
YDTSRLHAGVPSRFSGSGSGT
---------FR3------>   CDR3     <---FR4--->
DYSLTISNLEQEDIATYFCQQGVTLPLTFGAGTKLEIK
```

Nucleic acid sequence

GACATCCAGATGATTCAGTCTACATCCTCCCTGTCTGCCTCTCTGGGAAACAGA

GTCACCATCAGTTGCAGGGCAAGTCAGGACATTAGCAATTATTTAAACTGGTAT

CAGCAGAAACCAGATGGAACTGTTAAACTCCTGATCTACGACACATCAAGATTA

CACGCAGGAGTCCCATCAAGGTTCAGTGGCAGTGGGTCTGGAACAGATTATTC

TCTCACCATTAGCAACCTGGAGCAAGAAGATATCGCCACTTACTTTTGCCAACA

GGGTGTTACGCTTCCTCTCACGTTCGGTGCTGGGACCAAGCTGGAAATAAAA

Clone: 1H21-D5

The amino acid sequence of 1H21-D5 heavy chain VH is shown in SEQ ID NO: 81; its encoding nucleic acid is shown in SEQ ID NO: 82; and its CDR1, CDR2, CDR3 are shown in SEQ ID NO: 83, 84, 85, respectively.

```
<-----------FR1---------->    CDR1     <----FR2----->       CDR2
<---
QVQLKQSGPGLVQPSQSLSITCTVSGFSLTSYGIHWVRQSPGKGLE
WLGVIWSGGSTDFNAAFISRLSI
------------FR3------------>    CDR3     <---FR4--->
SKDNSKSQVFFKVISLQANDTAIYYCGRNSYYGNLYAMDYWGQGT
SVTVSS
```

Nucleic acid sequence

CAGGTCCAGCTGAAGCAGTCAGGACCTGGCCTAGTGCAGCCCTCACAGAGCCT
GTCCATCACCTGCACAGTCTCTGGTTTCTCATTAACTAGCTATGGTATACACTGG
GTTCGCCAGTCTCCAGGAAAGGGTCTGGAGTGGCTGGGAGTGATATGGAGTGG
TGGAAGCACAGACTTTAATGCAGCTTTCATATCAAGACTGAGCATCAGCAAGG
ACAATTCCAAGAGCCAAGTTTTCTTTAAAGTGATCAGTCTGCAAGCTAATGACA
CAGCCATATATTACTGTGGCAGAAATTCCTACTATGGTAATCTCTATGCTATGGAC
TACTGGGGTCAAGGAACCTCAGTCACCGTCTCCTCA

The amino acid sequence of 1H21-D5 light chain VK is shown in SEQ ID NO: 86; its encoding nucleic acid is shown in SEQ ID NO: 87; and its CDR1, CDR2, CDR3 are shown in SEQ ID NO: 88, 11, 12, respectively.

```
        <----------FR1-------->          CDR1          <-----FR2----->   CDR2
        <--------
        DIVLTQSPTSLAVSLGQRATISCRASKSVSASGYSFMHWYQQKPGQ
        PPKLLIYLASNLESGVPARFSGS
        -----------FR3-------->     CDR3     <---FR4--->
        GSGTDFTLNIHPVEEEDAATYYCQHSRELPLTFGAGTKLELK
```

Nucleic acid sequence

GACATTGTGCTGACACAGTCTCCTACTTCCTTAGCTGTATCTCTGGGGCAGAGG
GCCACCATCTCATGCAGGGCCAGCAAAAGTGTCAGTGCATCTGGCTATAGTTTT
ATGCACTGGTACCAACAGAAACCAGGACAGCCACCCAAACTCCTCATCTATCTT
GCATCCAACCTAGAATCTGGGGTCCCTGCCAGGTTCAGTGGCAGTGGGTCTGG
GACAGACTTCACCCTCAACATCCATCCTGTGGAGGAGGAGGATGCTGCAACCT
ATTACTGTCAGCACAGTCGGGAGCTTCCTCTCACGTTCGGTGCTGGGACCAAG
CTGGAGCTGAAA

Clone: 10C19-D11

The amino acid sequence of 10C19-D11 heavy chain VH is shown in SEQ ID NO: 89; its encoding nucleic acid is shown in SEQ ID NO: 90; and its CDR1, CDR2, CDR3 are shown in SEQ ID NO: 5, 91, 85, respectively.

```
        <-----------FR1---------->     CDR1     <----FR2----->          CDR2
        <---
        QVQLQQSGPGLVQPSQSLSITCTVSGFSLTTYAVHWFRQSPGQGLE
        WLGVIWSTGITDFNAAFISRLNI
        ------------FR3------------>     CDR3     <---FR4--->
        NKDSSKSQIFFKMISLQPNDTAIYYCGRNSYYGNLYAMDYWGQGTSVTV
```
SS

Nucleic acid sequence

CAGGTGCAGCTGCAGCAGTCAGGACCTGGCCTAGTGCAGCCCTCACAGAGCCT
GTCCATCACCTGCACAGTCTCTGGTTTCTCATTAACTACCTATGCTGTTCACTGG
TTTCGCCAGTCTCCAGGACAGGGTCTGGAGTGGCTGGGAGTGATATGGAGTAC
TGGAATCACAGACTTTAATGCAGCTTTCATATCAAGACTGAACATCAACAAAGA
CAGTTCCAAGAGCCAGATTTTCTTTAAAATGATCAGTCTGCAACCTAATGACAC
AGCCATATATTACTGTGGCAGAAATTCCTACTATGGTAATCTCTATGCTATGGACT
ACTGGGGTCAAGGAACCTCAGTCACCGTCTCCTCA

The amino acid sequence of 10C19-D11 light chain VK is shown in SEQ ID NO: 92; its encoding nucleic acid is shown in SEQ ID NO: 93; and its CDR1, CDR2, CDR3 are shown in SEQ ID NO: 19, 11, 12, respectively.

```
        <----------FR1-------->        CDR1        <-----FR2-----> CDR2
        <--------
        DIVLTQSPTSLAVSLGQRATISCRASKSVSASGYNFMHWYQQKPGQ
        PPKLLIYLASNLESGVPARFSGS
        -----------FR3-------->    CDR3     <---FR4--->
        GSETDFTLNIHPVEEGDAATYYCQHSRELPLTFGAGTKLELK
```

Nucleic acid sequence

GACATTGTGCTGACACAGTCTCCTACTTCCTTAGCTGTATCTCTGGGGCAGAGG
GCCACCATCTCATGCAGGGCCAGTAAAAGTGTCAGTGCATCTGGCTATAATTTTA
TGCACTGGTACCAACAGAAACCAGGTCAGCCACCCAAACTCCTCATCTATCTTG
CATCCAACCTAGAATCTGGGGTCCCTGCCAGGTTCAGTGGCAGTGGGTCTGAG
ACAGACTTCACCCTCAACATCCATCCTGTGGAGGAGGGGGATGCTGCAACCTAT
TACTGTCAACACAGTCGGGAGCTTCCTCTCACGTTCGGTGCTGGGACCAAGCT
GGAGCTGAAA

Clone: 16J15-H3

The amino acid sequence of 16J15-H3 heavy chain VH is shown in SEQ ID NO: 94; its encoding nucleic acid is shown in SEQ ID NO: 95; and its CDR1, CDR2, CDR3 are shown in SEQ ID NO: 96, 97, 98, respectively.

```
<-----------FR1----------->    CDR1    <----FR2----->         CDR2
<---
QVQLKQSGPGLVQPSQSLSITCTVSNFSLSTYGVHWVRQSPGKGLE
WLGVIWGGGYTDYNAAFISRLTI
------------FR3------------>       CDR3      <---FR4--->
SKDNSKSQVFFKMNSLQADDTAIYYCARNNYYGNLYAMDYWGQG
TSVTVSS
```

Nucleic acid sequence

CAGGTCCAGCTGAAGCAGTCAGGACCTGGCCTAGTGCAGCCCTCACAGAGCCT

GTCCATCACCTGCACAGTCTCTAATTTCTCATTAAGTACCTATGGTGTACACTGG

GTTCGCCAGTCTCCAGGAAAGGGTCTGGAGTGGCTGGGAGTGATCTGGGGTGG

TGGATACACAGACTATAATGCAGCTTTCATATCCAGACTGACCATCAGCAAGGA

CAACTCCAAGAGCCAAGTTTTCTTTAAAATGAACAGTCTGCAAGCTGATGACA

CAGCCATATATTATTGTGCCAGAAATAACTACTATGGTAACCTCTATGCTATGGAC

TACTGGGGTCAAGGAACCTCAGTCACCGTCTCCTCA

The amino acid sequence of 16J15-H3 light chain VK is shown in SEQ ID NO: 99; its encoding nucleic acid is shown in SEQ ID NO: 100; and its CDR1, CDR2, CDR3 are shown in SEQ ID NO: 101, 102, 103, respectively.

```
<----------FR1-------->         CDR1         <-----FR2----->   CDR2
<--------
DIVLTQSPASLPVSLGQRATISCRANKSVSASGYSFMHWYQQKPGQ
PPKLLIYLASNLDSGVPARFSGS
-----------FR3-------->    CDR3    <---FR4--->
GSGTDFTLNIHPVEEEDTATYYCQHSREFPLTFGAGTKLELK
```

Nucleic acid sequence

GACATTGTGCTGACACAGTCTCCTGCTTCCTTACCTGTATCTCTGGGGCAGAGG

GCCACCATCTCATGCAGGGCCAACAAAGTGTCAGTGCATCTGGCTATAGTTTT

ATGCACTGGTATCAACAGAAACCAGGACAGCCACCCAAACTCCTCATCTATCTT

GCATCCAACCTAGATTCTGGGGTCCCTGCCAGGTTCAGTGGCAGTGGGTCTGG

GACAGACTTCACCCTCAACATCCATCCTGTGGAGGAGGAGGATACTGCAACCT

ATTACTGTCAGCACAGTAGGGAGTTTCCGCTCACGTTCGGTGCTGGGACCAAG

CTGGAGCTGAAA

Clone: 17A9-G5

The amino acid sequence of 17A9-G5 heavy chain VH is shown in SEQ ID NO: 104; its encoding nucleic acid is shown in SEQ ID NO: 105; and its CDR1, CDR2, CDR3 are shown in SEQ ID NO: 15, 106, 107, respectively.

```
        <-----------FR1----------->   CDR1    <----FR2----->         CDR2
        <---

QVQLKQSGPGLVQPSQSLSITCTVSGFSLTTYGVHWVRQSPGKGLE
WLGVIWSAGITDYNAPFISRLSI
------------FR3------------>        CDR3        <---FR4--->
TKDNSKSQVFFKMNSLQADDTAIYYCARNDYYGNLYAMDYWGQG
TSVTVSS
```

Nucleic acid sequence

```
CAGGTCCAGCTGAAGCAGTCAGGACCTGGCCTAGTGCAGCCCTCACAGAGCCT
GTCCATCACCTGCACAGTCTCTGGTTTCTCATTAACTACCTATGGTGTACACTGG
GTTCGCCAGTCTCCAGGAAAGGGTCTGGAGTGGCTGGGAGTGATATGGAGTGC
TGGAATCACAGACTATAATGCACCTTTCATATCCAGACTGAGCATCACCAAGGA
CAATTCCAAGAGCCAAGTTTTCTTTAAAATGAACAGTCTGCAAGCTGATGACAC
AGCCATATATTACTGTGCCAGAAATGACTACTATGGTAACCTCTATGCTATGGAC
TACTGGGGTCAAGGAACCTCAGTCACCGTCTCCTCA
```

The amino acid sequence of 17A9-G5 light chain VK is shown in SEQ ID NO: 108; its encoding nucleic acid is shown in SEQ ID NO: 109; and its CDR1, CDR2, CDR3 are shown in SEQ ID NO: 110, 111, 103, respectively.

```
        <----------FR1-------->          CDR1          <-----FR2-----> CDR2
        <--------

DIVLTQSPASLAVSLGQRATISCRANKSVSASGYTFMHWYQQKPGQ
PPKLLIYLVSNLASGVPARFSGS
-----------FR3-------->     CDR3    <---FR4--->
GSGTDFTLNIHPVEGEDAATYYCQHSREFPLTFGAGTKLELK
```

Nucleic acid sequence

GACATTGTGCTGACACAGTCTCCTGCTTCCTTAGCTGTATCTCTGGGGCAGAGG
GCCACCATCTCATGCAGGGCCAACAAGAGTGTCAGTGCATCTGGCTATACTTTT
ATGCACTGGTACCAACAGAAACCAGGACAGCCACCCAAGCTCCTCATCTATCTA
GTATCCAACCTAGCATCTGGGGTCCCTGCCAGGTTCAGTGGCAGTGGGTCTGG
GACAGACTTCACCCTCAACATCCATCCTGTGGAGGGGGAGGATGCTGCAACCT
ATTACTGTCAGCACAGTAGGGAGTTTCCGCTCACGTTCGGTGCTGGGACCAAG
CTGGAGCTGAAA

Clone: 36E7-D6

The amino acid sequence of 36E7-D6 heavy chain VH is shown in SEQ ID NO: 112; its encoding nucleic acid is shown in SEQ ID NO: 113; and its CDR1, CDR2, CDR3 are shown in SEQ ID NO: 114, 115, 116, respectively.

```
         <-----------FR1----------->    CDR1     <----FR2----->          CDR2
         <---
         QVQLKQSGPGLVQPSQSLSITCTVSDFSLTIFGVHWVRQSPGKGLE
         WLGVIWRDGSTDYNAALMSRLNI
         -----------FR3------------->     CDR3     <---FR4--->

         TKDNSKSQVFFKMNSLQTDDTAIYYCAKNRRYDYAWFPYWGQGT
         LVSVSA
```

Nucleic acid sequence

CAGGTGCAACTGAAaCAGTCAGGACCTGGCCTAGTGCAGCCCTCACAGAGCCT
GTCCATAACCTGCACAGTCTCTGATTTCTCATTAACTATCTTTGGGGTTCACTGG
GTTCGCCAGTCTCCAGGAAAGGGTCTGGAGTGGCTGGGAGTGATTTGGAGAGA
TGGAAGCACAGACTATAATGCAGCTCTCATGTCCAGACTGAATATCACCAAGGA
CAACTCCAAGAGCCAAGTTTTCTTTAAAATGAACAGTCTGCAAACTGATGACA
CTGCCATATATTACTGTGCCAAAAATCGGAGGTATGATTACGCCTGGTTTCCTTA
TTGGGGCCAAGGGACTCTGGTCTCTGTCTCTGCA

The amino acid sequence of 36E7-D6 light chain VK is shown in SEQ ID NO: 117; its encoding nucleic acid is shown in SEQ ID NO: 118; and its CDR1, CDR2, CDR3 are shown in SEQ ID NO: 119, 120, 121, respectively.

```
<----------FR1-------->        CDR1         <-----FR2----->  CDR2
<--------
DIVLTQSPASLAVSLGQRATISCRASKSVSASGYSFLHWYQQKPGQP
PKLLIYLTSNLESGVPARFSGS
-----------FR3-------->    CDR3   <---FR4--->
 GSGTDFTLNIHPVEEEDAATYYCQHSRDFPPTFGAGTKLELK
```

Nucleic acid sequence

GACATTGTGCTGACACAGTCTCCTGCTTCCTTAGCTGTATCTCTGGGGCAGAGG

GCCACCATCTCATGCAGGGCCAGCAAAAGTGTCAGTGCATCTGGCTATAGTTTT

TTGCACTGGTACCAACAGAAACCAGGACAGCCACCCAAACTCCTCATTTATCTT

ACATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGACTTTCCTCCCACGTTCGGGGCTGGGACCAAG

CTGGAGCTGAAA

Clone: 4H6-A6

The amino acid sequence of 4H6-A6 heavy chain VH is shown in SEQ ID NO: 122; its encoding nucleic acid is shown in SEQ ID NO: 123; and its CDR1, CDR2, CDR3 are shown in SEQ ID NO: 15, 124, 16, respectively.

```
<-----------FR1----------->  CDR1    <----FR2----->        CDR2
<---
QVQLKQSGPGLVQPSQKLSITCTVSGFSLTTYGVHWIRQSPVKGLE
WLGVIWTTGVVDYNAAFISRLNI
------------FR3------------>    CDR3      <---FR4--->
TKDNSKSQIFFKMNSLQTNDTAMYYCARNSYYGNLYVMDYWGQG
TSVTVSS
```

Nucleic acid sequence

CAGGTGCAACTGAAaCAGTCAGGACCTGGCCTAGTGCAGCCCTCACAGAAGTT
GTCCATTACCTGCACAGTCTCTGGTTTCTCATTAACTACCTATGGTGTGCACTGG
ATTCGCCAGTCTCCAGTAAAGGGTCTGGAGTGGCTGGGAGTGATATGGACTACT
GGAGTTGTAGACTATAATGCAGCTTTCATATCCAGACTGAACATCACCAAGGAC
AATTCCAAGAGCCAAATTTTCTTTAAAATGAACAGTCTGCAAACTAATGACACA
GCCATGTATTACTGTGCCAGAAATTCCTACTATGGAAACCTCTATGTTATGGACT
ACTGGGGTCAAGGAACCTCAGTCACCGTCTCCTCA

The amino acid sequence of 4H6-A6 light chain VK is shown in SEQ ID NO: 125; its encoding nucleic acid is shown in SEQ ID NO: 126; and its CDR1, CDR2, CDR3 are shown in SEQ ID NO: 127, 128, 103, respectively.

```
<----------FR1--------->          CDR1          <-----FR2-----> CDR2
<--------
DIVLTQSPASLPIFLGQRATISCRASRSVSASGYTFMHWYQQKPGQP
PKLLIYLVSNLESGVPARFSGS
-----------FR3-------->    CDR3    <---FR4--->
GSGTDFTLNIHPVEEEDAATYYCQHSREFPLTFGAGTKLELK
```

Nucleic acid sequence

GACATTGTGCTGACACAGTCTCCTGCTTCCTTACCTATATTTCTGGGGCAGAGG
GCCACCATCTCATGCAGGGCCAGCAGAAGTGTCAGTGCATCTGGCTATACTTTT
ATGCACTGGTACCAACAGAAACCAGGACAGCCACCCAAACTCCTCATCTATCTT
GTATCCAACCTAGAATCTGGGGTCCCTGCCAGGTTCAGTGGCAGTGGGTCTGG
GACAGACTTCACCCTCAACATCCATCCTGTGGAGGAGGAGGATGCTGCAACCT
ATTACTGTCAGCACAGTAGGGAGTTTCCTCTCACGTTCGGTGCTGGGACCAAG
CTGGAGCTGAAA

Clone: 42H19-G4

The amino acid sequence of 42H19-G4 heavy chain VH is shown in SEQ ID NO: 129; its encoding nucleic acid is shown in SEQ ID NO: 130; and its CDR1, CDR2, CDR3 are shown in SEQ ID NO: 131, 132, 116, respectively.

```
<-----------FR1---------->    CDR1    <----FR2-----> CDR2
<---
QVQLKQSGPGPVQPSQSLSITCTVSDFSLTIFGIHWVRQSPGKGLEW
LGVIWRDGSTDYNVALMSRLNI
------------FR3------------>    CDR3    <---FR4--->
TKDNSKSQVFFKMNSLQTDDTAIYYCAKNRRYDYAWFPYWGQGT
LVSVSA
```

Nucleic acid sequence

CAGGTGCAACTGAAaCAGTCAGGACCTGGCCCAGTGCAGCCCTCACAGAGCCT

ATCCATAACCTGCACAGTCTCTGATTTCTCATTAACTATCTTTGGGATTCACTGG

GTTCGCCAGTCTCCAGGAAAGGGTCTGGAGTGGCTGGGAGTGATTTGGAGAGA

TGGAAGCACAGACTACAATGTAGCTCTCATGTCCAGACTGAACATCACCAAGG

ACAACTCCAAGAGTCAAGTTTTCTTTAAAATGAACAGTCTACAAACTGATGAC

ACTGCCATATACTACTGTGCCAAAAATCGGAGGTATGATTACGCCTGGTTTCCTT

ACTGGGGCCAAGGGACTCTGGTCtCTGTCTCTGCA

The amino acid sequence of 42H19-G4 light chain VK is shown in SEQ ID NO: 117; its encoding nucleic acid is shown in SEQ ID NO: 118; and its CDR1, CDR2, CDR3 are shown in SEQ ID NO: 119, 120, 121, respectively.

```
<----------FR1-------->        CDR1        <-----FR2----->  CDR2
<--------
DIVLTQSPASLAVSLGQRATISCRASKSVSASGYSFLHWYQQKPGQP
PKLLIYLTSNLESGVPARFSGS
-----------FR3-------->    CDR3     <---FR4--->
 GSGTDFTLNIHPVEEEDAATYYCQHSRDFPPTFGAGTKLELK
```

Nucleic acid sequence

GACATTGTGCTGACACAGTCTCCTGCTTCCTTAGCTGTATCTCTGGGGCAGAGG

GCCACCATCTCATGCAGGGCCAGCAAAAGTGTCAGTGCATCTGGCTATAGTTTT

TTGCACTGGTACCAACAGAAACCAGGACAGCCACCCAAACTCCTCATTTATCTT

ACATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGACTTTCCTCCCACGTTCGGGGCTGGGACCAAG

CTGGAGCTGAAA

The heavy chain variable region was cloned into a vector comprising a human heavy chain constant region and regulatory elements to express the entire IgG heavy chain in a mammalian cell. Similarly, the light chain variable region was cloned into a vector comprising a human light chain constant region and regulatory elements to express the entire IgG light chain in a mammalian cell. After correct sequencing, the cells were transfected into a CHO-S mammalian cell. IgG was expressed and secreted into culture medium. The supernatant was collected and purified after filtration. The IgG was purified by Protein A chromatography. The culture supernatant was loaded onto a Protein A column with an appropriate size and washed with 50 mM Tris-HCl with a pH of 8.0 and 250mM NaCl. The bound IgG was eluted with 0.1 M Glycine-HCl (pH 3.0). The protein was concentrated by ultrafiltration using a concentration tube. The OD280 was measured, and the concentration of IgG was determined by spectrophotometry.

4. CDC activity of chimeric antibody on Daudi cell

Daudi cells were resuspended and plated in a 96-well plate at 50 μL/well followed by the addition of 50 μL antibody per well. The chimeric antibody was diluted to 10 μg/mL as the starting concentration in 3-fold gradients for a total of 3 or 4 gradients. 50 μL of complement mixture solution was added to each well and the 96-well plate was placed in a 37°C and 5% $CO_2$ incubator for 2 hours. Then 50 μL of 40% CCK-8 solution was added to each well, shaken for 10 seconds, and incubated in a 5% $CO_2$ incubator for 3 hours. The 96-well plate was placed in a microplate reader (BioTek, Synergy HT) and OD450 values were read. The data was collected to calculate results by using the GraphPad Prism 5 software. See FIG.s 4-1 and 4-2, and Table 4 for the results. Chimeric antibody 39H5 showed very strong CDC activity with a maximum killing of tumor cells of close to 80% at a concentration of 66.7 nM.

Table 4. CD70 monoclonal antibodies CDC activity screening

| CD70 Antibodies | CDC activity |
|---|---|
| 2B12 | + |
| 3B2 | + |
| 12A1 | + |
| 14C12 | ++ |
| 23A11 | + |
| 25H7 | + |
| 26G11 | + |
| 27A3 | ++ |
| 32D1 | + |
| 39H5 | +++ |
| 1H21-D5 | + |
| 10C19-D11 | + |
| 16J15-H3 | + |
| 17A9-G5 | + |
| 36E7-D6 | + |
| 4H6-A6 | + |
| 42H19-G4 | + |

5. ADCC Killing Activity of Chimeric Antibody on Duadi Cell

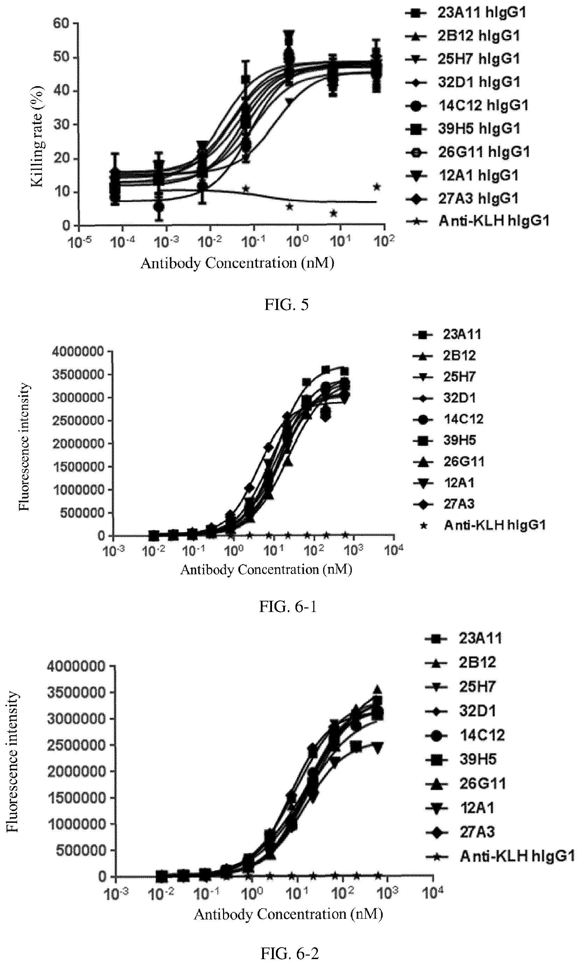

50 μL of Daudi cells were plated into a 96-well U-plate at a density of $1 \times 10^6$/well. 50 μL of chimeric antibody was added per well, and incubated at a 37°C and 5% $CO_2$ incubator for 30 minutes. NK92-MI-CD16a cells were taken and added to a RPMI 1640 medium. The cells were counted and centrifuged at 300 g for 10 minutes. The supernatant was discarded. The cells were resuspended to a density of $2 \times 10^6$/mL with the RPMI-1640 medium. The 96-well plate was taken out, added with 50 μL of NK92-MI-CD16a cells per well, and incubated in a 5% $CO_2$ incubator for 4 hours. Thirty minutes prior to assay, 2 μL of cell lysate (100×) was added to the maximal well of target cells, and the incubation was continued at 37°C. The centrifuge was performed at 300 * g for 3 minutes. 50 μL of the supernatant was taken out and added to a black microtiter plate. 50 μL of LDH detection substrate (placed at room temperature to dissolve in advance) was added and mixed evenly by gently shaking. After 10 minutes, 25 μL of a termination solution was added to terminate the reaction. After shaking for 10 seconds, a fluorescence (excitation wavelength 560 nm, emission wavelength 590 nm) was selected for plate reading. See FIG. 5 and Table 5 for the results. The chimeric antibody had a significant ADCC killing effect on Daudi cells in a dose-dependent manner.

Table 5. CD70 monoclonal antibody ADCC activity screening

| CD70 Antibodies | ADCC killing activity EC50(nM) |
|---|---|
| 2B12 | 0.03 |
| 3B2 | ND |
| 12A1 | 0.04 |
| 14C12 | 0.08 |
| 23A11 | 0.02 |

(continued)

| CD70 Antibodies | ADCC killing activity EC50(nM) |
|---|---|
| 25H7 | 0.32 |
| 26G11 | 0.11 |
| 27A3 | 0.05 |
| 32D1 | 0.07 |
| 39H5 | 0.05 |
| ND: not detected | |

6. Chimeric antibody FACS cell affinity assay

Different cells in logarithmic growth phase were taken, blocked with 3% BSA for 30 minutes, and plated to a 96-well U-plate at $5 \times 10^4$ cells /100 $\mu$L. The centrifuge was performed at 1100 rpm for 3 minutes. The supernatant was discarded and the cells were gently tapped to be loosening. 50 $\mu$L of gradient diluted antibody was added (antibody concentration: from 30 $\mu$g/mL, 3-fold dilution for 5 gradients) per well, and incubated at 4°C for 1 hour. After incubation, 180 $\mu$L of 0.5% BSA was added to each well for washing 3 times. 30$\mu$L/well of secondary antibody AlexaFluro647 anti-human IgG (Jackson Immuno Research, Cat. No: 109-606-170) was added and incubated at 4°C for 45 minutes. At the end of the incubation, each well was washed 3 times with 180$\mu$L of 0.5% BSA and finally resuspended in 50$\mu$L of PBS for iQue (Intellicyt, USA) assay. As shown in FIGs. 6-1, 6-2, and Table 6, FACS can detect high-affinity binding of the chimeric antibody to CD70 molecules expressed on the transfected cell lines CHO-human CD70 and CHO-cynomolgus monkey CD70, respectively.

Table 6. FACS Cell Affinity Assay

| CD70 Antibodies | CHO-Human CD70 EC50(nM) | CHO-Cynomolgus Monkey CD70 EC50 (nM) |
|---|---|---|
| 2B12 | 17.73 | 22.02 |
| 3B2 | ND | ND |
| 12A1 | 7.55 | 14.0 |
| 14C12 | 14.97 | 14.35 |
| 23A11 | 11.88 | 9.63 |
| 25H7 | 16.12 | 17.38 |
| 26G11 | 22.48 | 20.97 |
| 27A3 | 4.07 | 7.28 |
| 32D1 | 12.05 | 15.84 |
| 39H5 | 10.18 | 15.13 |
| ND: not detected | | |

7. Blocking effect of chimeric antibody on CD70 binding to CD27

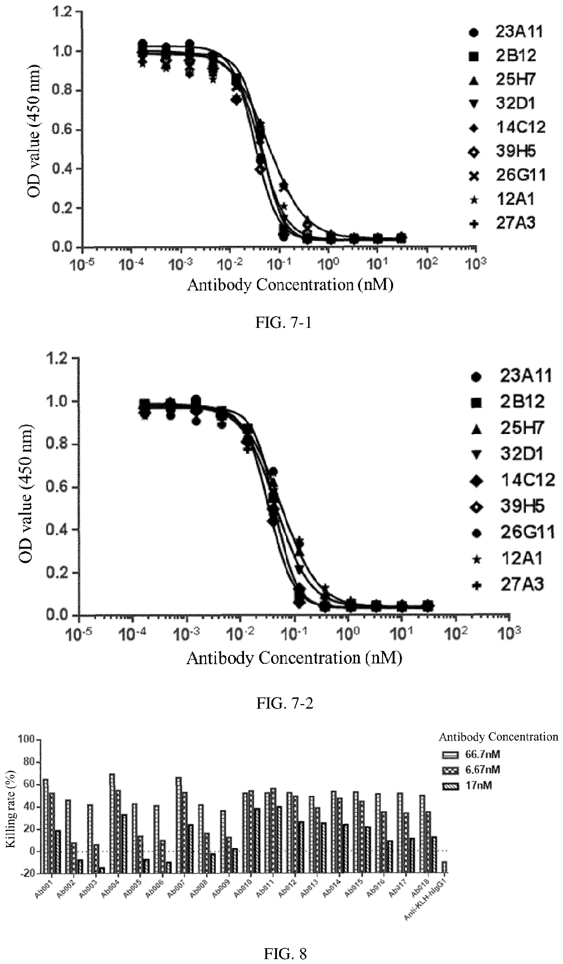

The recombinant CD27 protein was coated at 1$\mu$g/mL in a 96-well microtiter plate and incubated overnight at 4°C. The next day, the plate was washed 3 times with PBS, added with 200$\mu$L of 2% defatted dry milk/PBS, blocked at room temperature for 2 hours, and washed once with PBS. The purified chimeric antibody was divided to 12 concentration gradients starting at 100nM and diluted in a 1: 3 gradient. 30$\mu$l of the antibody was added per well and incubated at room temperature for 30 minutes. 30$\mu$L of biotin-labeled recombinant CD70 protein was then added to each well and incubated at room temperature for 60 minutes. The plate was washed 3 times with PBST and PBS and dried by spinning. A secondary antibody Streptavidin-coupled horseradish peroxidase SA-HRP (BD bioscience, cat. No: 6222697) was diluted with 0.5% BSA in a ratio of 1:8000. 100$\mu$L of the diluent was added into each well and incubated at room temperature for 30 minutes. The plate was washed 3 times with PBST and PBS and dried by spinning. 100$\mu$l of color development solution (TMB solution, Sigma, Cat. No: T2885) was added into each well and protected from light at 37°C for reaction. After the substrate developed moderately, 50$\mu$l of $H_2SO_4$ was added into each well to terminate the reaction. The OD450nm was determined by using an enzyme-linked detector within 30 minutes, and the data was collected. The results were calculated by using Graph Pad Prism 5 software. See FIGs. 7-1 and 7-2 and Table 7 for the results. The chimeric antibody can block the binding of human CD70 and CD27, as well as the cynomolgus monkey CD70 and CD27.

Table 7. Blocking effect assay on binding of human CD70 to CD27

| CD70 Antibodies | Human CD70/CD27 IC50(nM) | Cynomolgus Monkey CD70/CD27 IC50(nM) |
|---|---|---|
| 2B12 | 0.11 | 0.11 |
| 3B2 | ND | ND |
| 12A1 | 0.12 | 0.21 |
| 14C12 | 0.10 | 0.09 |
| 23A11 | 0.07 | 0.08 |
| 25H7 | 0.16 | 0.15 |
| 26G11 | 0.17 | 0.21 |
| 27A3 | 0.08 | 0.08 |
| 32D1 | 0.10 | 0.11 |
| 39H5 | 0.06 | 0.08 |

ND: not detected

## Example 5. Humanization of anti-CD70 antibodies

Mouse antibodies 39H5 and 32D1 with higher CDC activity were selected for the humanization of variable region sequences. Firstly, the sequence of mouse antibody 39H5 was aligned with that of human antibody germline to find out that the key amino acid sequences with good homology and maintaining the structural core (Upper hydrophobic core) of the antibodies were completely the same, at the same time, the mouse antibody CDR was transplanted in human germline light chain genes IMGT_hVK1-33 and IGKJ1*01 and human germline heavy chain genes IMGT_hVH7-4 and IGHJ4*01 with high frequency. At the same time, the sequence of mouse antibody 32D1 was aligned with that of human antibody germline to find out that the key amino acid sequences with good homology and maintaining the structural core of the antibody were completely the same, at the same time, the mouse antibody CDR was transplanted in human germline light chain genes IMGT_hVK7-3, IMGT_hVK3-20, and IGKJ1*01, and human germline heavy chain genes IMGT_hVH1-69 and IGHJ4*01 with high frequency. Using computer for homology modeling, the CDR region and its surrounding framework amino acid homology modeling to avoid the concentrated distribution of molecular surface charge or hydrophobic region.

A total of 6 heavy chain variants h39H5-VHv1-v3, h32D1-VHv1-v3 and 6 light chain variants h39H5-VKv1-v3, h32D1-VKv1-v3 were designed. The light and heavy chains were synthesized respectively and cloned into a eukaryotic expression vector comprising antibody kappa chain constant region Ckappa or human IgG1 constant region CH1-CH3. The light and heavy chain plasmids were combined and paired, then transfected into CHO-S cells and expressed at 37°C for 5-6 days. The culture supernatant was collected and purified by a Protein A column.

The humanized antibody heavy/light chain variable region sequences are as follows:

Table 8. Heavy chain variable region sequences of CD70 humanized antibodies

| CD3 humanized antibodies | SEQ ID NOs: | | | | |
|---|---|---|---|---|---|
| | HCVR | | HCDR1 | HCDR2 | HCDR3 |
| | Amino Acid Sequence | Nucleotide Sequence | Amino Acid Sequence | Amino Acid Sequence | Amino Acid Sequence |
| h39H5 VHv1 | 133 | 134 | 44 | 135 | 46 |
| h39H5 VHv2 | 136 | 137 | 44 | 135 | 46 |
| h39H5 VHv3 | 138 | 139 | 44 | 135 | 46 |
| h32D1 VHv1 | 140 | 141 | 29 | 142 | 31 |
| h32D1 VHv2 | 143 | 144 | 29 | 142 | 31 |
| h32D1 VHv3 | 145 | 146 | 29 | 142 | 31 |

Table 9. Light chain variable region sequences of CD70 humanized antibodies

| CD3 humanized antibodies | SEQ ID NOs: | | | | |
| --- | --- | --- | --- | --- | --- |
| | LCVR | | LCDR1 | LCDR2 | LCDR3 |
| | Amino Acid Sequence | Nucleotide Sequence | Amino Acid Sequence | Amino Acid Sequence | Amino Acid Sequence |
| h39H5 VKv1 | 147 | 148 | 149 | 80 | 51 |
| h39H5 VKv2 | 150 | 151 | 149 | 80 | 51 |
| h39H5 VKv3 | 152 | 153 | 149 | 80 | 51 |
| h32D1 VKv1 | 154 | 155 | 25 | 11 | 26 |
| h32D1 VKv2 | 156 | 157 | 25 | 11 | 26 |
| h32D1 VKv3 | 158 | 159 | 25 | 160 | 26 |

h39H5 VHv1

The amino acid sequence of the heavy chain VH is shown in SEQ ID NO: 133; its encoding nucleic acid is shown in SEQ ID NO: 134; and its CDR1, CDR2, CDR3 are shown in SEQ ID NO: 44, 135, 46, respectively.

```
        <-------------FR1--------->    CDR1    <----FR2----->          CDR2
        <--
        QIQLVQSGSELKKPGASVKVSCKASGYTFTNYGMNWVRQAPGQGL
        EWMGWINTYTGEPTYADGFTGRFV
        ------------FR3------------->     CDR3    <---FR4--->
        FSLDTSASTAYLQISSLKAEDTAVYFCAREEDYRYVFNYWGQGTLV
        TVSS
```

Nucleic acid sequence

```
        CAGATTCAGCTGGTGCAGTCTGGCAGCGAGCTGAAGAAACCTGGCGCCTCTGT
        GAAGGTGTCCTGCAAGGCTAGCGGCTACACATTCACCAACTACGGCATGAACT
        GGGTCCGACAGGCTCCTGGACAGGGACTCGAATGGATGGGCTGGATCAACACC
        TACACCGGCGAGCCTACCTACGCCGATGGCTTCACAGGCAGATTCGTGTTCAGC
        CTGGACACCAGCGCCAGCACAGCTTACCTGCAGATCAGCTCTCTGAAGGCCGA
        GGATACCGCCGTGTACTTCTGCGCCAGAGAAGAGGACTACCGCTACGTGTTCA
        ACTACTGGGGCCAGGGCACACTGGTCACCGTTTCTTCT
```

h39H5 VHv2

The amino acid sequence of the heavy chain VH is shown in SEQ ID NO: 136; its encoding nucleic acid is shown in SEQ ID NO: 137; and its CDR1, CDR2, CDR3 are shown in SEQ ID NO: 44, 135, 46, respectively.

```
<-------------FR1-------->    CDR1    <----FR2----->         CDR2
<--
QVQLVQSGSELKKPGASVKVSCKASGYTFTNYGMNWVRQAPGQG
LEWMGWINTYTGEPTYADGFTGRFV
------------FR3------------->    CDR3    <---FR4--->
FSLDTSASTAYLQISSLKAEDTAVYFCAREEDYRYVFNYWGQGTLV
TVSS
```

Nucleic acid sequence

```
CAGGTTCAGCTGGTGCAGTCTGGCAGCGAGCTGAAGAAACCTGGCGCCTCTGT
GAAGGTGTCCTGCAAGGCTAGCGGCTACACATTCACCAACTACGGCATGAACT
GGGTCCGACAGGCTCCTGGACAGGGACTCGAATGGATGGGCTGGATCAACACC
TACACCGGCGAGCCTACCTACGCCGATGGCTTCACAGGCAGATTCGTGTTCAGC
CTGGACACCAGCGCCAGCACAGCTTACCTGCAGATCAGCTCTCTGAAGGCCGA
GGATACCGCCGTGTACTTCTGCGCCAGAGAAGAGGACTACCGCTACGTGTTCA
ACTACTGGGGCCAGGGCACACTGGTCACCGTTTCTTCT
```

h39H5 VHv3

The amino acid sequence of the heavy chain VH is shown in SEQ ID NO: 138; its encoding nucleic acid is shown in SEQ ID NO: 139; and its CDR1, CDR2, CDR3 are shown in SEQ ID NO: 44, 135, 46, respectively.

```
<-------------FR1--------->    CDR1    <----FR2----->         CDR2
<--
QVQLVQSGSELKKPGASVKVSCKASGYTFTNYGMNWVRQAPGQG
LEWMGWINTYTGEPTYADGFTGRFV
------------FR3------------->    CDR3    <---FR4--->
FSLDTSASTAYLQISSLKAEDTAVYYCAREEDYRYVFNYWGQGTLV
TVSS
```

Nucleic acid sequence

CAGGTTCAGCTGGTGCAGTCTGGCAGCGAGCTGAAGAAACCTGGCGCCTCTGT
GAAGGTGTCCTGCAAGGCTAGCGGCTACACATTCACCAACTACGGCATGAACT
GGGTCCGACAGGCTCCTGGACAGGGACTCGAATGGATGGGCTGGATCAACACC
TACACCGGCGAGCCTACCTACGCCGATGGCTTCACAGGCAGATTCGTGTTCAGC
CTGGACACCAGCGCCAGCACAGCTTACCTGCAGATCAGCTCTCTGAAGGCCGA
GGATACCGCCGTGTACTACTGCGCCAGAGAAGAGGACTACCGCTACGTGTTCA
ACTACTGGGGCCAGGGCACACTGGTCACCGTTTCTTCT

h32D1 VHv1

The amino acid sequence of the heavy chain VH is shown in SEQ ID NO: 140; its encoding nucleic acid is shown in SEQ ID NO: 141; and its CDR1, CDR2, CDR3 are shown in SEQ ID NO: 29, 142, 31, respectively.

```
        <-------------FR1--------->    CDR1      <----FR2----->           CDR2
        <--
        QVQLVQSGAEVKKPGSSVKVSCKASG YTFTDYVIH WVRQAPGQGL
        EWMG YLHPYNDDTKYNEKFQG RVT
        ------------FR3------------->     CDR3      <---FR4--->
        LTSDKSSSTAYMELSSLRSEDTAVYYCAR RGYYDYAWFAD WGQGT
        LVTVSS
```

Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACATTCACCGACTACGTGATCCACT
GGGTCCGACAGGCTCCAGGACAGGGACTTGAGTGGATGGGCTATCTGCACCCC
TACAACGACGACACCAAGTACAACGAGAAGTTCCAGGGCAGAGTGACCCTCA
CCAGCGACAAGTCTAGCAGCACCGCCTACATGGAACTGAGCAGCCTGAGAAG
CGAGGACACCGCCGTGTACTACTGTGCCAGAAGAGGCTACTACGACTACGCTT
GGTTTGCCGATTGGGGCCAGGGAACCCTGGTCACAGTTAGCTCT

h32D1 VHv2

The amino acid sequence of the heavy chain VH is shown in SEQ ID NO: 143; its encoding nucleic acid is shown in SEQ ID NO: 144; and its CDR1, CDR2, CDR3 are shown in SEQ ID NO: 29, 142, 31, respectively.

```
<------------FR1-------->    CDR1      <----FR2----->          CDR2
<--
QVQLVQSGAEVKKPGSSVKVSCKASGYTFTDYVIHWVRQAPGQGL
EWMGYLHPYNDDTKYNEKFQGRVT
------------FR3------------->    CDR3      <---FR4--->
ITADKSSSTAYMELSSLRSEDTAVYYCARRGYYDYAWFADWGQGTLVT
VSS
```

Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACATTCACCGACTACGTGATCCACT
GGGTCCGACAGGCTCCAGGACAGGGACTTGAGTGGATGGGCTATCTGCACCCC
TACAACGACGACACCAAGTACAACGAGAAGTTCCAGGGCAGAGTGACCATCA
CCGCCGACAAGTCTAGCAGCACCGCCTACATGGAACTGAGCAGCCTGAGAAGC
GAGGACACCGCCGTGTACTACTGTGCCAGAAGAGGCTACTACGACTACGCTTG
GTTTGCCGATTGGGGCCAGGGAACCCTGGTCACAGTTAGCTCT

h32D1 VHv3

The amino acid sequence of the heavy chain VH is shown in SEQ ID NO: 145; its encoding nucleic acid is shown in SEQ ID NO: 146; and its CDR1, CDR2, CDR3 are shown in SEQ ID NO: 29, 142, 31, respectively.

```
<------------FR1-------->    CDR1      <----FR2----->          CDR2
<--
QVQLVQSGAEVKKPGSSVKVSCKASGYTFTDYVIHWVRQAPGQGL
EWMGYLHPYNDDTKYNEKFQGRVT
------------FR3------------->    CDR3      <---FR4--->
ITADESTSTAYMELSSLRSEDTAVYYCARRGYYDYAWFADWGQGT
LVTVSS
```

Nucleic acid sequence

CAGGTTCAGCTGGTTCAGTCTGGCGCCGAAGTGAAGAAACCTGGCAGCAGCG

TGAAGGTGTCCTGCAAGGCTAGCGGCTACACATTCACCGACTACGTGATCCACT

GGGTCCGACAGGCTCCAGGACAGGGACTTGAGTGGATGGGCTATCTGCACCCC

TACAACGACGACACCAAGTACAACGAGAAGTTCCAGGGCAGAGTGACCATCA

CCGCCGACGAGTCTACCAGCACCGCCTACATGGAACTGAGCAGCCTGAGAAGC

GAGGACACCGCCGTGTACTACTGTGCCAGAAGAGGCTACTACGACTACGCTTG

GTTTGCCGATTGGGGCCAGGGAACCCTGGTCACAGTTAGCTCT

h39H5 VKv1

The amino acid sequence of the light chain VH is shown in SEQ ID NO: 147; its encoding nucleic acid is shown in SEQ ID NO: 148; and its CDR1, CDR2, CDR3 are shown in SEQ ID NO: 149, 80, 51, respectively.

```
       <----------FR1-------->     CDR1      <-----FR2----->  CDR2  <------------
       DIQMTQSPSSLSASVGDRVTITCQASQDISNYLNWYQQKPGKAPKL
       LIYDTSRLHAGVPSRFSGSGSGT
       ----FR3----------->    CDR3    <---FR4--->
       DYTFTISSLQPEDIATYFCQQGVTLPLTFGQGTKLEIK
```

Nucleic acid sequence

GACATCCAGATGACACAGAGCCCTAGCAGCCTGTCTGCCAGCGTGGGAGACAG

AGTGACCATCACCTGTCAGGCCAGCCAGGACATCAGCAACTACCTGAACTGGT

ATCAGCAGAAGCCCGGCAAGGCCCCTAAGCTGCTGATCTACGACACCAGCAGA

CTGCACGCTGGCGTGCCATCTAGATTCAGCGGCTCTGGCTCTGGCACCGACTAC

ACCTTCACAATCAGCAGCCTGCAGCCTGAGGATATCGCTACCTACTTCTGCCAG

CAAGGCGTGACCCTGCCTCTGACATTTGGCCAGGGCACCAAGCTGGAAATCAA

G

h39H5 VKv2

The amino acid sequence of the light chain VH is shown in SEQ ID NO: 150; its encoding nucleic acid is shown in SEQ ID NO: 151; and its CDR1, CDR2, CDR3 are shown in SEQ ID NO: 149, 80, 51, respectively.

```
       <----------FR1-------->     CDR1      <-----FR2----->  CDR2  <------------
       DIQMTQSPSSLSASVGDRVTITCQASQDISNYLNWYQQKPGKAPKL
       LIYDTSRLHAGVPSRFSGSGSGT
       ----FR3----------->    CDR3    <---FR4--->
       DFTFTISSLQPEDIATYFCQQGVTLPLTFGQGTKLEIK
```

Nucleic acid sequence

GACATCCAGATGACACAGAGCCCTAGCAGCCTGTCTGCCAGCGTGGGAGACAG
AGTGACCATCACCTGTCAGGCCAGCCAGGACATCAGCAACTACCTGAACTGGT
ATCAGCAGAAGCCCGGCAAGGCCCCTAAGCTGCTGATCTACGACACCAGCAGA
CTGCACGCTGGCGTGCCATCTAGATTCAGCGGCTCTGGCTCTGGCACCGACTTC
ACCTTCACAATCAGCAGCCTGCAGCCTGAGGATATCGCTACCTACTTCTGCCAG
CAAGGCGTGACCCTGCCTCTGACATTTGGCCAGGGCACCAAGCTGGAAATCAA
G

h39H5 VKv3

The amino acid sequence of the light chain VH is shown in SEQ ID NO: 152; its encoding nucleic acid is shown in SEQ ID NO: 153; and its CDR1, CDR2, CDR3 are shown in SEQ ID NO: 149, 80, 51, respectively.

```
       <----------FR1-------->    CDR1      <-----FR2----->  CDR2   <------------
       DIQMTQSPSSLSASVGDRVTITCQASQDISNYLNWYQQKPGKAPKL
       LIYDTSRLHAGVPSRFSGSGSGT
       ----FR3----------->    CDR3    <---FR4--->
        DFTFTISSLQPEDIATYYCQQGVTLPLTFGQGTKLEIK
```

Nucleic acid sequence

GACATCCAGATGACACAGAGCCCTAGCAGCCTGTCTGCCAGCGTGGGAGACAG
AGTGACCATCACCTGTCAGGCCAGCCAGGACATCAGCAACTACCTGAACTGGT
ATCAGCAGAAGCCCGGCAAGGCCCCTAAGCTGCTGATCTACGACACCAGCAGA
CTGCACGCTGGCGTGCCATCTAGATTCAGCGGCTCTGGCTCTGGCACCGACTTC
ACCTTCACAATCAGCAGCCTGCAGCCTGAGGATATCGCTACCTACTaCTGCCAG
CAAGGCGTGACCCTGCCTCTGACATTTGGCCAGGGCACCAAGCTGGAAATCAA
G

h32D1 VKv1

The amino acid sequence of the light chain VH is shown in SEQ ID NO: 154; its encoding nucleic acid is shown in SEQ ID NO: 155; and its CDR1, CDR2, CDR3 are shown in SEQ ID NO: 25, 11, 26, respectively.

```
       <----------FR1-------->        CDR1        <-----FR2----->  CDR2
              <--------
```

DIVLTQSPASLAVSPGQRATITC**RASKSVSASGYTWMH**WYQQKPG
QPPKLLIY**LASNLES**GVPARFSGS
----FR3--------------->     CDR3   <---FR4--->
GSGTDFTLTINPVEANDTANYYC**QHSRELPPT**FGQGTKLEIK

Nucleic acid sequence

GACATCGTGCTGACACAGAGCCCTGCTTCTCTGGCTGTGTCTCCTGGCCAGAG

AGCCACCATCACCTGTAGAGCCAGCAAGAGCGTGTCCGCCTCTGGCTACACAT

GGATGCACTGGTATCAGCAGAAGCCCGGCCAGCCTCCTAAGCTGCTGATCTACC

TGGCCAGCAACCTGGAAAGCGGAGTGCCTGCTAGATTCAGCGGCTCTGGCTCT

GGCACCGACTTCACCCTGACAATCAACCCCGTGGAAGCCAACGACACCGCCA

ACTACTACTGCCAGCACAGCAGAGAGCTGCCTCCAACATTTGGCCAGGGCACC

AAGCTGGAAATCAAG

h32D1 VKv2

The amino acid sequence of the light chain VH is shown in SEQ ID NO: 156; its encoding nucleic acid is shown in SEQ ID NO: 157; and its CDR1, CDR2, CDR3 are shown in SEQ ID NO: 25, 11, 26, respectively.

<----------FR1-------->       CDR1      <-----FR2----->   CDR2
<--------
EIVLTQSPGTLSLSPGERATLSC**RASKSVSASGYTWMH**WYQQKPGQ
APRLLIY**LASNLES**GIPDRFSGS
----FR3--------------->     CDR3   <---FR4--->
GSGTDFTLTISRLEPEDFAVYYC**QHSRELPPT**FGQGTKLEIK

Nucleic acid sequence

GAGATCGTGCTGACACAGAGCCCTGGCACACTGTCACTGTCTCCAGGCGAGAG

AGCCACACTGAGCTGTAGAGCCAGCAAGAGCGTGTCCGCCTCTGGCTACACAT

GGATGCACTGGTATCAGCAGAAGCCCGGCCAGGCTCCTAGACTGCTGATCTACC

TGGCCAGCAACCTGGAAAGCGGCATCCCCGATAGATTCAGCGGCTCTGGCTCT

GGCACCGACTTCACCCTGACAATCAGCAGACTGGAACCCGAGGACTTCGCCGT

GTACTACTGCCAGCACAGCAGAGAGCTGCCTCCAACATTTGGCCAGGGCACCA

AGCTGGAAATCAAG

h32D1 VKv3

The amino acid sequence of the light chain VH is shown in SEQ ID NO: 158; its encoding nucleic acid is shown in SEQ ID NO: 159; and its CDR1, CDR2, CDR3 are shown in SEQ ID NO: 25, 160, 26, respectively.

           <----------FR1-------->        CDR1       <-----FR2----->   CDR2

           <--------

EIVLTQSPGTLSLSPGERATLSC**RASKSVSASGYTWMH**WYQQKPGQ
APRLLIY**LASSLES**GIPDRFSGS

           ----FR3--------------->   CDR3  <---FR4--->
GSGTDFTLTISRLEPEDFAVYYC**QHSRELPPT**FGQGTKLEIK

Nucleic acid sequence

GAGATCGTGCTGACACAGAGCCCTGGCACACTGTCACTGTCTCCAGGCGAGAG
AGCCACACTGAGCTGTAGAGCCAGCAAGAGCGTGTCCGCCTCTGGCTACACAT
GGATGCACTGGTATCAGCAGAAGCCCGGCCAGGCTCCTAGACTGCTGATCTACC
TGGCCAGCAgCCTGGAAAGCGGCATCCCCGATAGATTCAGCGGCTCTGGCTCTG
GCACCGACTTCACCCTGACAATCAGCAGACTGGAACCCGAGGACTTCGCCGTG
TACTACTGCCAGCACAGCAGAGAGCTGCCTCCAACATTTGGCCAGGGCACCAA
GCTGGAAATCAAG

**Example 6. CDC killing of different humanized CD70 antibodies on tumor cell Daudi**

Daudi cells were resuspended and plated in a 96-well plate at 50μL/well followed by the addition of 50μL antibodies per well. The humanized antibodies were diluted to 10μg/mL as a starting concentration, and performed 3-fold gradient dilution, for a total of 3 gradients. 50μL of complement mixture solution was added to each well and the 96-well plate was placed in a 37°C and 5% $CO_2$ incubator for 2 hours. Then 50μL of 40% CCK-8 solution was added to each well, shaken for 10 seconds, and incubated in a 5% $CO_2$ incubator for 3 hours. The 96-well plate was placed in a microtiter reader (BioTek, Synergy HT) and OD450 values were read. The data was collected to calculate results by using the GraphPad Prism 5 software. See FIG. 8 and Table 10 for the results. Among the different humanized CD70 antibodies, humanized variants Ab001, Ab004 and Ab007 had the highest maximum killing rate of about 60% on Dauai cells.

Table 10. CDC killing of different humanized CD70 antibodies on tumor cell Daudi

| Humanized antibodies | VH | VK | CDC |
|---|---|---|---|
| Ab001 | h39H5 VHv1 | h39H5 VKv1 | ++ |
| Ab002 | h39H5 VHv1 | h39H5 VKv2 | + |
| Ab003 | h39H5 VHv1 | h39H5 VKv3 | + |
| Ab004 | h39H5 VHv2 | h39H5 VKv1 | ++ |
| Ab005 | h39H5 VHv2 | h39H5 VKv2 | + |
| Ab006 | h39H5 VHv2 | h39H5 VKv3 | + |
| Ab007 | h39H5 VHv3 | h39H5 VKv1 | ++ |
| Ab008 | h39H5 VHv3 | h39H5 VKv2 | + |
| Ab009 | h39H5 VHv3 | h39H5 VKv3 | + |
| Ab010 | h32D1 VHv1 | h32D1 VKv1 | + |
| Ab011 | h32D1 VHv1 | h32D1 VKv2 | + |
| Ab012 | h32D1 VHv1 | h32D1 VKv3 | + |

(continued)

| Humanized antibodies | VH | VK | CDC |
|---|---|---|---|
| Ab013 | h32D1 VHv2 | h32D1 VKv1 | + |
| Ab014 | h32D1 VHv2 | h32D1 VKv2 | + |
| Ab015 | h32D1 VHv2 | h32D1 VKv3 | + |
| Ab016 | h32D1 VHv3 | h32D1 VKv1 | + |
| Ab017 | h32D1 VHv3 | h32D1 VKv2 | + |
| Ab018 | h32D1 VHv3 | h32D1 VKv3 | + |

**Example 7 Affinity assay of humanized CD70 antibodies**

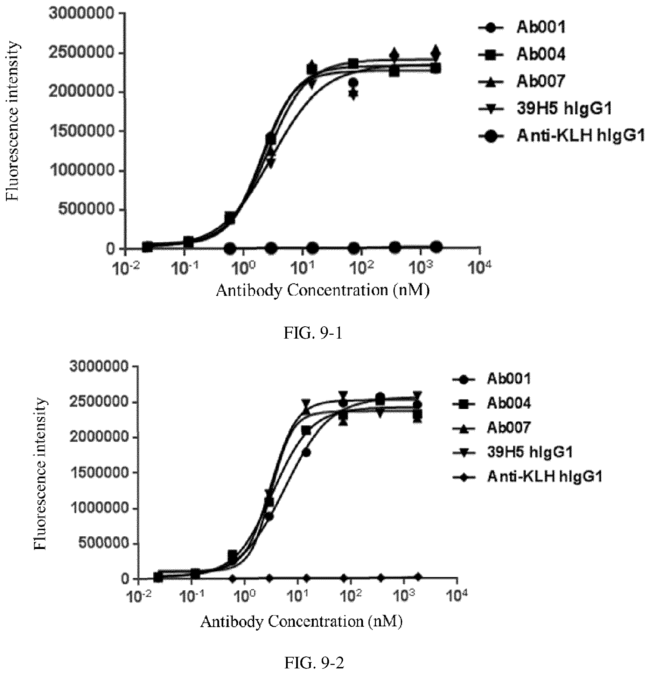

The stably-transfected cells CHO human CD70 and CHO cynomolgus monkey CD70 were taken, respectively, blocked with 3% BSA for 30 minutes, and plated to a 96-well U-plate at $5 \times 10^4$ cells /100μL. The centrifuge was performed at 1100 rpm for 3 minutes. The supernatant was discarded and the cells were gently tapped to be loosening. 50μL of gradient diluted antibodies were added (antibody concentration: from 30 μg/mL, 3-fold dilution for 8 gradients) per well, and incubated at 4°C for 1 hour. After incubation, 200μL of 0.5% BSA was added to each well for washing 3 times. 50μL/well of secondary antibody Alexa Fluro647 anti-human IgG (Jackson ImmunoResearch, Cat. No: 109-606-170) was added and incubated at 4°C for 45 minutes. At the end of the incubation, each well was washed 3 times with 180μL of 0.5% BSA and finally resuspended in 50μL of PBS for iQue (Intellicyt) assay. As shown in FIGs. 9-1, 9-2 and Table 11, FACS can detect high-affinity binding of humanized antibodies Ab001, Ab004 and Ab007 to CD70 molecules expressed on stably-transfected cell lines CHO-human CD70 and CHO-monkey CD70, respectively.

Table 11. FACS cell affinity assay

| Humanized antibodies | CHO-Human CD70 EC50(nM) | CHO-Cynomolgus Monkey CD70 EC50(nM) |
|---|---|---|
| Ab001 | 2.00 | 5.78 |
| Ab004 | 2.15 | 3.39 |
| Ab007 | 2.57 | 3.14 |

**Example 8. Blocking effect of humanized CD70 antibodies on CD70 binding to CD27**

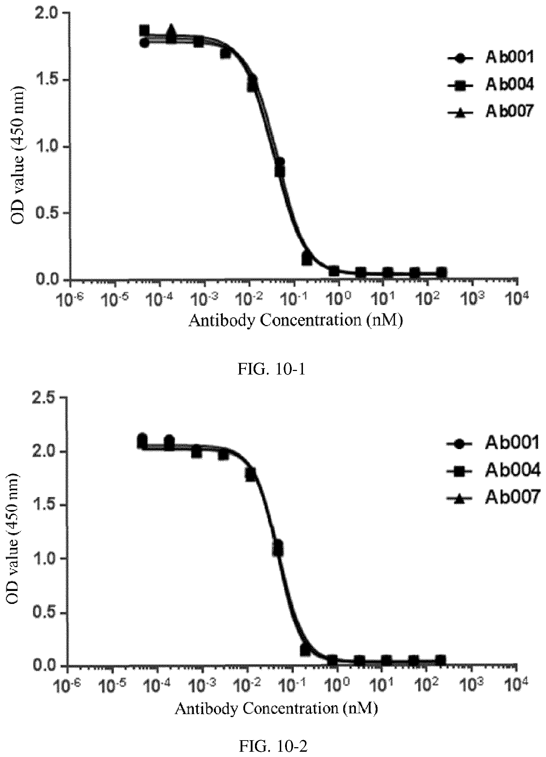

The recombinant human CD27 protein was coated at 1 μg/mL in a 96-well microtiter plate and incubated overnight at 4°C. The next day, the plate was washed 3 times with PBS, added with 200μL of 2% defatted dry milk/PBS, blocked at room temperature for 2 hours, and washed once with PBS. The purified chimeric antibody was divided into 12 concentration gradients starting at 100 nM and diluted in a 1: 3 gradient. 30μl of the antibody was added per well and incubated at room temperature for 30 minutes. 30μL of biotin-labeled recombinant CD70 protein was then added to each well and incubated at room temperature for 60 minutes. The plate was washed 3 times with PBST and PBS and dried by spinning. A secondary antibody solution Streptavidin-coupled horseradish peroxidase SA-HRP (BD bioscience, Cat. No: 6222697) was diluted with 0.5% BSA in a ratio of 1:8000. 100μL of the diluent was added into each well and incubated at room temperature for 30 minutes. The plate was washed 3 times with PBST and PBS and dried by spinning. 100μl of color development solution (TMB solution, Sigma, Cat. No: T2885) was added into each well and protected from light at 37°C for reaction. After the substrate developed moderately, 50μl of $H_2SO_4$ was added into each well to terminate the reaction. The OD450nm was determined using an enzyme-linked detector within 30 minutes, and the data was collected. The results were calculated by using Graph Pad Prism 5 software. See FIG. 10-1, 10-2, and Table 12 for the results. Humanized antibodies Ab001, Ab004 and Ab007 can effectively block the binding of human or cynomolgus monkey CD70 receptor and ligand CD27.

Table 12. Blocking effect assay on binding of human CD70 to CD27

| Humanized antibodies | Human CD70/CD27 IC50(nM) | Cynomolgus Monkey CD70/CD27 IC50(nM) |
|---|---|---|
| Ab001 | 0.04 | 0.05 |
| Ab004 | 0.04 | 0.05 |
| Ab007 | 0.04 | 0.05 |

**Example 9. CDC killing of humanized CD70 antibodies on different tumor cells**

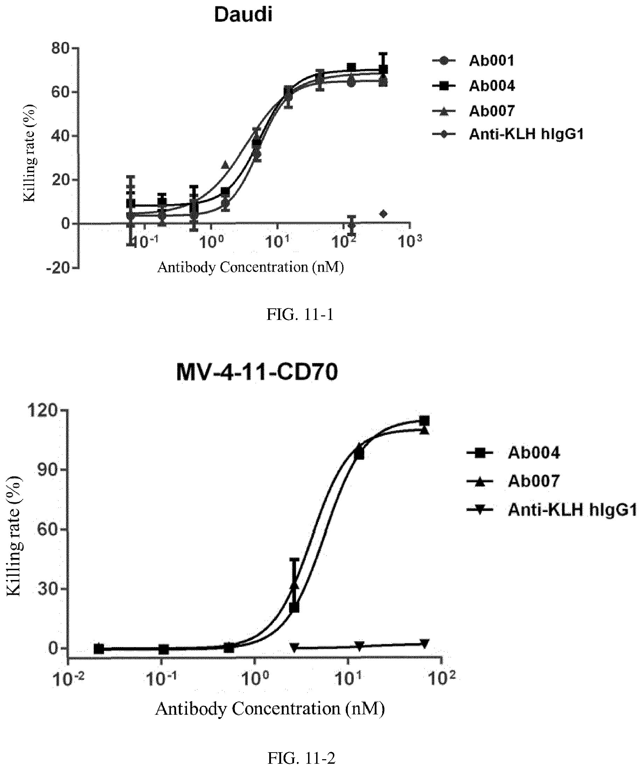

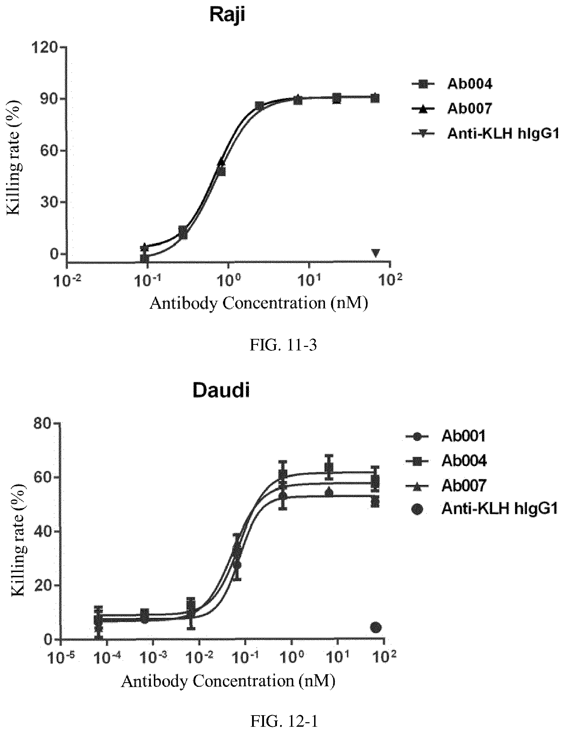

Daudi cells were resuspended and plated in a 96-well plate at 50$\mu$L/well followed by the addition of 50$\mu$L of antibodies per well. The humanized antibodies were diluted to 10$\mu$g/mL as a starting concentration and performed 3-fold gradient dilution. 50$\mu$L of complement mixture solution was added and the 96-well plate was placed in a 37°C and 5% $CO_2$ incubator for 2 hours. Then 50$\mu$L of 40% CCK-8 solution was added to each well, shaken for 10 seconds, and incubated in a 5% $CO_2$ incubator for 3 hours. The 96-well plate was placed in a microtiter reader (BioTek, Synergy HT) and OD450 values were read. The data was collected to calculate results by using the GraphPad Prism 5 software. See FIGs. 11-1, 11-2, and 11-3, and Table 13 for the results. The humanized antibodies Ab001, Ab004 and Ab007 all have a significant killing effect on tumor cells Daudi, MV-4-11-CD70 and Raji.

Table 13. CDC Killing of different humanized CD70 antibodies on different tumor cells

| Humanized antibodies | CDC Killing EC50(nM) | | |
|---|---|---|---|
| | Daudi | MV-4-11-CD70 | Raji |
| **Ab001** | 5.32 | ND | ND |
| **Ab004** | 5.60 | 5.67 | 0.71 |
| **Ab007** | 3.49 | 4.07 | 0.70 |
| ND: not detected | | | |

Example 10. ADCC killing of humanized CD70 antibodies on different tumor cells

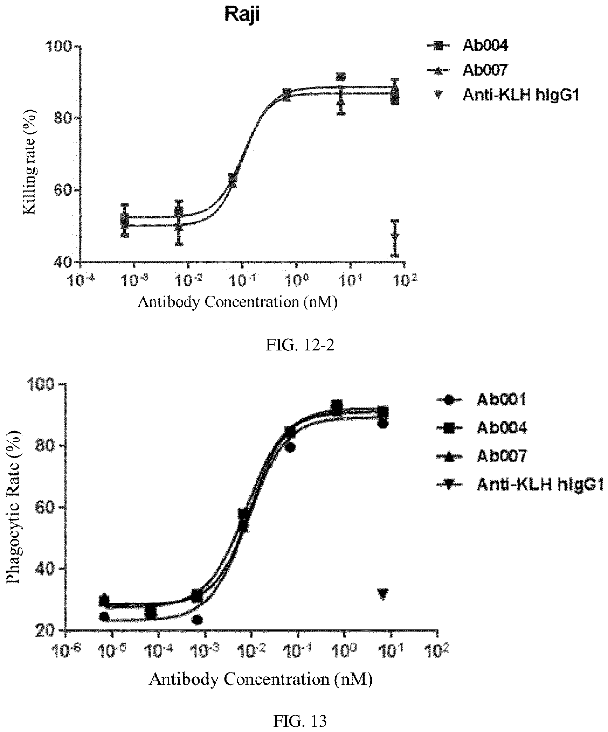

Daudi cells were inoculated into a 96-well U-plate. Various concentrations of humanized antibodies were added to each well, and cultured in a $CO_2$ incubator for half an hour. NK92-MI-CD16a cells were taken and resuspended to a cell density of 2 $\times$ 10$^6$/mL with the RPMI-1640 medium. Then, 50$\mu$L of NK92-MI-CD16a cells were added to each well of a 96-well plate and incubated in a $CO_2$ incubator for 4 hours. Thirty minutes prior to assay, 2 $\mu$L of cell lysate (100$\times$) was added to the maximal well of target cells, and the incubation was continued at 37°C. The centrifuge was performed at 300 g for 3 minutes. 50 $\mu$L of the supernatant was taken out and added to a black microtiter plate. 50$\mu$L of LDH detection substrate (placed at room temperature to dissolve in advance) was added per well and mixed evenly by gently shaking. After 10 minutes, 25$\mu$L of a termination solution was added to terminate the reaction. After shaking for 10 seconds, a fluorescence (excitation wavelength 560 nm, emission wavelength 590 nm) was selected for plate reading. The killing rate was calculated. The killing rate is calculated as follows: Killing rate %=( Test well - control well)/(Maxium release well - control well) $\times$ 100% See FIGs. 12-1, and 12-2, and Table 14 for the results. The humanized antibodies Ab001, Ab004 and Ab007 all have significant killing effect on tumor cells Daudi and Raji.

Table 14. ADCC killing of different humanized CD70 antibodies on different tumor cells

| Humanized antibodies | ADCC Killing EC50(nM) | |
|---|---|---|
| | Daudi | Raji |
| **Ab001** | 0.08 | ND |
| **Ab004** | 0.08 | 0.11 |
| **Ab007** | 0.05 | 0.10 |
| ND: not detected | | |

**Example 11. ADCP killing of humanized CD70 antibodies on different tumor cells** Daudi cells labeled with 10nM fluorescent dye CFSE were used as CD70+ target cells, and seeded into a 96-well plate. A gradient-diluted humanized antibodies were added and cultured in a $CO_2$ incubator. Finally, GM-CSF1 was added to induce mature macrophages at the ratio of macrophage: Daudi = 1: 4 at 37°C and 5% $CO_2$. The diluted solution of Anti-CD14 APC antibody was added and protected from light on ice for 20 minutes. The cells were collected and detected by flow cytometry. The single positive cell population of CD14 was circled with the macrophage well alone as a control. The single positive cell population of CD14 and double-positive cell population of CD14 and CSFE were circled in test wells, and the number of cells in two populations was counted. See FIG. 13 and Table 15 for the results. The humanized antibodies Ab001, Ab004 and Ab007 all have significant ADCP phagocytosis on tumor cell Daudi.

Table 15. ADCP killing of different humanized CD70 antibodies on tumor cell Daudi

| Humanized antibodies | ADCP phagocytosis EC50(nM) |
| --- | --- |
| Ab001 | 8.30 |
| Ab004 | 7.70 |
| Ab007 | 9.45 |

**Example 12. Inhibition of humanized CD70 antibodies on CD70-mediated stimulation of IL-8 secretion**

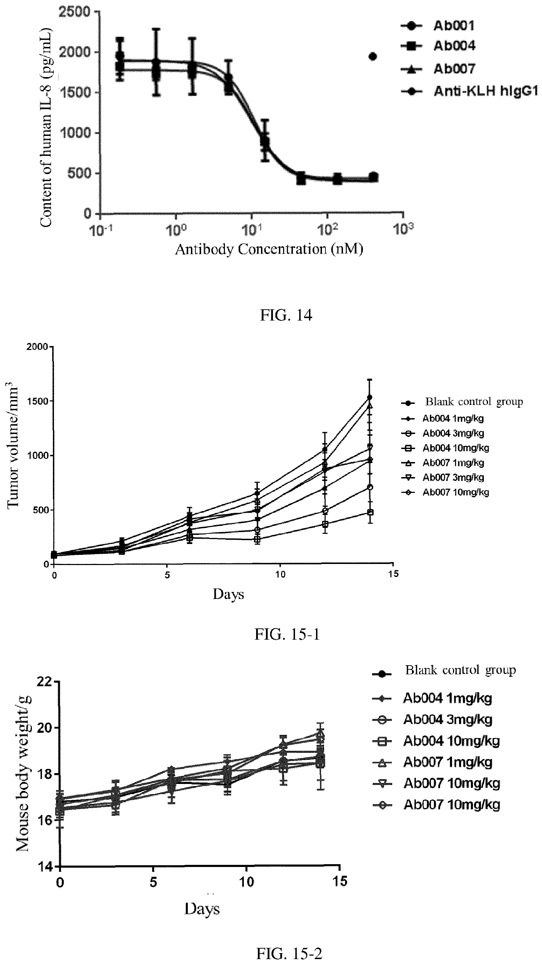

$3 \times 10^6$ HT1080-human CD27 cells were taken and subjected to centrifugation at 300 g for 10 minutes. The supernatant was discarded. 10mL of PBS was added for washing once. The cell was re-suspended by using a DMEM complete medium to a density of $2 \times 10^5$/mL, 100μL of which was added to each well, and placed in a 5% $CO_2$ incubator for overnight culture. The next day, recombinant human CD70 protein prepared in Example 1 and Anti-mouse IgG Fc (Jackson Immuno Research) were added in a 1: 1 molar ratio to a total volume of 110μL in a 96-well U-well plate, mixed well and pre-incubated at 37°C for 30 minutes to form a CD70-mer. A new 96-well plate was taken and added 110μL of humanized CD70 antibodies diluted in different gradients and 110μL of the sample pre-incubated in the previous step into each well. After mixing well, the plate was incubated at 37°C for 30 minutes. The cell culture plate was taken out and centrifuged. The supernatant was discarded. 200μL of antibodies and recombinant protein were added into each well and incubated in a 5% $CO_2$ incubator for 6 hours. Then, a centrifuge was performed at 300g for 10 minutes. The supernatant was collected. The supernatant was assayed for IL-8 content by using a human IL-8 cytokine detection kit (BD, Cat. No: 555244). See FIG. 14 and Table 16 for the results. Humanized antibodies Ab001, Ab004 and Ab007 all can inhibit CD70-mediated stimulation of IL-8 secretion.

Table 16. Inhibition of CD70-mediated stimulation of IL-8 secretion by different humanized CD70 antibodies.

| Humanized antibodies | Inhibition of IL-8 secretion IC50(nM) |
| --- | --- |
| Ab001 | 10.71 |
| Ab004 | 10.99 |
| Ab007 | 9.92 |

**Example 13. Anti-tumor effect of humanized CD70 antibodies in Raji cell line subcutaneous xenograft NOD/SCID mouse model**

Raji cells were cultured in a RPMI1640 culture medium comprising 10% fetal bovine serum. Raji cells in logarithmic growth phase were harvested, resuspended in PBS to an appropriate concentration, and mixed 1: 1 with matrigel for subcutaneous tumor inoculation in a NOD/SCID mouse. Female mice were subcutaneously inoculated with $1 \times 10^7$ Raji cells on the right side. When the average tumor volume was 100mm$^3$, the mice were randomly divided into groups according to the tumor size. There were 5 mice per group. The mice in the treatment group were dosed with 1mg/kg, 3 mg/kg and 10mg/kg by tail vein injection twice a week for two weeks. The tumor volume was monitored. The calculation method for tumor volume is:

$$\text{Tumor volume (mm}^3) = 1/2 \times (a \times b^2) \text{ (where a represents long diameter and b represents short diameter).}$$

See FIG. 15-1 for the results. Human Burkitt's lymphoma Raji mice, resulting in partial tumor regression; the humanized CD70 antibody Ab004 showed 69.4% tumor inhibition rate at the dose of 10mg/kg.

Meanwhile, as shown in FIG. 15-2, humanized CD70 antibody did not cause weight loss in mice in different dose groups, without obvious toxic effects.

**Example 14. CDC activity enhanced CD70 humanized antibodies**

Based on a structural model of IgG1 and complement C1 (Ugurlar et al., 2017), mutations were introduced at the first binding site of IgG1 and complement C1 binding region to reduce polarity ($R_{292}$ and/or $Y_{300}$), to reduce rejection of R129 and R114 of C1q, or to simultaneously introduce mutation $P_{396}L$ at CH3 to optimize the angle between CH2 and CH3. The CD70 antibody comprising a Fc mutation was expressed in a CHO cell and the effect of each mutation on the biological activity of the CD70 antibody was examined after purification. Control antibody 41D12 was synthesized according to CN201280013552.5 and expressed and purified in a CHO cell.

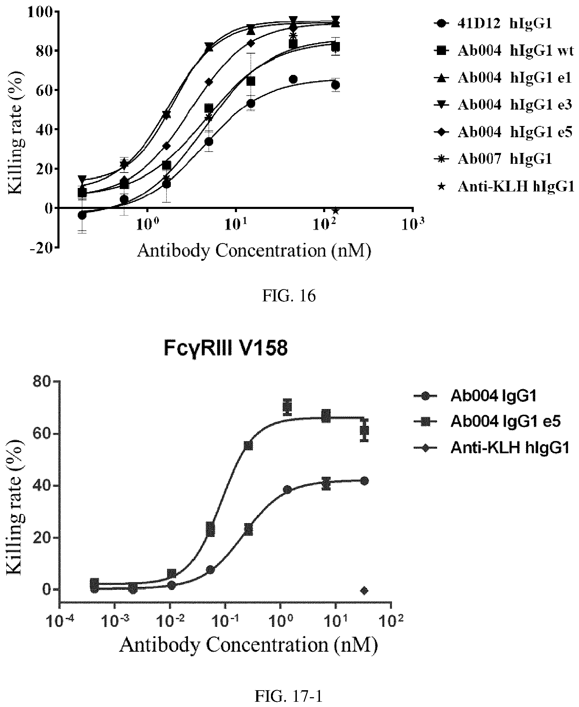

The results showed that the CDC activity of CD70 humanized antibodies Ab004 and Ab007 were significantly superior to the control antibody 41D12, in which the CDC killing effect of CD70 humanized antibody containing Fc mutation ( mutation sites are shown in Table 17), Ab004 IgG1 e1, Ab004 IgG1 e3 and Ab004 IgG1 e5 was significantly enhanced, with the maximum killing rate of more than 90% on Daudi cells, and the median killing concentration EC50 was about 2-3 times lower than that of the wild type (FIG. 16).

Two CD16a receptor alleles, FcγRIII $_{V158}$ and FcγRIII $_{F158}$, were widely present in human population. The allele FcγRIII $_{V158}$ encodes a receptor with a high affinity for antibody IgG1. NK cells expressing FcγRIII $_{V158}$ mediate more effective antibody-dependent cell-mediated ADCC, which is thought to be responsible for the insignificant efficacy of antibody drugs on low-affinity FcγRIII $_{F158}$ populations. The Biacore results showed that mutant Ab004 IgG1 e5 had a significant affinity enhancement to the low-affinity FcγRIII $_{F158}$ and comparable affinity to wild-type IgG1 and the high-affinity FcγRIII $_{V158}$ receptor (Table 17).

Table 17. Evaluation of biological activity of different Fc mutants

|  | Mutation site | CDC EC50(nM) | FcγRIII V158 KD (nM) | FcγRIII F158 KD (nM) |
|---|---|---|---|---|
| Ab004 IgG1 wt | None | 4.58 | 565 | 1305 |
| Ab004 IgG1 e1 | $Y_{300}L/P_{396}L$ | 1.74 | 118 | 602 |
| Ab004 IgG1 e3 | $R_{292}P/Y_{300}L/P_{396}L$ | 2.00 | 75 | 172 |
| Ab004 IgG1 e5 | $R_{292}P/Y_{300}L$ | 3.06 | 217 | 342 |
| Ab007 IgG 1 | None | 4.48 | ND | ND |
| 41D12 IgG1 | None | 4.39 | ND | ND |
| KLH IgG 1 | None | - | ND | ND |
| ND: not detected | | | | |

PBMCs were isolated from healthy volunteers with different FcγRIIIa allele receptors and resuspended in a RPMI-1640 medium. Daudi cells were inoculated into a 96-well U-plate. Various concentrations of humanized antibodies were added to each well, and cultured in a $CO_2$ incubator for half an hour. $5 \times 10^5$ PBMCs per well were then added to the 96-well plate and incubated in the $CO_2$ incubator for 4 hours. Thirty minutes prior to assay, 2μL of cell lysate (100×) was added to the maximal well of target cells, and the incubation was continued at 37°C. The centrifuge was performed at 300g for 3 minutes. 50μL of the supernatant was taken out and added to a black microtiter plate. 50μL of LDH detection substrate (placed at room temperature to dissolve in advance) was added per well and mixed evenly by gently shaking. After 10 minutes, 25μL of a termination solution was added to terminate the reaction. After shaking for 10 seconds, a fluorescence (excitation wavelength 560nm, emission wavelength 590nm) was selected for plate reading. The killing rate was calculated.

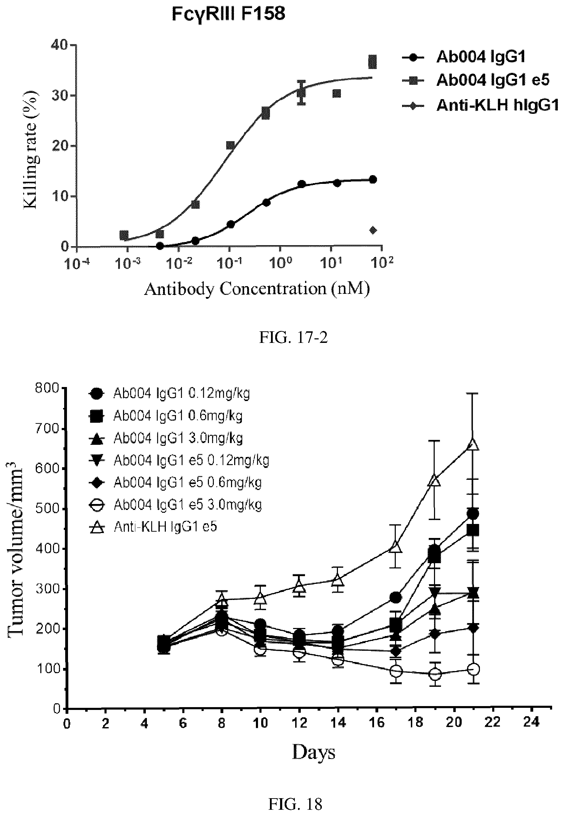

See FIGs. 17-1, 17-2, and Table 18 for the results. Ab004 IgG1 e5 signifcantly enhanced killing of tumor cell Daudi relative to wild-type Ab004 IgG1 by using FcγRIII $_{V158/V158}$ volunteer PBMC or FcγRIII $_{F158/F158}$ volunteer PBMC as effector cells. Among them, the ADCC killing effect mediated by Ab004 IgG1 e5 in volunteer PBMC of low-affinity receptor FcγRIII$_{F158/F158}$ was equivalent to the ADCC effect mediated by wild-type Ab004 IgG1 in volunteer PBMC of high-affinity receptor FcγRIII$_{V158/V158}$, suggesting that Fc antibody using mutant IgG1 e5 has equivalent ADCC efficacy in a wide range of the human population.

Table 18. ADCC killing of different humanized CD70 antibodies on different tumor cells

| Humanized antibodies | ADCC Killing | | | |
|---|---|---|---|---|
| | FcγRIII$_{V158}$ | | FcγRIII$_{F158}$ | |
| | EC$_{50}$ | Maximum killing rate | EC$_{50}$ | Maximum killing rate |
| Ab004 IgG1 e5 | 0.09nM | 66% | 0.08nM | 34% |
| Ab004 IgG1 | 0.22nM | 40% | 0.23nM | 13% |

**Example 15. Anti-tumor effect of CD70 antibodies with enhanced CDC activity in Daudi cell line subcutaneous xenograft NOD/SCID mouse model**

Daudi cells in logarithmic growth phase were taken and adjusted to a cell density of $1.2 \times 10^8$/ml. An equal volume of Matrigel Matrix was added and mixed well with cell suspension. 100μl of the mixture was inoculated subcutaneously in the right limb of the BALB/c-Nude mouse. When the average tumor volume was 100-200 mm$^3$, 5 mice in each group were randomly divided into groups. The first administration was started on the day of grouping, once every three days for a total of three times, and 100μl tail vein injection was administered for each mouse. Setting the day of the subcutaneous tumor as D0, the tumor growth curve was drawn (tumor volume was not more than 2000 mm$^3$, and mice were sacrificed when reaching 2000 mm$^3$).

See FIG. 18 for the results. Both the CDC activity-enhanced CD70 antibody and the parental antibody can dose-dependently inhibit the growth of human lymphoma Daudi subcutaneous xenografts. The tumor inhibition rates of CDC-enhanced CD70 antibodies were 114%, 92% and 67%, respectively. The tumor inhibition rates of CD70 parental antibodies were 81%, 36% and 24%, respectively. The tumor-bearing mice tolerated the doses above well, without adverse reactions such as weight loss.

**Claims**

1. An antibody or antigen-binding portion thereof that binds to CD70, comprising heavy chain CDRs selected from the group consisting of amino acid sequences SEQ ID NOs: 5, 6, 7, 15, 16, 22, 29, 30, 31, 37, 38, 44, 45, 46, 54, 55, 56, 64, 65, 77, 83, 84, 85, 91, 97, 98, 106, 107, 114, 115, 116, 124, 131, 132, 135, 142, 160 or any variant thereof, and/or light chain CDRs selected from the group consisting of amino acid sequences SEQ ID NOs: 10, 11, 12, 19, 25, 26, 34, 41, 49, 50, 51, 59, 60, 61, 68, 69, 74, 80, 88, 101, 102, 103, 110, 111, 119, 120, 121, 127, 128 or any variant thereof.

2. The antibody or antigen-binding portion thereof according to claim 1, comprising a heavy chain CDR1 selected from the group consisting of amino acid sequences SEQ ID NOs: 5, 15, 29, 44, 54, 83, 96, 114, 131 or any variant thereof, a heavy chain CDR2 selected from the group consisting of amino acid sequences SEQ ID NOs: 6, 30, 37, 45, 55, 64, 77, 84, 91, 97, 106, 115, 124, 132, 135, 142 or any variant thereof, a heavy chain CDR3 selected from the group consisting of amino acid sequences SEQ ID NOs: 7, 16, 22, 31, 38, 46, 56, 65, 85, 98, 107, 116 or any variant thereof; and/or a light chain CDR1 selected from the group consisting of amino acid sequences SEQ ID NOs: 10, 16, 25, 34, 51, 59, 59, 38, 74, 88, 101, 110, 119, 127, 149 or any variant thereof, a light chain CDR2 selected from the group consisting of amino acid sequences SEQ ID NOs: 11, 50, 60, 80, 102, 111, 120, 128, 160 or any variant thereof, a light chain CDR3 selected from the group consisting of amino acid sequences SEQ ID NOs: 12, 26, 51, 61, 69, 103, 121 or any variant thereof.

3. The antibody or antigen-binding portion thereof according to claim 1 or 2, comprising a CDR combination of a heavy chain and a light chain selected from the group consisting of:

(1) heavy chain CDR1, CDR2, CDR3 sequences comprising SEQ ID NOs: 5, 6, 7, and/or light chain CDR1, CDR2, CDR3 sequences comprising SEQ ID NOs: 10, 11, 12, respectively;
(2) heavy chain CDR1, CDR2, CDR3 sequences comprising SEQ ID NOs: 15, 6, 16, and/or light chain CDR1, CDR2, CDR3 sequences comprising SEQ ID NOs: 19, 11, 12, respectively;
(3) heavy chain CDR1, CDR2, CDR3 sequences comprising SEQ ID NOs: 5, 6, 22, and/or light chain CDR1, CDR2, CDR3 sequences comprising SEQ ID NOs: 25, 11, 26, respectively;
(4) heavy chain CDR1, CDR2, CDR3 sequences comprising SEQ ID NOs: 29, 30, 31, and/or light chain CDR1, CDR2, CDR3 sequences comprising SEQ ID NOs: 34, 11, 26, respectively;
(5) heavy chain CDR1, CDR2, CDR3 sequences comprising SEQ ID NOs: 5, 37, 38, and/or light chain CDR1, CDR2, CDR3 sequences comprising SEQ ID NOs: 41, 11, 12, respectively;
(6) heavy chain CDR1, CDR2, CDR3 sequences comprising SEQ ID NOs: 44, 45, 46, and/or light chain CDR1, CDR2, CDR3 sequences comprising SEQ ID NOs: 49, 50, 51, respectively;
(7) heavy chain CDR1, CDR2, CDR3 sequences comprising SEQ ID NOs: 54, 55, 56, and/or light chain CDR1, CDR2, CDR3 sequences comprising SEQ ID NOs: 59, 60, 61, respectively;
(8) heavy chain CDR1, CDR2, CDR3 sequences comprising SEQ ID NOs: 5, 64, 65, and/or light chain CDR1, CDR2, CDR3 sequences comprising SEQ ID NOs: 68, 11, 69, respectively;
(9) heavy chain CDR1, CDR2, CDR3 sequences comprising SEQ ID NOs: 29, 30, 31, and/or light chain CDR1, CDR2, CDR3 sequences comprising SEQ ID NOs: 74, 11, 26, respectively;

(10) heavy chain CDR1, CDR2, CDR3 sequences comprising SEQ ID NOs: 44, 77, 46, and/or light chain CDR1, CDR2, CDR3 sequences comprising SEQ ID NOs: 49, 80, 51, respectively;

(11) heavy chain CDR1, CDR2, CDR3 sequences comprising SEQ ID NOs: 83, 84, 85, and/or light chain CDR1, CDR2, CDR3 sequences comprising SEQ ID NOs: 88, 11, 12, respectively;

(12) heavy chain CDR1, CDR2, CDR3 sequences comprising SEQ ID NOs: 5, 91, 85, and/or light chain CDR1, CDR2, CDR3 sequences comprising SEQ ID NOs: 19, 11, 12, respectively;

(13) heavy chain CDR1, CDR2, CDR3 sequences comprising SEQ ID NOs: 96, 97, 98, and/or light chain CDR1, CDR2, CDR3 sequences comprising SEQ ID NOs: 101, 102, 103, respectively;

(14) heavy chain CDR1, CDR2, CDR3 sequences comprising SEQ ID NOs: 15, 106, 107, and/or light chain CDR1, CDR2, CDR3 sequences comprising SEQ ID NOs: 110, 111, 103, respectively;

(15) heavy chain CDR1, CDR2, CDR3 sequences comprising SEQ ID NOs: 114, 115, 116, and/or light chain CDR1, CDR2, CDR3 sequences comprising SEQ ID NOs: 119, 120, 121, respectively;

(16) heavy chain CDR1, CDR2, CDR3 sequences comprising SEQ ID NOs: 15, 124, 16, and/or light chain CDR1, CDR2, CDR3 sequences comprising SEQ ID NOs: 127, 128, 103, respectively;

(17) heavy chain CDR1, CDR2, CDR3 sequences comprising SEQ ID NOs: 131, 132, 116, and/or light chain CDR1, CDR2, CDR3 sequences comprising SEQ ID NOs: 119, 120, 121, respectively;

(18) heavy chain CDR1, CDR2, CDR3 sequences comprising SEQ ID NOs: 44, 135, 46, and/or light chain CDR1, CDR2, CDR3 sequences comprising SEQ ID NOs: 149, 80, 51, respectively;

(19) heavy chain CDR1, CDR2, CDR3 sequences comprising SEQ ID NOs: 29, 142, 31, and/or light chain CDR1, CDR2, CDR3 sequences comprising SEQ ID NOs: 25, 11, 26, respectively; and

(20) heavy chain CDR1, CDR2, CDR3 sequences comprising SEQ ID NOs: 29, 142, 31, and/or light chain CDR1, CDR2, CDR3 sequences comprising SEQ ID NOs: 25, 160, 26, respectively.

4. The antibody or antigen-binding portion thereof according to claim 1 or 2, comprising a heavy chain variable region selected from the group consisting of amino acid sequences SEQ ID NOs: 3, 13, 20, 27, 35, 42, 52, 62, 70, 75, 81, 89, 94, 104, 112, 122, 129, 133, 136, 138, 140, 143, 145 or any variant thereof, and/or a light chain variable region selected from the group consisting of amino acid sequences SEQ ID NOs: 8, 17, 23, 32, 39, 47, 57, 66, 72, 78, 86, 92, 99, 108, 117, 125, 147, 150, 152, 154, 156, 158 or any variant thereof;

preferably, comprising the heavy chain variable region of the amino acid sequence SEQ ID NO: 3 or any variant thereof, and the light chain variable region of the amino acid sequence SEQ ID NO: 8 or any variant thereof;

preferably, comprising the heavy chain variable region of the amino acid sequence SEQ ID NO: 13 or any variant thereof, and the light chain variable region of the amino acid sequence SEQ ID NO: 17 or any variant thereof;

preferably, comprising the heavy chain variable region of the amino acid sequence SEQ ID NO: 20 or any variant thereof, and the light chain variable region of the amino acid sequence SEQ ID NO: 23 or any variant thereof;

preferably, comprising the heavy chain variable region of the amino acid sequence SEQ ID NO: 27 or any variant thereof, and the light chain variable region of the amino acid sequence SEQ ID NO: 32 or any variant thereof;

preferably, comprising the heavy chain variable region of the amino acid sequence SEQ ID NO: 35 or any variant thereof, and the light chain variable region of the amino acid sequence SEQ ID NO: 39 or any variant thereof;

preferably, comprising the heavy chain variable region of the amino acid sequence SEQ ID NO: 42 or any variant thereof, and the light chain variable region of the amino acid sequence SEQ ID NO: 47 or any variant thereof;

preferably, comprising the heavy chain variable region of the amino acid sequence SEQ ID NO: 52 or any variant thereof, and the light chain variable region of the amino acid sequence SEQ ID NO: 57 or any variant thereof;

preferably, comprising the heavy chain variable region of the amino acid sequence SEQ ID NO: 62 or any variant thereof, and the light chain variable region of the amino acid sequence SEQ ID NO: 66 or any variant thereof;

preferably, comprising the heavy chain variable region of the amino acid sequence SEQ ID NO: 70 or any variant thereof, and the light chain variable region of the amino acid sequence SEQ ID NO: 72 or any variant thereof;

preferably, comprising the heavy chain variable region of the amino acid sequence SEQ ID NO: 75 or any variant thereof, and the light chain variable region of the amino acid sequence SEQ ID NO: 78 or any variant thereof;

preferably, comprising the heavy chain variable region of the amino acid sequence SEQ ID NO: 81 or any variant thereof, and the light chain variable region of the amino acid sequence SEQ ID NO: 86 or any variant thereof;

preferably, comprising the heavy chain variable region of the amino acid sequence SEQ ID NO: 89 or any variant thereof, and the light chain variable region of the amino acid sequence SEQ ID NO: 92 or any variant thereof;

preferably, comprising the heavy chain variable region of the amino acid sequence SEQ ID NO: 94 or any variant thereof, and the light chain variable region of the amino acid sequence SEQ ID NO: 99 or any variant thereof;

preferably, comprising the heavy chain variable region of the amino acid sequence SEQ ID NO: 104 or any variant thereof, and the light chain variable region of the amino acid sequence SEQ ID NO: 108 or any variant thereof;

preferably, comprising the heavy chain variable region of the amino acid sequence SEQ ID NO: 112 or any variant thereof, and the light chain variable region of the amino acid sequence SEQ ID NO: 117 or any variant thereof;

preferably, comprising the heavy chain variable region of the amino acid sequence SEQ ID NO: 122 or any variant thereof, and the light chain variable region of the amino acid sequence SEQ ID NO: 125 or any variant thereof;

preferably, comprising the heavy chain variable region of the amino acid sequence SEQ ID NO: 129 or any variant thereof, and the light chain variable region of the amino acid sequence SEQ ID NO: 117 or any variant thereof;

preferably, comprising the heavy chain variable region of the amino acid sequence SEQ ID NO: 133 or any variant thereof, and the light chain variable region of the amino acid sequence SEQ ID NO: 147 or any variant thereof;

preferably, comprising the heavy chain variable region of the amino acid sequence SEQ ID NO: 133 or any variant thereof, and the light chain variable region of the amino acid sequence SEQ ID NO: 150 or any variant thereof;

preferably, comprising the heavy chain variable region of the amino acid sequence SEQ ID NO: 133 or any variant thereof, and the light chain variable region of the amino acid sequence SEQ ID NO: 152 or any variant thereof;

preferably, comprising the heavy chain variable region of the amino acid sequence SEQ ID NO: 136 or any variant thereof, and the light chain variable region of the amino acid sequence SEQ ID NO: 147 or any variant thereof;

preferably, comprising the heavy chain variable region of the amino acid sequence SEQ ID NO: 136 or any variant thereof, and the light chain variable region of the amino acid sequence SEQ ID NO: 150 or any variant thereof;

preferably, comprising the heavy chain variable region of the amino acid sequence SEQ ID NO: 136 or any variant thereof, and the light chain variable region of the amino acid sequence SEQ ID NO: 152 or any variant thereof;

preferably, comprising the heavy chain variable region of the amino acid sequence SEQ ID NO: 138 or any variant thereof, and the light chain variable region of the amino acid sequence SEQ ID NO: 147 or any variant thereof;

preferably, comprising the heavy chain variable region of the amino acid sequence SEQ ID NO: 138 or any variant thereof, and the light chain variable region of the amino acid sequence SEQ ID NO: 150 or any variant thereof;

preferably, comprising the heavy chain variable region of the amino acid sequence SEQ ID NO: 138 or any variant thereof, and the light chain variable region of the amino acid sequence SEQ ID NO: 152 or any variant thereof;

preferably, comprising the heavy chain variable region of the amino acid sequence SEQ ID NO: 140 or any variant thereof, and the light chain variable region of the amino acid sequence SEQ ID NO: 154 or any variant thereof;

preferably, comprising the heavy chain variable region of the amino acid sequence SEQ ID NO: 140 or any variant thereof, and the light chain variable region of the amino acid sequence SEQ ID NO: 156 or any variant thereof;

preferably, comprising the heavy chain variable region of the amino acid sequence SEQ ID NO: 140 or any variant thereof, and the light chain variable region of the amino acid sequence SEQ ID NO: 158 or any variant thereof;

preferably, comprising the heavy chain variable region of the amino acid sequence SEQ ID NO: 143 or any variant thereof, and the light chain variable region of the amino acid sequence SEQ ID NO: 154 or any variant thereof;

preferably, comprising the heavy chain variable region of the amino acid sequence SEQ ID NO: 143 or any variant thereof, and the light chain variable region of the amino acid sequence SEQ ID NO: 156 or any variant thereof;

preferably, comprising the heavy chain variable region of the amino acid sequence SEQ ID NO: 143 or any variant thereof, and the light chain variable region of the amino acid sequence SEQ ID NO: 158 or any variant thereof;

preferably, comprising the heavy chain variable region of the amino acid sequence SEQ ID NO: 145 or any variant thereof, and the light chain variable region of the amino acid sequence SEQ ID NO: 154 or any variant thereof;

preferably, comprising the heavy chain variable region of the amino acid sequence SEQ ID NO: 145 or any variant thereof, and the light chain variable region of the amino acid sequence SEQ ID NO: 156 or any variant thereof; and

preferably, comprising the heavy chain variable region of the amino acid sequence SEQ ID NO: 145 or any variant thereof, and the light chain variable region of the amino acid sequence SEQ ID NO: 158 or any variant thereof.

5. A bispecific or multispecific molecule comprising the antibody or antigen-binding portion thereof that binds to CD70 according to any one of claims 1-4.

6. The antibody or antigen-binding portion thereof according to any one of claims 1-4 or the bispecific or multispecific molecule according to claim 5, wherein the antibody or the antigen-binding portion thereof is humanized.

7. A nucleic acid molecule encoding the antibody or antigen-binding portion thereof according to any one of claims 1-4, the bispecific or multispecific molecule of claim 5, or the antibody or antigen-binding portion thereof or the bispecific or multispecific molecule of claim 6;

preferably, the nucleic acid molecule comprises an antibody heavy chain nucleic acid sequence selected from the group consisting of SEQ ID NOs: 4, 14, 21, 28, 36, 43, 53, 63, 71, 76, 82, 90, 95, 105, 113, 123, 130, 134, 137, 139, 141, 144, 146 or any variant thereof, and/or an antibody light chain nucleic acid sequence selected from the group consisting of SEQ ID NOs: 9, 18, 24, 33, 40, 48, 58, 67, 73, 79, 87, 93, 100, 109, 118, 126, 148, 151, 153, 155, 157, 159 or any variant thereof;

preferably, the nucleic acid molecule comprises the antibody heavy chain variable region nucleotide sequence of the nucleotide sequence SEQ ID NO: 4 or any variant thereof, and/or the light chain variable region nucleotide sequence of the nucleotide sequence SEQ ID NO: 9 or any variant thereof;

preferably, the nucleic acid molecule comprises the antibody heavy chain variable region nucleotide sequence of the nucleotide sequence SEQ ID NO: 14 or any variant thereof, and/or the light chain variable region nucleotide sequence of the nucleotide sequence SEQ ID NO: 18 or any variant thereof;

preferably, the nucleic acid molecule comprises the antibody heavy chain variable region nucleotide sequence of the nucleotide sequence SEQ ID NO: 21 or any variant thereof, and/or the light chain variable region nucleotide sequence of the nucleotide sequence SEQ ID NO: 24 or any variant thereof;

preferably, the nucleic acid molecule comprises the antibody heavy chain variable region nucleotide sequence of the nucleotide sequence SEQ ID NO: 28 or any variant thereof, and/or the light chain variable region nucleotide sequence of the nucleotide sequence SEQ ID NO: 33 or any variant thereof;

preferably, the nucleic acid molecule comprises the antibody heavy chain variable region nucleotide sequence of the nucleotide sequence SEQ ID NO: 36 or any variant thereof, and/or the light chain variable region nucleotide sequence of the nucleotide sequence SEQ ID NO: 40 or any variant thereof;

preferably, the nucleic acid molecule comprises the antibody heavy chain variable region nucleotide sequence of the nucleotide sequence SEQ ID NO: 43 or any variant thereof, and/or the light chain variable region nucleotide sequence of the nucleotide sequence SEQ ID NO: 48 or any variant thereof;

preferably, the nucleic acid molecule comprises the antibody heavy chain variable region nucleotide sequence of the nucleotide sequence SEQ ID NO: 53 or any variant thereof, and/or the light chain variable region nucleotide sequence of the nucleotide sequence SEQ ID NO: 58 or any variant thereof;

preferably, the nucleic acid molecule comprises the antibody heavy chain variable region nucleotide sequence of the nucleotide sequence SEQ ID NO: 63 or any variant thereof, and/or the light chain variable region nucleotide sequence of the nucleotide sequence SEQ ID NO: 67 or any variant thereof;

preferably, the nucleic acid molecule comprises the antibody heavy chain variable region nucleotide sequence of the nucleotide sequence SEQ ID NO: 71 or any variant thereof, and/or the light chain variable region nucleotide sequence of the nucleotide sequence SEQ ID NO: 73 or any variant thereof;

preferably, the nucleic acid molecule comprises the antibody heavy chain variable region nucleotide sequence of the nucleotide sequence SEQ ID NO: 76 or any variant thereof, and/or the light chain variable region nucleotide sequence of the nucleotide sequence SEQ ID NO: 79 or any variant thereof;

preferably, the nucleic acid molecule comprises the antibody heavy chain variable region nucleotide sequence of the nucleotide sequence SEQ ID NO: 82 or any variant thereof, and/or the light chain variable region nucleotide sequence of the nucleotide sequence SEQ ID NO: 87 or any variant thereof;

preferably, the nucleic acid molecule comprises the antibody heavy chain variable region nucleotide sequence of the nucleotide sequence SEQ ID NO: 90 or any variant thereof, and/or the light chain variable region nucleotide sequence of the nucleotide sequence SEQ ID NO: 93 or any variant thereof;

preferably, the nucleic acid molecule comprises the antibody heavy chain variable region nucleotide sequence of the nucleotide sequence SEQ ID NO: 95 or any variant thereof, and/or the light chain variable region nucleotide sequence of the nucleotide sequence SEQ ID NO: 100 or any variant thereof;

preferably, the nucleic acid molecule comprises the antibody heavy chain variable region nucleotide sequence of the nucleotide sequence SEQ ID NO: 105 or any variant thereof, and/or the light chain variable region nucleotide sequence of the nucleotide sequence SEQ ID NO: 109 or any variant thereof;

preferably, the nucleic acid molecule comprises the antibody heavy chain variable region nucleotide sequence of the nucleotide sequence SEQ ID NO: 113 or any variant thereof, and/or the light chain variable region nucleotide sequence of the nucleotide sequence SEQ ID NO: 118 or any variant thereof;

preferably, the nucleic acid molecule comprises the antibody heavy chain variable region nucleotide sequence of the nucleotide sequence SEQ ID NO: 123 or any variant thereof, and/or the light chain variable region nucleotide sequence of the nucleotide sequence SEQ ID NO: 126 or any variant thereof;

preferably, the nucleic acid molecule comprises the antibody heavy chain variable region nucleotide sequence of the nucleotide sequence SEQ ID NO: 130 or any variant thereof, and/or the light chain variable region nucleotide sequence of the nucleotide sequence SEQ ID NO: 118 or any variant thereof;

preferably, the nucleic acid molecule comprises the antibody heavy chain variable region nucleotide sequence of the nucleotide sequence SEQ ID NO: 134 or any variant thereof, and/or the light chain variable region nucleotide sequence of the nucleotide sequence SEQ ID NO: 148 or any variant thereof;

preferably, the nucleic acid molecule comprises the antibody heavy chain variable region nucleotide sequence of the nucleotide sequence SEQ ID NO: 134 or any variant thereof, and/or the light chain variable region nucleotide sequence of the nucleotide sequence SEQ ID NO: 151 or any variant thereof;

preferably, the nucleic acid molecule comprises the antibody heavy chain variable region nucleotide sequence of the nucleotide sequence SEQ ID NO: 134 or any variant thereof, and/or the light chain variable region nucleotide sequence of the nucleotide sequence SEQ ID NO: 153 or any variant thereof;

preferably, the nucleic acid molecule comprises the antibody heavy chain variable region nucleotide sequence of the nucleotide sequence SEQ ID NO: 137 or any variant thereof, and/or the light chain variable region nucleotide sequence of the nucleotide sequence SEQ ID NO: 148 or any variant thereof;

preferably, the nucleic acid molecule comprises the antibody heavy chain variable region nucleotide sequence of the nucleotide sequence SEQ ID NO: 137 or any variant thereof, and/or the light chain variable region nucleotide sequence of the nucleotide sequence SEQ ID NO: 151 or any variant thereof;

preferably, the nucleic acid molecule comprises the antibody heavy chain variable region nucleotide sequence of the nucleotide sequence SEQ ID NO: 137 or any variant thereof, and/or the light chain variable region nucleotide sequence of the nucleotide sequence SEQ ID NO: 153 or any variant thereof;

preferably, the nucleic acid molecule comprises the antibody heavy chain variable region nucleotide sequence of the nucleotide sequence SEQ ID NO: 139 or any variant thereof, and/or the light chain variable region nucleotide sequence of the nucleotide sequence SEQ ID NO: 148 or any variant thereof;

preferably, the nucleic acid molecule comprises the antibody heavy chain variable region nucleotide sequence of the nucleotide sequence SEQ ID NO: 139 or any variant thereof, and/or the light chain variable region nucleotide sequence of the nucleotide sequence SEQ ID NO: 151 or any variant thereof;

preferably, the nucleic acid molecule comprises the antibody heavy chain variable region nucleotide sequence of the nucleotide sequence SEQ ID NO: 139 or any variant thereof, and/or the light chain variable region nucleotide sequence of the nucleotide sequence SEQ ID NO: 153 or any variant thereof;

preferably, the nucleic acid molecule comprises the antibody heavy chain variable region nucleotide sequence of the nucleotide sequence SEQ ID NO: 141 or any variant thereof, and/or the light chain variable region nucleotide sequence of the nucleotide sequence SEQ ID NO: 155 or any variant thereof;

preferably, the nucleic acid molecule comprises the antibody heavy chain variable region nucleotide sequence of the nucleotide sequence SEQ ID NO: 141 or any variant thereof, and/or the light chain variable region nucleotide sequence of the nucleotide sequence SEQ ID NO: 157 or any variant thereof;

preferably, the nucleic acid molecule comprises the antibody heavy chain variable region nucleotide sequence of the nucleotide sequence SEQ ID NO: 141 or any variant thereof, and/or the light chain variable region nucleotide sequence of the nucleotide sequence SEQ ID NO: 159 or any variant thereof;

preferably, the nucleic acid molecule comprises the antibody heavy chain variable region nucleotide sequence of the nucleotide sequence SEQ ID NO: 144 or any variant thereof, and/or the light chain variable region nucleotide sequence of the nucleotide sequence SEQ ID NO: 155 or any variant thereof;

preferably, the nucleic acid molecule comprises the antibody heavy chain variable region nucleotide sequence of the nucleotide sequence SEQ ID NO: 144 or any variant thereof, and/or the light chain variable region nucleotide sequence of the nucleotide sequence SEQ ID NO: 157 or any variant thereof;

preferably, the nucleic acid molecule comprises the antibody heavy chain variable region nucleotide sequence of the nucleotide sequence SEQ ID NO: 144 or any variant thereof, and/or the light chain variable region nucleotide sequence of the nucleotide sequence SEQ ID NO: 159 or any variant thereof;

preferably, the nucleic acid molecule comprises the antibody heavy chain variable region nucleotide sequence of the nucleotide sequence SEQ ID NO: 146 or any variant thereof, and/or the light chain variable region nucleotide sequence of the nucleotide sequence SEQ ID NO: 155 or any variant thereof;

preferably, the nucleic acid molecule comprises the antibody heavy chain variable region nucleotide sequence of the nucleotide sequence SEQ ID NO: 146 or any variant thereof, and/or the light chain variable region nucleotide sequence of the nucleotide sequence SEQ ID NO: 157 or any variant thereof; and

preferably, the nucleic acid molecule comprises the antibody heavy chain variable region nucleotide sequence of the nucleotide sequence SEQ ID NO: 146 or any variant thereof, and/or the light chain variable region nucleotide sequence of the nucleotide sequence SEQ ID NO: 159 or any variant thereof.

8. A vector comprising the nucleic acid molecule of claim 7.

9. A cell comprising the nucleic acid of claim 7 or the vector of claim 8.

10. A composition comprising the antibody or antigen-binding portion thereof according to any one of claims 1-4, the bispecific or multispecific molecule of claim 5, the antibody or antigen-binding portion thereof or bispecific or multispecific molecule of claim 6, the nucleic acid molecule of claim 7, the vector of claim 8, and/or the cell of claim 9.

11. An antibody-drug conjugate comprising the antibody or the antigen-binding portion thereof according to any one of claims 1-4, the bispecific or multispecific molecule of claim 5, the antibody or the antigen-binding portion thereof or the bispecific or multispecific molecule of claim 6, covalently attached to a therapeutic portion;

preferably, the therapeutic portion is selected from the group consisting of a cytotoxic portion, a chemotherapeutic agent, a cytokine, an immunosuppressant, an immunostimulant, a lytic peptide, or a radioisotope;

preferably, the cytotoxic portion is selected from the group consisting of: paclitaxel; cytochalasin B; gramicidin D; ethidium bromide; emetine; mitomycin; etoposide; teniposide; vincristine; vinblastine; colchicine; doxorubicin; daunorubicin; dihydroxy anthracenedione; a tubulin inhibitor such as maytansine and an analog or derivative thereof; an antimitotic agent such as monomethyl auristatin E and F and analogs or derivatives thereof; hareotoxin 10 and 15 and analogs thereof; irinotecan and an analog thereof; mitoxantrone; mithramycin; actinomycin D; 1-dehydrotestosterone; a glucocorticoid; procaine; tetracaine; lidocaine; propranolol; puromycin; calicheamicin and an analog or derivative thereof; an anti-metabolite such as methotrexate, 6-mercaptopurine, 6-thioguanine, cytarabine, fludarabine, 5-fluorouracil, decadiazine, hydroxyurea, asparaginase, gemcitabine, and cladribine; an alkylating agent such as dichloromethyldiethylamine, thiopurine, chlorambucil, melphalan, carmustine (BSNU), lomustine (CCNU), cyclophosphamide, busulfan, mitobronitol, streptozotocin, dacarbazine (DTIC), procarbazine, and mitomycin C; a platinum derivative such as cisplatin or carboplatin; duocarmycin A, duocarmycin SA, rapamycin (CC-1065), and analogs or derivatives thereof; an antibiotic such as actinomycin, bleomycin, daunorubicin, doxorubicin, idarubicin, mithramycin, mitomycin, mitoxantrone, perimycin, and anthramycin (AMC); pyrrolo[2,1-c][1,4]-benzodiazepine (PDB); diphtheria toxin and related molecules such as diphtheria A chain and an active fragment and a hybrid molecule thereof, ricin such as ricin A and deglycosylated ricin A chain toxin, cholera toxin, a Shiga-like toxin such as SLT I, SLT II and SLT IIV, LT toxin, C3 toxin, Shiga toxin, pertussis toxin, tetanus toxin, soybean Bowman-Birk protease inhibitor, Pseudomonas exotoxin, alorin, saporin, modeccin, gelsolin, abrin A chain, modeccin A chain, $\alpha$-sarcin, Aleurites fordii protein, caryophyllin protein, Phytolacca americana protein such as PAPI, PAPII and PAP-S, Momordica charantia inhibitor, curcin, crotin, Sapaonaria officinalis inhibitor, gelonin, mitomycin, restrictocin, phenomycin, and enomycin toxin; ribonuclease (RNase); DNase I and Staphylococcus endotoxin A; pokeweed antiviral protein; and diphtheria toxin and Pseudomonas endotoxin;

preferably, the cytokine is selected from the group consisting of IL-2, IL-4, IL-6, IL-7, IL-10, IL-12, IL-13, IL-15, IL-18, IL-23, IL-24, IL-27, IL-28a, IL-28b, IL-29, KGF, IFN $\alpha$, IFN $\beta$, IFN $\gamma$, GM-CSF, CD40L, Flt3 ligand, stem cell factor, ancestine, and TNF $\alpha$;

preferably, the radioisotope is selected from the group consisting of $^3$H, $^{14}$C, $^{15}$N, $^{35}$S, $^{67}$Cu, $^{90}$Y, $^{99}$Tc, $^{125}$I, $^{131}$I, $^{186}$Re, $^{188}$Re, $^{211}$At, $^{212}$Bi, $^{212}$Pb, $^{213}$Bi, $^{225}$Ac and $^{227}$Th.

12. A kit comprising the antibody or antigen-binding portion thereof according to any one of claims 1-4, the bispecific or multispecific molecule of claim 5, the antibody or antigen-binding portion thereof or bispecific or multispecific molecule of claim 6, the nucleic acid molecule of claim 7, the vector of claim 8, the cell of claim 9, the composition of claim 10, and/or the antibody-drug conjugate of claim 11.

13. Use of the antibody or antigen-binding portion thereof according to any one of claims 1-4, the bispecific or multispecific molecule of claim 5, the antibody or antigen-binding portion or bispecific or multispecific molecule of claim 6, the nucleic acid molecule of claim 7, the vector of claim 8, the cell of claim 9, the composition of claim 10, and/or the antibody-drug conjugate of claim 11, in the manufacture of a medicament or kit for diagnosis, treatment or prevention of a CD70-associated condition;

preferably, the CD70-associated condition includes a tumor and an autoimmune disease; preferably, the tumor is a cancer disease; preferably, the cancer disease is selected from the group consisting of renal cell carcinoma (RCC), clear cell RCC, glioblastoma, non-Hodgkin's lymphoma (NHL), acute lymphocytic leukemia (ALL), chronic lymphocytic leukemia (CLL), Burkitt's lymphoma, anaplastic large cell lymphoma (ALCL), multiple myeloma, cutaneous T-cell lymphoma, nodular small cleaved cell lymphoma, lymphocytic lymphoma, peripheral T-cell lymphoma, Lennert's lymphoma, immunoblastic lymphoma, T-cell leukemia/lymphoma (ATLL), adult T-ALL, centroblastic/centrocytic (cb/cc) follicular lymphoma, B-lineage diffuse large cell lymphoma, angioimmunoblastic lymphadenopathy (AILD)-like T-cell lymphoma, HIV-associated body cavity lymphoma, embryonal carcinoma, undifferentiated nasopharyngeal carcinoma, Schmincke's tumor, Castleman's disease, Kaposi's sarcoma, multiple myeloma, Waldenstrom's macroglobulinemia, and B-cell lymphoma;

preferably, the autoimmune disease is lupus.

14. Use of the antibody or antigen-binding portion thereof according to any one of claims 1-4, the bispecific or multispecific molecule of claim 5, the antibody or antigen-binding portion thereof or bispecific or multispecific molecule of claim 6, the nucleic acid molecule of claim 7, the vector of claim 8, the cell of claim 9, the composition of claim 10, and/or the antibody-drug conjugate of claim 11, in the manufacture of a medicament or kit for treatment of a viral infection in a subject;

preferably, the viral infection is an infection by a virus selected from the group consisting of: human immunodeficiency virus (HIV), hepatitis (A, B, C), herpes viruses (e.g. VZV, HSV-1, HAV-6, HSV-II and CMV, Epstein-Barr virus), adenovirus, influenza virus, arbovirus, echovirus, rhinovirus, coxsackie virus, coronavirus, respiratory syncytial virus, mumps virus, rotavirus, measles virus, rubella virus, parvovirus, vaccinia virus, HTLV virus, dengue virus, papilloma virus, molluscum virus, polio virus, rabies virus, JC virus and arbovirus encephalitis virus, and lymphocytic choriomeningitis virus (LCMV).

Human CD70

FIG. 1

FIG. 2-1

FIG. 2-2

FIG. 3

FIG. 4-1

FIG. 4-2

FIG. 5

FIG. 6-1

FIG. 6-2

FIG. 7-1

FIG. 7-2

FIG. 8

FIG. 9-1

FIG. 9-2

FIG. 10-1

FIG. 10-2

## Daudi

FIG. 11-1

## MV-4-11-CD70

FIG. 11-2

## Raji

FIG. 11-3

## Daudi

FIG. 12-1

**Raji**

FIG. 12-2

FIG. 13

FIG. 14

FIG. 15-1

FIG. 15-2

FIG. 16

FIG. 17-1

# FcγRIII F158

FIG. 17-2

FIG. 18

## INTERNATIONAL SEARCH REPORT

| International application No. |
|---|
| **PCT/CN2021/143371** |

**A. CLASSIFICATION OF SUBJECT MATTER**

C07K 16/28(2006.01)i; C07K 16/46(2006.01)i; C07K 19/00(2006.01)i; C12N 15/13(2006.01)i; C12N 15/63(2006.01)i; A61K 39/395(2006.01)i; A61P 31/12(2006.01)i; A61P 35/00(2006.01)i; A61P 37/06(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNTXT; WPABS; WPABSC; ENTXTC; DWPI; VEN; CNKI; ISI; ELESEVER; NCBI; PUBMED; GOOGLE; GenBank; EMBL; STN; 万方, WANFANG; 中国专利生物序列检索系统, Chinese Patent Biological Sequence Retrieval System: CD-70, CD27, 抗体, antibody, SEQ ID NOS: 3-26, 35-41, 85, 89-93

**C. DOCUMENTS CONSIDERED TO BE RELEVANT**

| Category* | Citation of document, with indication, where appropriate, of the relevant passages | Relevant to claim No. |
|---|---|---|
| A | CN 101203241 A (SEATTLE GENETICS INC.) 18 June 2008 (2008-06-18) see entire document | 1-14 (in part) |
| A | CN 101370830 A (MEDAREX, INC.) 18 February 2009 (2009-02-18) see entire document | 1-14 (in part) |
| A | CN 103596979 A (ARGEN-X BV) 19 February 2014 (2014-02-19) see entire document | 1-14 (in part) |
| A | US 2009028872 A1 (TERRET, J. A. et al.) 29 January 2009 (2009-01-29) see entire document | 1-14 (in part) |
| A | US 2010150950 A1 (MEDAREX, INC) 17 June 2010 (2010-06-17) see entire document | 1-14 (in part) |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 March 2022** | **31 March 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/143371**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13ter.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13ter.1(a)).

        ☐ on paper or in the form of an image file (Rule 13ter.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2021/143371**

| Box No. III | Observations where unity of invention is lacking (Continuation of item 3 of first sheet) |
|---|---|

This International Searching Authority found multiple inventions in this international application, as follows:

[1]  Group 1: claims 1-14 (in part), which set forth the technical solutions relating to an antibody of which an HCVR and an LCVR are respectively as shown in SEQ ID NOs: 3 and 8, 13 and 17, 20 and 23, 35 and 39, and 89 and 92 or having the sequences of 6 CDR regions, or an antigen binding portion thereof.

[2]  Group 2: claims 1-14 (in part), which set forth the technical solutions relating to an antibody of which an HCVR and an LCVR are respectively as shown in SEQ ID NOs: 27 and 32, and 70 and 72 or the HCVR is selected from SEQ ID NOs: 140, 143, and 145 and the LCVR is selected from SEQ ID NOs: 154, 156, and 158, or having the sequences of 6 CDR regions, or an antigen binding portion thereof.

[3]  Group 3: claims 1-14 (in part), which set forth the technical solutions relating to an antibody of which an HCVR and an LCVR are respectively as shown in SEQ ID NOs: 42 and 47, and 75 and 78 or the HCVR is selected from SEQ ID NOs: 133, 136, and 138 and the LCVR is selected from SEQ ID NOs: 147, 150, and 152, or having the sequences of 6 CDR regions, or an antigen binding portion thereof.

[4]  Groups 4-9: claims 1-14 (in part), which set forth the technical solutions relating to an antibody of which an HCVR and an LCVR are respectively as shown in SEQ ID NOs: 52 and 57, 62 and 66, 81 and 86, 94 and 99, 104 and 108, and 122 and 125, or having the sequences of 6 CDR regions, or an antigen binding portion thereof.

[5]  Group 10: claims 1-14 (in part), which set forth the technical solutions relating to an antibody of which an HCVR and an LCVR are respectively as shown in SEQ ID NOs: 112 and 117, and 129 and 117 or having the sequences of 6 CDR regions, or an antigen binding portion thereof. Group 11: claims 1-14 (in part), which set forth the technical solutions relating to an antibody of which an HCVR and an LCVR or the sequences of 6 CDR regions thereof are different from those in groups 1-10, or an antigen binding portion thereof.

[6]  The common technical feature among groups 1-11 is that the antibody or antigen binding portion thereof binds to CD70. However, the prior art document CN101203241A (published on 18 June 2008, see the claims) discloses an anti-CD70 humanized antibody. Therefore, the described technical feature cannot constitute a special technical feature distinguished from the prior art. No corresponding experimental data is provided in the description to prove that the antibody of group 11 can also bind to CD70 and has the same function as the antibodies of groups 1-10. Therefore, group 11 and groups 1-10 do not share a same or corresponding special technical feature and do not comply with PCT Rules 13.1 and 13.2. Regarding groups 1-10, it is known in the art that except for a camel antibody, the binding of antibodies from other sources to antigens depends on a combined action of 6 CDRs, the 6 CDRs together constitute a functional unit thereof, and different antibodies involved in groups 1-10 do not have exactly the same 6 CDR sequences. Therefore, the described ten inventions likewise do not have a special technical feature that makes a contribution over the prior art, cannot be linked to each other, and do not comply with PCT Rules 13.1 and 13.2.

Form PCT/ISA/210 (continuation of first sheet) (January 2015)

## INTERNATIONAL SEARCH REPORT

| International application No. |
|---|
| **PCT/CN2021/143371** |

**Box No. III      Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-14 (in part), which set forth the technical solutions relating to an antibody of which an HCVR and an LCVR are respectively as shown in SEQ ID NOs: 3 and 8, 13 and 17, 20 and 23, 35 and 39, and 89 and 92, or having the sequences of 6 CDR regions, or an antigen binding portion thereof.**

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

International application No.

**PCT/CN2021/143371**

| Patent document cited in search report | | | Publication date (day/month/year) | Patent family member(s) | | | Publication date (day/month/year) |
|---|---|---|---|---|---|---|---|
| CN | 101203241 | A | 18 June 2008 | WO | 2006113909 | A2 | 26 October 2006 |
| | | | | WO | 2006113909 | A3 | 31 January 2008 |
| | | | | AU | 2006236225 | A1 | 26 October 2006 |
| | | | | AU | 2006236225 | B2 | 10 May 2012 |
| | | | | AU | 2006236225 | C1 | 02 May 2013 |
| | | | | US | 2012045436 | A1 | 23 February 2012 |
| | | | | US | 8562987 | B2 | 22 October 2013 |
| | | | | US | 2009148942 | A1 | 11 June 2009 |
| | | | | US | 8067546 | B2 | 29 November 2011 |
| | | | | CA | 2605507 | A1 | 26 October 2006 |
| | | | | CA | 2605507 | C | 28 June 2016 |
| | | | | JP | 2008538292 | A | 23 October 2008 |
| | | | | JP | 5122441 | B2 | 16 January 2013 |
| | | | | CN | 101203241 | B | 22 February 2012 |
| | | | | US | 2021002380 | A1 | 07 January 2021 |
| | | | | ES | 2477765 | T3 | 17 July 2014 |
| | | | | EP | 1871418 | A2 | 02 January 2008 |
| | | | | EP | 1871418 | A4 | 06 January 2010 |
| | | | | EP | 1871418 | B1 | 19 March 2014 |
| | | | | US | 2017022282 | A1 | 26 January 2017 |
| | | | | US | 9701752 | B2 | 11 July 2017 |
| | | | | DK | 1871418 | T3 | 10 June 2014 |
| | | | | JP | 2012205596 | A | 25 October 2012 |
| | | | | EP | 2511299 | A1 | 17 October 2012 |
| | | | | US | 2014178936 | A1 | 26 June 2014 |
| | | | | US | 9428585 | B2 | 30 August 2016 |
| | | | | US | 2017342157 | A1 | 30 November 2017 |
| CN | 101370830 | A | 18 February 2009 | ZA | 200802641 | B | 25 February 2009 |
| CN | 103596979 | A | 19 February 2014 | JP | 2016196471 | A | 24 November 2016 |
| | | | | JP | 6261651 | B2 | 17 January 2018 |
| | | | | US | 2015266963 | A1 | 24 September 2015 |
| | | | | US | 2017369581 | A9 | 28 December 2017 |
| | | | | US | 11072665 | B2 | 27 July 2021 |
| | | | | BR | 112013021562 | A2 | 08 November 2016 |
| | | | | DK | 2686347 | T3 | 25 June 2018 |
| | | | | PL | 2686347 | T3 | 31 October 2018 |
| | | | | JP | 2014509861 | A | 24 April 2014 |
| | | | | JP | 5982409 | B2 | 07 September 2016 |
| | | | | TR | 201807202 | T4 | 21 June 2018 |
| | | | | CY | 1120471 | T1 | 10 July 2019 |
| | | | | CA | 2828753 | A1 | 20 September 2012 |
| | | | | IL | 228001 | D0 | 30 September 2013 |
| | | | | IL | 228001 | A | 28 June 2018 |
| | | | | US | 2014141016 | A1 | 22 May 2014 |
| | | | | US | 9765148 | B2 | 19 September 2017 |
| | | | | HU | E039849 | T2 | 28 February 2019 |
| | | | | PT | 2686347 | T | 05 July 2018 |
| | | | | AU | 2012228194 | A1 | 12 September 2013 |
| | | | | AU | 2012228194 | B2 | 01 June 2017 |
| | | | | US | 2014147450 | A1 | 29 May 2014 |

Form PCT/ISA/210 (patent family annex) (January 2015)

**INTERNATIONAL SEARCH REPORT**
Information on patent family members

International application No.

**PCT/CN2021/143371**

| Patent document cited in search report | | | Publication date (day/month/year) | Patent family member(s) | | | Publication date (day/month/year) |
|---|---|---|---|---|---|---|---|
| | | | | US | 8834882 | B2 | 16 September 2014 |
| | | | | RU | 2013146106 | A | 27 April 2015 |
| | | | | RU | 2604196 | C2 | 10 December 2016 |
| | | | | ES | 2670874 | T3 | 01 June 2018 |
| | | | | SI | 2686347 | T1 | 31 August 2018 |
| | | | | EP | 2686347 | A1 | 22 January 2014 |
| | | | | EP | 2686347 | B1 | 02 May 2018 |
| | | | | WO | 2012123586 | A1 | 20 September 2012 |
| | | | | US | 2019270823 | A1 | 05 September 2019 |
| | | | | US | 2014235843 | A1 | 21 August 2014 |
| | | | | US | 9765149 | B2 | 19 September 2017 |
| US | 2009028872 | A1 | 29 January 2009 | US | 8124738 | B2 | 28 February 2012 |
| | | | | BR | PI0617549 | A2 | 26 July 2011 |
| | | | | NZ | 566395 | A | 30 March 2012 |
| | | | | EP | 1934261 | A2 | 25 June 2008 |
| | | | | EP | 1934261 | B1 | 29 October 2014 |
| | | | | NO | 20081987 | L | 25 June 2008 |
| | | | | ES | 2527961 | T3 | 02 February 2015 |
| | | | | CA | 2623236 | A1 | 05 April 2007 |
| | | | | WO | 2007038637 | A2 | 05 April 2007 |
| | | | | WO | 2007038637 | A3 | 04 October 2007 |
| | | | | JP | 2009509510 | A | 12 March 2009 |
| | | | | AU | 2006294663 | A1 | 05 April 2007 |
| | | | | AU | 2006294663 | B2 | 22 March 2012 |
| | | | | IL | 189788 | D0 | 07 August 2008 |
| | | | | EA | 200800953 | A1 | 27 February 2009 |
| | | | | EA | 016186 | B1 | 30 March 2012 |
| | | | | KR | 20080056167 | A | 20 June 2008 |
| US | 2010150950 | A1 | 17 June 2010 | WO | 2008074004 | A2 | 19 June 2008 |
| | | | | WO | 2008074004 | A3 | 04 December 2008 |
| | | | | TW | 200836760 | A | 16 September 2008 |
| | | | | AU | 2007333098 | A1 | 19 June 2008 |
| | | | | JP | 2010513306 | A | 30 April 2010 |
| | | | | MX | 2009006277 | A | 24 July 2009 |
| | | | | IN | 2404KON2009 | A | 07 August 2015 |
| | | | | CA | 2672468 | A1 | 19 June 2008 |
| | | | | KR | 20090088946 | A | 20 August 2009 |
| | | | | NZ | 578354 | A | 12 January 2012 |
| | | | | EP | 2097534 | A2 | 09 September 2009 |
| | | | | EP | 2097534 | A4 | 12 May 2010 |
| | | | | CL | 2007003649 | A1 | 23 January 2009 |
| | | | | AR | 064360 | A1 | 01 April 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200680021851
- CN 200680035376
- CN 201280013552
- CN 200680035376X
- US 5635483 A
- US 5780588 A
- US 6214345 B
- WO 02088172 A
- WO 2004010957 A
- WO 2005081711 A
- WO 2005084390 A
- WO 2006132670 A
- WO 03026577 A
- WO 200700860 A
- WO 207011968 A
- WO 205082023 A
- WO 2012059882 A

**Non-patent literature cited in the description**

- **CARRILLO, H. ; LIPMAN, D.** *SIAM J Applied Math,* 1988, vol. 48, 1073
- **SMITH et al.** *J.Clin.Pathol.,* 2004, vol. 57, 912-917
- **NELSON et al.** *J Clin Pathol,* 2000, vol. 53, 111-117
- **RICH ; MYSZKA.** *Curr. Opin.Biotechnol,* 2000, vol. 11, 54
- **ENGLEBIENNE.** *Analyst.,* 1998, vol. 123, 1599
- **MALMQVIST.** *Biochem.Soc.Trans.,* 2000, vol. 27, 335
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc.Natl.Acad.Sci.USA,* 1997, vol. 94, 412-417
- **TARABAN et al.** *J.Immunol.,* vol. 173, 6542-6
- **MATTER et al.** *J Exp Med,* 2006, vol. 203, 2145-55
- **HARTLEY J.A. et al.** *Cancer Res,* 2010, vol. 70 (17), 6849-6858
- **ANTONOW D. et al.** *Cancer J,* 2008, vol. 14 (3), 154-169
- **HOWARD P.W. et al.** *Bioorg Med Chem Lett,* 2009, vol. 19, 6463-6466
- **SAGNOU et al.** *Bioorg MedChem Lett,* 2000, vol. 10 (18), 2083-2086
- **BLAUG ; SEYMOUR.** Remington's Pharmaceutical Sciences. Mack Publishing Company, 1975
- Remington's Pharmaceutical Sciences: The Science And Practice Of Pharmacy. Mack Pub. Co, 1995
- **MARASCO et al.** *Proc. Natl. Acad. Sci. USA,* 1993, vol. 90, 7889-7893